# EUROPEAN PATENT SPECIFICATION

(11) **EP 1 791 853 B1**
(45) Date of publication and mention of the grant of the patent: **26.02.2014**
(21) Application number: 05762779.6
(22) Date of filing: 14.06.2005
(51) Int. Cl.: C12N 9/18, A23L 1/27, C11B 3/00, A23K 1/165, A23L 1/227

(54) **COMPOSITIONS AND METHODS FOR ENZYMATIC DECOLORIZATION OF CHLOROPHYLL**
ZUSAMMENSETZUNGEN UND VERFAHREN ZUR ENZYMATISCHEN ENTFÄRBUNG VON CHLOROPHYLL
COMPOSITIONS ET PROCEDES POUR LA DECOLORATION ENZYMATIQUE DE LA CHLOROPHYLLE

(30) Priority: 16.06.2004 US 580447 P
(43) Date of publication of application: 06.06.2007
(60) Divisional application: 12150400.5
(73) Proprietor: DSM IP Assets B.V., 6411 TE Heerlen (NL)
(72) Inventor: LAM, David, San Marcos, CA 92078 (US); WEINER, David, Del Mar, CA 92014 (US); HITCHMAN, Timothy, San Diego, CA 92109 (US); BARTON, Nelson Robert, San Diego, CA 92131 (US); BURK, Mark J., San Diego, CA 92130 (US)
(74) Representative: Cazemier, Anne Engeline
(86) International application number: PCT/US2005/020866
(87) International publication number: WO 2006/009676

(56) References cited:
- EP-A2- 0 268 456
- WO-A2-02/29022
- WO-A2-2005/032496
- WO-A2-2006/096834
- KHAMESSAN A; KERMASHA S: "Biocatalysis of chlorophyllase in canola oil using organic solvent systems." JOURNAL OF FOOD BIOCHEMISTRY 1996 CORRESPONDENCE (REPRINT) ADDRESS, S. KERMASHA, DEP. OF FOOD SCI. & AGRIC. CHEM., MCGILL UNIV., 21 111 LAKESHORE, ST. ANNE DE BELLEVUE, QUE. H9X 3V9, CANADA. T, vol. 20, no. 4, 1 January 1996 (1996-01-01), page 311, XP002543900
- BITAR MARIANNE; KARBOUNE SALWA; BISAKOWSKI BARBARA; KERMASHA SELIM: "Chlorophyllase biocatalysis in an aqueous/miscible organic solvent medium containing canola oil" JOURNAL OF THE AMERICAN OIL CHEMISTS' SOCIETY, vol. 81, no. 10, October 2004 (2004-10), pages 927-932, XP002543901 ISSN: 0003-021X
- DATABASE Geneseq [Online] 27 July 2006 (2006-07-27), "Environmental isolate DNA encoding a hydrolase, SEQ ID NO:11." XP002543902 retrieved from EBI accession no. GSN:AEH46911 Database accession no. AEH46911
- DATABASE Geneseq [Online] 27 July 2006 (2006-07-27), "Environmental isolate hydrolase, SEQ ID NO:12." XP002543903 retrieved from EBI accession no. GSP:AEH46912 Database accession no. AEH46912

## Description

### TECHNICAL FIELD

This invention relates to the fields of industrial processing of foods, feeds or vegetable oils, plant and animal products, and enzymology. In particular, the invention provides compositions and methods for the enzymatic treatment ("bleaching" or "de-colorizing") of chlorophyll-containing or chlorophyll-contaminated compositions, e.g., algal, animal or plant preparations, foods, feeds or oils, for example, vegetable oils, including oils processed from oilseeds, such as canola (rapeseed) oil or soybean oil, or oil fruits, such as palm oil. In one aspect, the invention provides methods using enzymes from chlorophyll catabolism (e.g., a chlorophyllase) for the enzymatic modification of a chlorophyll, e.g., in an algal, animal or plant preparation, or a food, a feed or an oil.

### BACKGROUND

Vegetable oils coming from oilseeds such as canola or soybean or oilfruits such as palm contain chlorophyll. Chlorophyll is removed during many stages of the oil production process, including seed crushing, oil extraction, degumming, caustic treatment and bleaching steps. In the last of these, the bleaching process residual chlorophyll is removed to achieve acceptable levels. This chlorophyll is typically removed from the oil in a bleaching process step involving heating the oil and running it through an adsorbent to remove chlorophyll and other color-bearing compounds that impact the appearance and/or stability of the finished oil. This technology is also used to treat other chlorophyll-containing oils or plant or algal preparations, such as polyunsaturated fatty acid (PUFA) (e.g., eicosapentaenoic acid (EPA) and docosahexaenoic acid (DHA)) containing oils.

High level of chlorophyll pigments impart undesirable color and induce oxidation of oil during storage leading to a deterioration of the oil. In the edible oil processing industry, a bleaching step is employed to lower chlorophyll levels to as low as 0.1 ppm to guarantee oil quality in terms of color and organolepticity. Typical desired finished chlorophyll levels are between 0.02 to 0.05 ppm. This bleaching step increases processing cost and reduces oil yield due to entrainment in the bleaching clay.

In plants, chlorophyllase (chlase) is the first enzyme involved in chlorophyll degradation; it catalyzes the hydrolysis of an ester bond in chlorophyll to yield chlorophyllide and phytol.

The degradation of chlorophyll in canola oil with a chlorophyllase derived from the alga *Phaeodactylum tricornutum* is known from Khamessan and Kermasha, J. Food Biochemistry 1996 (20), p. 311-328. The chlorophyllase activity disclosed herein, appears to be influenced by the presence of a surfactant, an organic solvent and the concentration of canola oil.

WO2005/032496 discloses polynucleotides encoding polypeptides having hydrolase activity. WO2005/032496 does not disclose the use of hydrolases in chlorophyll degradation.

### SUMMARY

The invention provides compositions and methods for enzymatic treatment ("bleaching" or "de-colorizing") of chlorophyll-containing or chlorophyll-contaminated compositions such as plant, animals (e.g., fish, meat preparations) or algal preparations, foods, feeds or oils, such as polyunsaturated fatty acid (PUFA)-containing or docosahexaenoic acid (DHA)-containing oils, or compositions comprising mixtures thereof. , The "enzymatic bleaching" of the compositions and methods of the invention comprises use of a chlorophyll modifying enzyme, e.g., a polypeptide having chlorophyllase activity, including chlases and chlorophyll chlorophyllido-hydrolyases, and related polypeptides, or any chlorophyll catabolic enzyme. Thus, as used herein, the term "enzymatic bleaching" includes any modification of a chlorophyll molecule or equivalent, including partial or complete decolorization. Tthe compositions and methods of the invention can reduce yield loss from entrainment and fat splitting attributed to catalysis by clay/bleach conditions.

In the methods and processes of the invention, a chlorophyllase, which can be a novel chlorophyllase of the invention, or a known enzyme, including chlases and chlorophyll chlorophyllido-hydrolyases and related polypeptides, or a combination thereof, or any chlorophyll catabolic enzyme, is added anytime or anywhere in the method or process, e.g., as discussed herein. For example, the chlorophyllase (which can be a novel chlorophyllase of the invention, or a known enzyme, or a combination thereof) and/or any chlorophyll catabolic enzyme can be added into a composition, such as a crude oil, with or without another enzyme, e.g., a phospholipase (e.g., phospholipase C) at a mixing step or in a degumming step, in a caustic tank step, in a static mixer, in a day tank or in a retention mixer. Alternatively, a method or process is disclosed wherein the chlorophyllase (of the invention, or known) and/or any chlorophyll catabolic enzyme can be added into any combination of these steps, or in all of these steps.

The invention provides methods or processes for enzymatic modification of chlorophyll to facilitate its removal from a composition, e.g., through an aqueous separation process, as illustrated in page 1, Appendix A, or hydrophobic separation process, or affinity separation process, and the like.

The invention provides methods and processes comprising enzymatic modification (e.g., catabolism) of chlorophyll, or equivalent compounds, in a composition (e.g., a food, feed, plant, animal, algae, etc.) further comprising removal of components of that composition (e.g., compounds not desirable in a finished product), such as residual chlorophyll (e.g., chlorophyll or equivalent compounds not modified by a chlorophyllase), a pesticide, a polycyclic aromatic hydrocarbon, etc. Undesirable components, e.g., residual chlorophyll, pesticides, polycyclic aromatic hydrocarbons and the like, can be removed with either significantly smaller amounts of bleaching clay or other adsorbent, such as silica or equivalent compounds.

These components of the composition are removed using a bleaching clay, e.g., in a plurality of steps using bleaching clay, where components of the composition are removed with either significantly smaller amounts of bleaching clay and/or at least one other adsorbent (e.g., a silica). Finished chlorophyll levels are between about 0.02 ppm to 0.0.5 ppm. In this exemplary process, the bleaching step can increase processing costs and reduce oil yields due to entrainment in the bleaching clay. The compositions and processes as disclosed herein can reduce yield loss from entrainment and fat splitting attributed to catalysis by clay/bleach conditions.

In an exemplary illustrated method (reaction) of the invention, a chlorophyllase catalyzes the hydrolysis of chlorophyll to generate chlorophyllide, which is aqueous extracted, and phytol, which remains in the oil phase. In another exemplary method, pheophorbide can be removed in manner similar to chlorophyllide. Compositions and methods are disclosed, wherein an aqueous separation process can partially or completely eliminate the need for adsorbants. However, the methods comprise partial or complete extraction of the aqueous soluble chlorophyllide or pheophorbide using a silica-based extraction process (e.g., adsorbent-free or reduced adsorbent silica refining). In one aspect, the chlorophyllase is immobilized onto a silica (which then adsorbs the chlorophyllide), e.g., a silica gel. The silica can comprise a TriSyl Silica or a SORBSIL R™ silica.

The invention provides methods, including industrial processes, for enzymatic treatment of pheophytin-containing or pheophytin-contaminated compositions comprising the following steps: (a) providing a pheophytin-containing or pheophytin-contaminated composition; (b) providing a polypeptide having a chlorophyllase or pheophytinase activity (which can be a novel chlorophyllase of the invention, or a known enzyme, or a combination thereof); and (c) reacting the composition of step (a) with the polypeptide of step (b) under conditions wherein the polypeptide can catalyze a pheophytin-modifying reaction. The magnesium-less derivative of chlorophyll is called pheophytin. Pheophytin is colored and often present in oil, especially if acid treatment has been used. In some applications, it is desirable to remove the pheophytin. The product of chlorophyllase treatment of pheophytin is pheophorbide, which can be removed in a similar manner to chlorophyllide.

In one aspect, the compositions and methods of the invention are practiced as or with industrial processes, e.g., oil bleaching or caustic neutralization or degumming processes. Using the compositions and methods as disclosed herein facilitate reducing the amount of or eliminating the need for adsorbants in current bleaching processing, which typically involve heating the oil or other chlorophyll-containing composition and running it through an adsorbent to remove chlorophyll and other color-bearing compounds that impact the appearance and/or stability of the finished oil. Thus, in practicing the invention, by partially or completely eliminate the need for adsorbants, processing costs can be decreased, e.g., adsorbents (e.g., clay) costs, disposal costs, water costs, energy costs, steam costs can be decreased. Other benefits in practicing various aspects of the invention include yield improvements, e.g., reduced entrained oils in adsorbent substrates, increased end product value, including retention of valuable micronutrients such as beta carotene, process efficiencies, including reduced processing steps, capital savings and an environmental benefit, e.g., reducing or eliminating land-filling of bleaching adsorbents.

In practicing the compositions and methods of the invention, the chlorophyll-modifying polypeptides (which can be a novel chlorophyllase of the invention, or a known enzyme, or a combination thereof) can be employed at any point in a degumming (e.g., enzymatic degumming) process. For example, the chlorophyll-modifying polypeptides can be added before or after any step in a process, or before or after any combination of steps, or before or after all of the steps, in a process, e.g., prior to, during or following mechanical and/or chemical extraction, and/or degumming and/or caustic neutralization and/or bleaching and the like.

In alternative aspects of any of the methods of the invention, at least one step is performed in a reaction vessel, e.g., an oil degumming apparatus. Alternatively In the methods of the invention, at least one step is performed in a cell extract. Alternatively any of the methods of the invention, at least one step is performed in a whole cell. The cell can be of any source, e.g., a plant cell, a bacterial cell, a fungal cell, an animal cell (e.g., a mammalian cell, a fish cell) or a yeast cell.

Herein disclosed are methods for enzymatic treatment of chlorophyll-containing or chlorophyll-contaminated compositions comprising the following steps: (a) providing a chlorophyll-containing or chlorophyll-contaminated composition (which can be a novel chlorophyllase) of the invention, or a known enzyme, or a combination thereof); (b) providing a polypeptide having a chlorophyllase activity; and (c) reacting the composition of step (a) with the polypeptide of step (b) under conditions wherein the polypeptide can catalyze a chlorophyll-modifying reaction.

Herein disclosed are industrial processes for enzymatic treatment ("bleaching") of chlorophyll-containing or chlorophyll-contaminated compositions comprising the following steps: (a) providing a chlorophyll-containing or chlorophyll-contaminated composition (which can be a novel chlorophyllase of the invention, or a known enzyme, or a combination thereof); (b) providing a polypeptide having chlorophyllase activity; and (c) reacting the composition of step (a) with the polypeptide of step (b) under conditions wherein the polypeptide can catalyze a chlorophyll-modifying reaction.

The invention provides degumming processes comprising a step for enzymatic bleaching of chlorophyll-containing or chlorophyll-contaminated compositions comprising the following steps: (a) providing a chlorophyll-containing or chlorophyll-contaminated composition (which can be a novel chlorophyllase of the invention, or a known enzyme, or a combination thereof); (b) providing a polypeptide having chlorophyllase activity; and (c) reacting the composition of step (a) with the polypeptide of step (b) under conditions wherein the polypeptide can catalyze a chlorophyll-modifying reaction.

There is a second ester on chlorophylls and pheophytins - a methyl ester. The methods can further comprise hydrolysis of this methyl ester by an esterase. This can increase the tendency of the reaction derivative (now a diacid) to partition into an aqueous layer.

In an exemplary method, a phospholipase, e.g., a phospholipase C, or another hydrolase (e.g., a cellulase, a hemicellulase, an esterase, a protease and/or a phosphatase) is used, e.g., to improve oil extraction and oil degumming.

Herein further disclosed are methods and processes which can further comprise hydrolysis of methyl ester on a chlorophyll or a pheophytin by an esterase (which can be a novel enzyme of the invention, or a known enzyme, or a combination thereof). In alternative aspects, the methods of the invention can further comprise removal of the modified chlorophyll in an aqueous extraction. The methods can further comprise modifying pH (e.g., increasing pH) to promote aqueous separation of chlorophyllide. The enzymes used in the methods, e.g., a chlorophyllase, can be added during this increased pH, or "caustic" phase in the separation process. The methods can further comprise a caustic neutralization step. The methods can further comprise an adsorbent-free or reduced adsorbent silica refining step to remove a chlorophilide generated by the enzymatic degradation of the chlorophyll. The methods can further comprise use of a hydrolase, e.g., a phospholipase C.

In the methods and processes, the polypeptide is an esterase (e.g., an enzyme of the invention), e.g., a chlorophyllase, or has chlorophyllase-like activity, or has chlorophyll catabolic activity. In one aspect of the methods, the polypeptide is immobilized. The polypeptide can be immobilized on an inorganic support or organic support. The inorganic support can comprise alumina, celite, Dowex-1-chloride, glass beads or silica gel or equivalent. The polypeptide can be immobilized on an alginate hydrogel or alginate bead or equivalent. In the methods, the polypeptide further comprises a liposome, a hydrogel or a gel.

In one aspect of the methods, the polypeptide is at least one step is performed in a reaction vessel, e.g., a vessel comprising a gravitational gum separation device or a holding tank or the like. In one aspect of the methods, at least one step is performed in a cell extract, or a whole cell. The cell can be a plant cell, a bacterial cell, a fungal cell, a yeast cell, a mammalian cell, an insect cell and the like.

In one aspect of the methods, the chlorophyll-containing or chlorophyll-contaminated composition comprises a plant material, plant oil or plant extract. The plant material, plant oil or plant extract can comprise a vegetable oil or a seed oil. The vegetable oil can comprise a palm oil or a canola oil. Alternatively, the plant material, plant oil or plant extract can comprise an algal preparation. In one aspect of the methods, the chlorophyll-containing or chlorophyll-contaminated compositions comprise a non-wood or wood product. In the methods, the chlorophyll-containing or chlorophyll-contaminated compositions comprise a fabric or cloth. The methods, the chlorophyll-containing or chlorophyll-contaminated compositions comprise a pharmaceutical formulation, a food, an oil, a feed, or a dietary supplement.

The compositions and methods of the invention can be used to treat crude or refined oils, e.g., oils derived from plant (e.g., vegetable), algae, animal or fish, or synthetic, sources. The compositions and methods of the invention can be used to treat crude or refined oils at higher oil concentrations, or, in one aspect, used to treat unrefined and non-diluted crude oils.

The methods disclosed herein further comprise removal of a chlorophilide generated by enzymatic degradation of a chlorophyll by adsorbing onto a silica gel or equivalent. The chlorophyll-containing or chlorophyll-contaminated compositions can comprise a textile, cloth, thread or fabric or related composition, a wood or paper product or by-product, such as a wood pulp, a paper pulp, a Kraft pulp, or, a non-wood paper product or by-product, such as a rice paper.

The disclosure provides products of manufacture comprising a degumming system for the enzymatic treatment of chlorophyll-containing or chlorophyll-contaminated compositions comprising: (a) a vegetable oil refining apparatus; and (b) a polypeptide having chlorophyllase activity (e.g., an enzyme of the invention), wherein the activity of the polypeptide comprises catalysis of a chlorophyll-modifying reaction, and the vegetable oil refining apparatus can react a chlorophyll-containing or chlorophyll-contaminated composition with the polypeptide to under conditions wherein the polypeptide can catalyze a chlorophyll-modifying reaction. The product of manufacture, the vegetable oil refining apparatus comprises an oil leaving expellor, a holding tank or a gravitational gum separation device. The chlorophyll-modifying reactions can comprise generation of chlorophyllide and phytol.

Disclosed herein are detergents-comprising an enzymatic treatment of chlorophyll-containing or chlorophyll-contaminated fabrics comprising: (a) a detergent composition; and (b) a polypeptide having chlorophyllase activity (e.g., an enzyme of the invention), wherein the activity comprises catalysis of a chlorophyll-modifying reaction. In one aspect, the chlorophyll-modifying reaction comprises generation of chlorophyllide and phytol.

Disclosed herein are methods for enzymatically treating a chlorophyll-containing or chlorophyll-contaminated fabrics comprising: (a) providing a detergent composition comprising a polypeptide having chlorophyllase activity (e.g., an enzyme of the invention), wherein the activity comprises catalysis of a chlorophyll-modifying reaction; and, (b) contacting the detergent composition with the chlorophyll-containing or chlorophyll-contaminated fabric under conditions wherein the polypeptide can catalyze a chlorophyll-modifying reaction. In one aspect, the chlorophyll-modifying reaction comprises generation of chlorophyllide and phytol.

The invention provides use of a polypeptide that encoded by an isolated, synthetic or recombinant nucleic acid comprising a nucleic acid sequence having at least about 90%, 91%, 92%, 93%, 94%, 95%, 96%, 97%, 98%, 99%, or more, or complete (100%) sequence identity to an exemplary nucleic acid of the invention, e.g., SEQ ID NO:9

The sequence identities are determined by analysis with a sequence comparison algorithm or by a visual inspection. The sequence comparison algorithm is a BLAST algorithm, e.g., a BLAST version 2.2.2 algorithm where a filtering setting is set to blastall -p blastp -d "nr pataa" -F F, and all other options are set to default.

Exemplary nucleic acids disclosed herein also include isolated, synthetic or recombinant nucleic acids encoding a polypeptide of the invention, e.g., a polypeptide having a sequence as set forth in SEQ ID NO:2, SEQ ID NO:4, SEQ ID NO:6, SEQ ID NO:8, SEQ ID NO:10, SEQ ID NO:12, SEQ ID NO:14, SEQ ID NO:16, SEQ ID NO:18 or SEQ ID NO:20, and subsequences thereof and variants thereof.

The polypeptide used in the present invention has an esterase enzyme activity, including chlorophyllase (a chlase) activity, or, enzyme activity comprising enzymatic modification of a chlorophyll molecule, e.g., wherein the enzymatic modification comprises catabolism of the chlorophyll molecule. In one aspect, the esterase activity comprises a chlorophyll chlorophyllido-hydrolyase activity.

The isolated, synthetic or recombinant nucleic acid of the invention encodes a polypeptide having an enzyme activity that is thermostable. The polypeptide can retain enzyme activity under conditions comprising a temperature range of between about 37°C to about 95°C; between about 55°C to about 85°C, between about 70°C to about 95°C, or, between about 90°C to about 95°C.

An isolated, synthetic or recombinant nucleic acid of the invention encodes a polypeptide having enzyme that is thermotolerant. The polypeptide can retain enzyme activity after exposure to a temperature in the range from greater than 37°C to about 95°C or anywhere in the range from greater than 55°C to about 85°C. The polypeptide can retain enzyme activity after exposure to a temperature in the range between about 1 °C to about 5°C, between about 5°C to about 15°C, between about 15°C to about 25°C, between about 25°C to about 37°C, between about 37°C to about 95°C, between about 55°C to about 85°C, between about 70°C to about 75°C, or between about 90°C to about 95°C, or more. The polypeptide retains enzyme activity after exposure to a temperature in the range from greater than 90°C to about 95°C at pH 4.5.

Disclosed herein are isolated, synthetic or recombinant nucleic acids comprising a sequence that hybridizes under stringent conditions to a nucleic acid comprising a sequence of the invention, e.g., a sequence as set forth in SEQ ID NO:1, SEQ ID NO:3, SEQ ID NO:5, SEQ ID NO:7, SEQ ID NO:9, SEQ ID NO:11, SEQ ID NO:13, SEQ ID NO:15, SEQ ID NO:17 or SEQ ID NO:19, or fragments or subsequences thereof (or complements thereof). In one aspect, the nucleic acid of the invention encodes a polypeptide having an esterase enzyme activity, including chlorophyllase (a chlase) activity, or, enzyme activity comprising enzymatic modification of a chlorophyll molecule, e.g., wherein the enzymatic modification comprises catabolism of the chlorophyll molecule. The esterase activity disclosed herein comprises a chlorophyll chlorophyllido-hydrolyase activity. The nucleic acid can be at least about 10, 15, 20, 25, 30, 35, 40, 45, 50, 75, 100, 150, 200, 250, 300, 350, 400, 450, 500, 550, 600, 650, 700, 750, 800, 850, 900, 950, 1000, 1050, 1100, 1150, 1200 or more residues in length or the full length of the gene or transcript. The stringent conditions include a wash step comprising a wash in 0.2X SSC at a temperature of about 65°C for about 15 minutes.

Disclosed herein is a nucleic acid probe for identifying a nucleic acid encoding a polypeptide having an enzyme activity as described herein (e.g., esterase enzyme activity, including chlorophyllase (a chlase) activity), wherein the probe comprises at least about 10, 15, 20, 25, 30, 35, 40, 45, 50, 55, 60, 65, 70, 75, 80, 85, 90, 95, 100, 150, 200, 250, 300, 350, 400, 450, 500, 550, 600, 650, 700, 750, 800, 850, 900, 950, 1000 or more, consecutive bases of a sequence comprising a sequence of the invention, or fragments or subsequences thereof, wherein the probe identifies the nucleic acid by binding or hybridization.

Disclosed herein is a nucleic acid probe for identifying a nucleic acid encoding a polypeptide having at least one enzyme activity as described herein (e.g., esterase enzyme activity, including chlorophyllase (a chlase) activity), wherein the probe comprises a nucleic acid comprising a sequence at least about 10, 15, 20, 30, 40, 50, 60, 70, 80, 90, 100, 150, 200, 250, 300, 350, 400, 450, 500, 550, 600, 650, 700, 750, 800, 850, 900, 950, 1000 or more residues having at least about 50%, 51%, 52%, 53%, 54%, 55%, 56%, 57%, 58%, 59%, 60%, 61%, 62%, 63%, 64%, 65%, 66%, 67%, 68%, 69%, 70%, 71 %, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or more, or complete (100%) sequence identity to a nucleic acid of the invention, wherein the sequence identities are determined by analysis with a sequence comparison algorithm or by visual inspection.

The probe can comprise an oligonucleotide comprising at least about 10 to 50, about 20 to 60, about 30 to 70, about 40 to 80, or about 60 to 100 consecutive bases of a nucleic acid sequence of the invention, or a subsequence thereof.

Disclosed herein are nucleic acids encoding proteins (e.g., enzymes), including the polypeptides of the invention, generated by amplification, e.g., polymerase chain reaction (PCR), using an amplification primer pair of the invention. The invention provides nucleic acids encoding polypeptides having at least one enzyme activity as described herein (e.g., esterase enzyme activity, including chlorophyllase (a chlase) activity) using an amplification primer pair of the invention. The invention provides methods of making and/or identifying enzymes by amplification, e.g., polymerase chain reaction (PCR), using an amplification primer pair of the invention. The amplification primer pair amplifies a nucleic acid from a library, e.g., a gene library, such as an environmental library.

Disclosed herein are expression cassettes comprising a nucleic acid of the invention or a subsequence thereof. The expression cassette can comprise the nucleic acid that is operably linked to a promoter. The promoter can be a viral, bacterial, mammalian or plant promoter..

The disclosure provides cloning vehicles comprising an expression cassette (e.g., a vector) of the invention or a nucleic acid of the invention..

The disclosure provides transformed cell comprising a nucleic acid of the invention or an expression cassette (e.g., a vector), or a cloning vehicle. The transformed cell can be a bacterial cell, a mammalian cell, a fungal cell, a yeast cell, an insect cell or a plant cell. Disclosed herein is an antisense oligonucleotide comprising a nucleic acid sequence complementary to or capable of hybridizing under stringent conditions to a nucleic acid of the invention. Methods of inhibiting the translation of a enzyme message (of an enzyme of the invention) in a cell comprising administering to the cell or expressing in the cell an antisense oligonucleotide comprising a nucleic acid sequence complementary to or capable of hybridizing under stringent conditions to a nucleic acid of the invention are herein disclosed. The antisense oligonucleotide is between about 10 to 50, about 20 to 60, about 30 to 70, about 40 to 80, or about 60 to 100 bases in length.

Methods of inhibiting the translation of an enzyme message in a cell comprising administering to the cell or expressing in the cell an antisense oligonucleotide comprising a nucleic acid sequence complementary to or capable of hybridizing under stringent conditions to a nucleic acid of the invention. The invention provides double-stranded inhibitory RNA (RNAi) molecules comprising a subsequence of a sequence of the invention. The RNAi is about 15, 16, 17, 18, 19, 20, 21, 22, 23, 24, 25 or more duplex nucleotides in length. The invention provides methods of inhibiting the expression of a polypeptide (e.g., an enzyme of the invention) in a cell comprising administering to the cell or expressing in the cell a double-stranded inhibitory RNA (iRNA), wherein the RNA comprises a subsequence of a sequence of the invention.

Disclosed herein is an isolated, synthetic or recombinant polypeptide comprising an amino acid sequence having at least about 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or more, or complete (100%) sequence identity to an exemplary polypeptide or peptide of the invention, such as 90%, 91%, 92%, 93%, 94%, 95%, 96%, 97%, 98%, 99% identical to SEQ ID NO: 10 that is used in the invention, over a region of at least about 20, 25, 30, 35, 40, 45, 50, 55, 60, 65, 70, 75, 100, 125, 150, 175, 200, 225, 250, 275, 300, 325, 350 or more residues, or over the full length of the polypeptide. The sequence identities are determined by analysis with a sequence comparison algorithm or by a visual inspection. Exemplary polypeptide or peptide sequences disclosed herein include SEQ ID NO:2, SEQ ID NO:4, SEQ ID NO:6, SEQ ID NO:8, SEQ ID NO:10, SEQ ID NO:12, SEQ ID NO:14, SEQ ID NO:16, SEQ ID NO:18 or SEQ ID NO:20, and subsequences thereof and variants thereof. Exemplary polypeptides also include fragments of at least about 10, 15, 20, 25, 30, 35, 40, 45, 50, 75, 80, 85, 90, 95, 100, 150, 200, 250, 300, 350, 400, 450, 500, 550, 600 or more residues in length, or over the full length of an enzyme. Exemplary polypeptide or peptide sequences of the invention include sequence encoded by a nucleic acid of the invention. Exemplary polypeptide or peptide sequences of the invention include polypeptides or peptides specifically bound by an antibody of the invention. The peptide can be, e.g., an immunogenic fragment, a motif (e.g., a binding site), a signal sequence, a prepro sequence, a catalytic domains (CDs) or an active site.

A polypeptide used in the invention has an esterase activity, such as a chlorophyllase (a chlase) activity, or, has an enzyme activity comprising enzymatic modification of a chlorophyll molecule, e.g., wherein the enzymatic modification comprises catabolism of the chlorophyll molecule. The esterase activity comprises a chlorophyll chlorophyllido-hydrolyase activity.

The present disclosure provides an isolated, synthetic or recombinant polypeptide or peptide including at least 10, 15, 20, 25, 30, 35, 40, 45, 50, 55, 60, 65, 70, 75, 80, 85, 90, 95 or 100 or more consecutive bases of a polypeptide or peptide sequenceused in the invention, sequences substantially identical thereto, and the sequences complementary thereto. The peptide can be, e.g., an immunogenic fragment, a motif (e.g., a binding site), a signal sequence, a prepro sequence or a catalytic domains (CDs) or active site.

The present disclosure provides biosynthetic systems comprising nucleic acids and/or plasmids of the invention in a cell, e.g., a yeast cell, a plant cell, a fungal cell, or a microbial (e.g., bacterial) cell. , The biosynthetic systems disclosed herein comprise coding sequences for all the enzymes necessary, or a subset thereof, for catabolism of a chlorophyll molecule. The coding sequences can be in a plasmid, a recombinant vector or virus and the like.

The enzyme activity of a polypeptide disclosed herein, for instance the polypeptide used in the present invention may be thermostable. The polypeptide disclosed herein, for instance the polypeptide used in the invention can retain activity under conditions comprising a temperature range of between about 1°C to about 5°C, between about 5°C to about 15°C, between about 15°C to about 25°C, between about 25°C to about 37°C, between about 37°C to about 95°C, between about 55°C to about 85°C, between about 70°C to about 75°C, or between about 90°C to about 95°C, or more. The enzyme activity of a polypeptide used in the invention may be thermotolerant. The polypeptide can retain activity after exposure to a temperature in the range from greater than 37°C to about 95°C, or in the range from greater than 55°C to about 85°C. The polypeptide can retain activity after exposure to a temperature in the range from greater than 90°C to about 95°C at pH 4.5.

The isolated, synthetic or recombinant polypeptide can comprise the polypeptide disclosed herein that lacks a signal sequence. The isolated, synthetic or recombinant polypeptide can comprise the polypeptide disclosed herein comprising a heterologous signal sequence.

The disclosure provides an isolated, synthetic or recombinant polypeptide of the invention, wherein the polypeptide comprises at least one glycosylation site. Glycosylation can be an N-linked glycosylation. The polypeptide can be glycosylated after being expressed in a *P. pastoris* or a *S. pombe.*

A polypeptide of the present disclosure can retain enzyme activity under conditions comprising about pH 6.5, pH 6, pH 5.5, pH 5, pH 4.5 or pH 4 or more acidic. Alternatively a polypeptide disclosed herein retains activity under conditions comprising about pH 7, pH 7.5 pH 8.0, pH 8.5, pH 9, pH 9.5, pH 10, pH 10.5 or pH 11 or more basic. A polypeptide of the present disclosure retains activity under conditions comprising about pH 6.5, pH 6, pH 5.5, pH 5, pH 4.5 or pH 4 or more acidic. Alternatively a polypeptide disclosed herein retains activity under conditions comprising about pH 7, pH 7.5 pH 8.0, pH 8.5, pH 9, pH 9.5, pH 10, pH 10.5 or pH 11 or more basic.

The invention provides immobilized polypeptides of the invention, a polypeptide encoded by a nucleic acid of the invention, or a polypeptide comprising a polypeptide of the invention and a second domain. In one aspect, the polypeptide can be immobilized on a cell, a metal, a resin, a polymer, a ceramic, a glass, a microelectrode, a graphitic particle, a bead, a gel, a plate, an array or a capillary tube.

The present disclosure provides a method of isolating or identifying a polypeptide involved in chlorophyll catabolism or having an esterase (e.g., chlorophyllase) activity, wherein the method comprises the steps of: (a) providing an antibody of the invention; (b) providing a sample comprising polypeptides; and (c) contacting the sample of step (b) with the antibody of step (a) under conditions wherein the antibody can specifically bind to the polypeptide, thereby isolating or identifying the polypeptide.

The present disclosure provides methods of producing a recombinant polypeptide comprising the steps of: (a) providing a nucleic acid of the invention operably linked to a promoter; and (b) expressing the nucleic acid of step (a) under conditions that allow expression of the polypeptide, thereby producing a recombinant polypeptide. IThe method can further comprise transforming a host cell with the nucleic acid of step (a) followed by expressing the nucleic acid of step (a), thereby producing a recombinant polypeptide in a transformed cell.

The present disclosure provides methods for identifying a polypeptide involved in chlorophyll catabolism or having an esterase (e.g., chlorophyllase) activity, comprising the following steps: (a) providing a polypeptidedisclosed herein; or a polypeptide encoded by a nucleic acid disclosed herein; (b) providing an appropriate substrate (e.g., substrate of the polypeptide; and (c) contacting the polypeptide or a fragment or variant thereof of step (a) with the substrate of step (b) and detecting a decrease in the amount of substrate or an increase in the amount of a reaction product, wherein a decrease in the amount of the substrate or an increase in the amount of the reaction product detects a polypeptide involved in chlorophyll catabolism or having an esterase (e.g., chlorophyllase) activity.

The present disclosure provides methods for identifying a substrate of a polypeptide involved in a chlorophyll catabolism or having an esterase (e.g., chlorophyllase) activity, wherein the method comprises the following steps: (a) providing a polypeptide disclosed herein; or a polypeptide encoded by a nucleic acid disclosed herein; (b) providing a test substrate; and (c) contacting the polypeptide of step (a) with the test substrate of step (b) and detecting a decrease in the amount of substrate or an increase in the amount of reaction product, wherein a decrease in the amount of the substrate or an increase in the amount of a reaction product identifies the test substrate a substrate of a polypeptide involved in chlorophyll catabolism or having an esterase (e.g., chlorophyllase) activity.

The present disclosure provides computer systems comprising a processor and a data storage device wherein said data storage device has stored thereon a polypeptide sequence or a nucleic acid sequence of the invention (e.g., a polypeptide encoded by a nucleic aciddisclosed herein). tThe computer system can further comprise a sequence comparison algorithm and a data storage device having at least one reference sequence stored thereon. Alternatively, the sequence comparison algorithm comprises a computer program that indicates polymorphisms. The computer system can further comprise an identifier that identifies one or more features in said sequence. The present disclosure provides computer readable media having stored thereon a polypeptide sequence or a nucleic acid sequence disclosed herein. The present disclosure provides methods for identifying a feature in a sequence comprising the steps of: (a) reading the sequence using a computer program which identifies one or more features in a sequence, wherein the sequence comprises a polypeptide sequence or a nucleic acid sequence of the invention; and (b) identifying one or more features in the sequence with the computer program. The disclosure provides methods for comparing a first sequence to a second sequence comprising the steps of: (a) reading the first sequence and the second sequence through use of a computer program which compares sequences, wherein the first sequence comprises a polypeptide sequence or a nucleic acid sequence of the invention; and (b) determining differences between the first sequence and the second sequence with the computer program. The step of determining differences between the first sequence and the second sequence can further comprise the step of identifying polymorphisms. The method can further comprise an identifier that identifies one or more features in a sequence. The method can comprise reading the first sequence using a computer program and identifying one or more features in the sequence.

The present disclosure provides methods for isolating or recovering a nucleic acid encoding a polypeptide having enzymatic activity involved in chlorophyll catabolism or having an esterase (e.g., chlorophyllase) activity from an environmental sample comprising the steps of: (a) providing an amplification primer sequence pair for amplifying a nucleic acid encoding a polypeptide involved in chlorophyll catabolism or having an esterase (e.g., chlorophyllase) activity, wherein the primer pair is capable of amplifying a nucleic acid disclosed herein; (b) isolating a nucleic acid from the environmental sample or treating the environmental sample such that nucleic acid in the sample is accessible for hybridization to the amplification primer pair; and, (c) combining the nucleic acid of step (b) with the amplification primer pair of step (a) and amplifying nucleic acid from the environmental sample, thereby isolating or recovering a nucleic acid encoding a polypeptide involved in chlorophyll catabolism or having an esterase (e.g., chlorophyllase) activity from an environmental sample. One or each member of the amplification primer pair can comprise an oligonucleotide comprising at least about 10 to 50 or more consecutive bases of a sequence disclosed herein. The amplification primer pair is an amplification pair disclosed herein.

The disclosure provides methods for isolating or recovering a nucleic acid encoding a polypeptide involved in chlorophyll catabolism or having an esterase (e.g., chlorophyllase) activity from an environmental sample comprising the steps of: (a) providing a polynucleotide probe comprising a nucleic acid disclosed hererin or a subsequence thereof; (b) isolating a nucleic acid from the environmental sample or treating the environmental sample such that nucleic acid in the sample is accessible for hybridization to a polynucleotide probe of step (a); (c) combining the isolated, synthetic nucleic acid or the treated environmental sample of step (b) with the polynucleotide probe of step (a); and (d) isolating a nucleic acid that specifically hybridizes with the polynucleotide probe of step (a), thereby isolating or recovering a nucleic acid encoding a polypeptide involved in chlorophyll catabolism or having an esterase (e.g., chlorophyllase) activity from an environmental sample. The environmental sample can comprise a water sample, a liquid sample, a soil sample, an air sample or a biological sample. The biological sample can be derived from a bacterial cell, a protozoan cell, an insect cell, a yeast cell, a plant cell, a fungal cell or a mammalian cell.

The disclosure provides methods of generating a variant of a nucleic acid encoding a polypeptide involved in chlorophyll catabolism or having an esterase (e.g., chlorophyllase) activity comprising the steps of: (a) providing a template nucleic acid comprising a nucleic acid of the invention; and (b) modifying, deleting or adding one or more nucleotides in the template sequence, or a combination thereof, to generate a variant of the template nucleic acid. The method can further comprise expressing the variant nucleic acid to generate a variant polypeptide involved in chlorophyll catabolism or having an esterase (e.g., chlorophyllase) activity. The modifications, additions or deletions can be introduced by a method comprising error-prone PCR, shuffling, oligonucleotide-directed mutagenesis, assembly PCR, sexual PCR mutagenesis, *in vivo* mutagenesis, cassette mutagenesis, recursive ensemble mutagenesis, exponential ensemble mutagenesis, site-specific mutagenesis, gene reassembly, Gene Site Saturation Mutagenesis (GSSM), synthetic ligation reassembly (SLR) or a combination thereof. Alternatively, the modifications, additions or deletions are introduced by a method comprising recombination, recursive sequence recombination, phosphothioate-modified DNA mutagenesis, uracil-containing template mutagenesis, gapped duplex mutagenesis, point mismatch repair mutagenesis, repair-deficient host strain mutagenesis, chemical mutagenesis, radiogenic mutagenesis, deletion mutagenesis, restriction-selection mutagenesis, restriction-purification mutagenesis, artificial gene synthesis, ensemble mutagenesis, chimeric nucleic acid multimer creation and a combination thereof.

The method can be iteratively repeated until a polypeptide involved in chlorophyll catabolism or having an esterase (e.g., chlorophyllase) activity having an altered or different activity or an altered or different stability from that of a polypeptide encoded by the template nucleic acid is produced. The variant polypeptide is thermotolerant, and retains some activity after being exposed to an elevated temperature. Alternatively, the variant polypeptide has increased glycosylation as compared to the polypeptide encoded by a template nucleic acid. Alternatively, the variant polypeptide has activity under a high (or higher) temperature, wherein the enzyme encoded by the template nucleic acid is not active under the high temperature. The method can be iteratively repeated until an enzyme coding sequence having an altered codon usage from that of the template nucleic acid is produced. The method can be iteratively repeated until an enzyme-encoding gene having higher or lower level of message expression or stability from that of the template nucleic acid is produced.

The disclosure provides methods of increasing thermotolerance or thermostability of a polypeptide disclosed herein, or, a polypeptide encoded by a nucleic aciddisclosed herein, the method comprising glycosylating a polypeptide comprising at least thirty contiguous amino acids of a polypeptideof the present disclosure; or a polypeptide encoded by a nucleic acid sequence of the invention, thereby increasing the thermotolerance or thermostability of the polypeptide. The specific activity can be thermostable or thermotolerant at a temperature in the range from greater than about 37°C to about 95°C.

The disclosure provides methods for overexpressing a recombinant polypeptide in a cell comprising expressing a vector comprising a nucleic acid comprising a nucleic acid disclosed herein or a nucleic acid sequence disclosed herein, wherein the sequence identities are determined by analysis with a sequence comparison algorithm or by visual inspection, wherein overexpression is effected by use of a high activity promoter, a dicistronic vector or by gene amplification of the vector.

The disclosure provides methods of expressing a heterologous nucleic acid sequence in a plant cell comprising the following steps: (a) transforming the plant cell with a heterologous nucleic acid sequence operably linked to a promoter, wherein the heterologous nucleic sequence comprises a nucleic acid disclosed herein; (b) growing the plant under conditions wherein the heterologous nucleic acids sequence is expressed in the plant cell. The invention provides methods of expressing a heterologous nucleic acid sequence in a plant cell comprising the following steps: (a) transforming the plant cell with a heterologous nucleic acid sequence operably linked to a promoter, wherein the heterologous nucleic sequence comprises a sequence of the invention; (b) growing the plant under conditions wherein the heterologous nucleic acids sequence is expressed in the plant cell. Alternatively, an enzyme used in the invention can be prepared by expression of a polynucleotide disclosed herein in an organism such as a bacterium, a yeast, a plant, an insect, a fungus or an animal. Exemplary organisms for expressing polypeptides of the invention can be *S. pombe, S. cerevisiae, Pichia* sp., e.g., *P. pastoris, E. coli, Streptomyces* sp., *Bacillus* sp. and *Lactobacillus* sp.

Also disclosed herein is a method of making a polypeptide disclosed herein. The method includes introducing a nucleic acid encoding the polypeptide into a host cell, wherein the nucleic acid is operably linked to a promoter and culturing the host cell under conditions that allow expression of the nucleic acid. Alternatively a method of making a polypeptide or peptideis disclosed. The method includes introducing a nucleic acid encoding the polypeptide into a host cell, wherein the nucleic acid is operably linked to a promoter and culturing the host cell under conditions that allow expression of the nucleic acid, thereby producing the polypeptide.

Also disclosed is a method of generating a variant including obtaining a nucleic acid having a sequence disclosed herein, sequences substantially identical thereto, sequences complementary to a sequence of the invention, and fragments thereof, and changing one or more nucleotides in the sequence to another nucleotide, deleting one or more nucleotides in the sequence, or adding one or more nucleotides to the sequence.

The disclosure provides biosynthetic systems for the catabolism of chlorophyll comprising at least one enzyme disclosed herein. The disclosure provides biosynthetic systems for the catabolism of chlorophyll comprising at least one nucleic acid encoding an enzyme involved in the catabolism of chlorophyll, wherein the nucleic acid comprises a sequence disclosed herein. The system comprises a plurality of enzyme-encoding nucleic acids, wherein the enzymes are involved in the catabolism of chlorophyll. The plurality of enzyme-encoding nucleic acids comprises all of the enzymes in a chlorophyll catabolism pathway. The plurality of enzyme-encoding nucleic acids are contained in at least one plasmid, expression cassette or expression vector.

The biosynthetic system of the invention is contained in (comprises) a cell. The cell can be a bacterial cell, a mammalian cell, a fungal cell, a yeast cell, an insect cell or a plant cell. The yeast cell can be a *Pichia* sp. or a *Saccharomyces* sp., such as a *Pichia pastoris, Saccharomyces cerevisiae* or *Schizosaccharomyces pombe.*

The details of one or more embodiments of the invention are set forth in the accompanying drawings and the description below. Other features, objects, and advantages of the invention will be apparent from the description and drawings, and from the claims.

### DESCRIPTION OF DRAWINGS

Figure 1 is a schematic representation of chlorophyll (Figure 1A), phytol (Figure 1B) and chlorophyllide (Figure 1C).
Figure 2 and Figure 3 illustrate data showing the results of an esterase (chlorophyllase activity) activity assay using exemplary enzymes of the invention, as described in detail in Example 1, below.

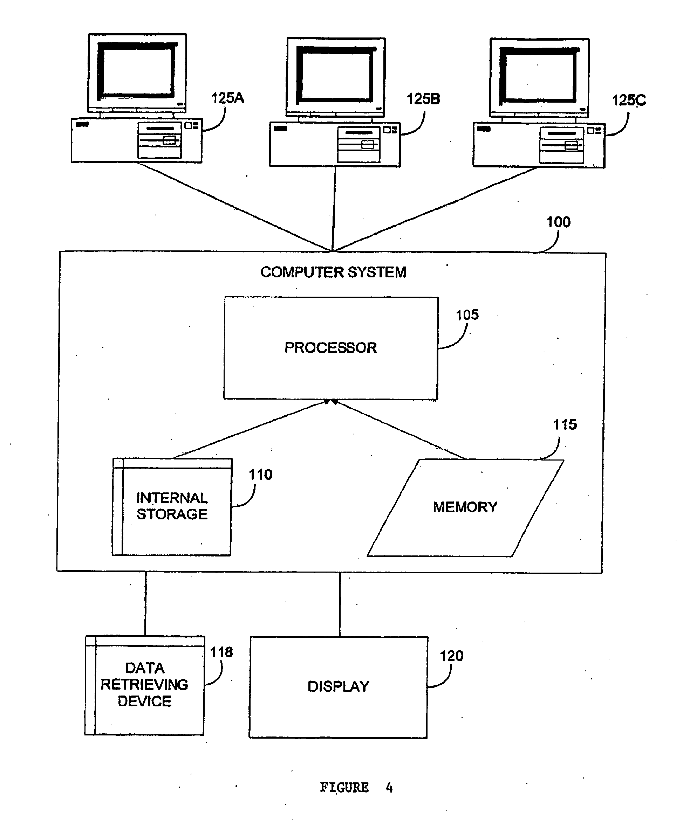

Figure 4 is a block diagram of an exemplary computer system of the invention, as described in detail, below.
Figure 5 is a flow diagram illustrating a process of the invention for comparing a new nucleotide or protein sequence with a database of sequences in order to determine the homology levels between the new sequence and the sequences in the database, as described in detail, below.
Figure 6 is a flow diagram illustrating a process in a computer for determining whether two sequences are homologous, as described in detail, below.
Figure 7 is a flow diagram illustrating an identifier process 300 for detecting the presence of a feature in a sequence, as described in detail, below.
Figure 8 illustrates the reaction of an exemplary esterase of the invention in chlorophyll degradation, as described in detail, below.
Figure 9 illustrates and compares traditional versus an exemplary enzymatic decoloring (bleaching) reaction of the invention, as described in detail, below.
Figure 10 illustrates an exemplary enzymatic decoloring (bleaching) reaction of the invention, as described in detail, below.
Figure 11 illustrates an exemplary enzymatic decoloring (bleaching) process of the invention that combines degumming, enzymatic bleaching ("decoloring") and caustic neutralization steps, as described in detail, below.
Figure 12 illustrates an exemplary enzymatic decoloring (bleaching) process of the invention as described in detail, below.
Figure 13 illustrates an exemplary oilseed refining scheme comprising extraction, refining and modification of an oilseed using an esterase of the invention, as described in detail, below.
Figure 14 illustrates an exemplary industrial process of the invention - a biodegumming process, comprising use of at least one polypeptide of the invention, as described in detail, below.
Figure 15 illustrates another exemplary industrial process of the invention comprising use of at least one polypeptide of the invention, as described in detail, below.
Figure 16 illustrates another exemplary industrial process of the invention comprising use of at least one polypeptide of the invention having chlorophyllase enzyme activity.

Like reference symbols in the various drawings indicate like elements.

### DETAILED DESCRIPTION

The present invention provides novel compositions and processes for the enzymatic treatment ("bleaching" or "de-colorizing") of chlorophyll-containing or chlorophyll-contaminated compositions, e.g., plant, animal or algal preparations, foods, feeds or oils. In one aspect, the treatment (or, "enzymatic bleaching" or "de-colorizing") of chlorophyll used in the compositions and methods of the invention comprises use of a chlorophyllase enzyme, or other enzyme involved in chlorophyll catabolism, to modify chlorophyll, e.g., to facilitate removal of the color-bearing porphyrin ring by, e.g., aqueous extraction. Chlorophyllase catalyzes the hydrolysis of chlorophyll to generate chlorophyllide, which can be aqueous extracted, and phytol, which remains in the oil phase.

For example, in one aspect, the invention provides compositions and processes for the enzymatic processing (e.g., hydrolysis) of chlorophyll in a feed, food or oil, e.g., a vegetable oils, including oils processed from oilseeds, such as canola (rapeseed) oil or soybean oil, or oil fruits, such as palm oil. In one aspect, the invention provides enzymatic bleaching methods using a chlorophyllase enzyme for the enzymatic hydrolysis of a chlorophyll or any color-bearing porphyrin ring in an animal or a plant oil, e.g., vegetable oils.

The invention includes methods for enzymatically treating (e.g., "bleaching") chlorophyll-containing foods or oils via *in vitro* or *in vivo* techniques, e.g., whole cells protocols, such as fermentation or other biocatalytic processes.

### Generating and Manipulating Nucleic Acids

The disclosure provides isolated, recombinant and synthetic nucleic acids (e.g., an exemplary nucleic acid of the invention, including SEQ ID NO:1, SEQ ID NO:3, SEQ ID NO:5, SEQ ID NO:7, SEQ ID NO:9, SEQ ID NO:11, SEQ ID NO:13, SEQ ID NO:15, SEQ ID NO:17 or SEQ ID NO:19), and sequences having a sequence identity to an exemplary nucleic acid; nucleic acids encoding polypeptides of the invention, e.g., the exemplary amino acid sequences as set forth in SEQ ID NO:2, SEQ ID NO:4, SEQ ID NO:6, SEQ ID NO:8, SEQ ID NO:10, SEQ ID NO:12, SEQ ID NO:14, SEQ ID NO:16, SEQ ID NO:18 or SEQ ID NO:20), such as nucleic acids encoding a polypeptide of SEQ ID NO: 10 used in the present invention. Also disclosed are expression cassettes such as expression vectors, comprising nucleic acids of the invention, which include polynucleotides which encode the polypeptides of the invention. The disclosure also shows methods for discovering new polypeptide sequences using the nucleic acids of the invention. The disclosure also includes methods for inhibiting the expression of genes, transcripts and polypeptides using the nucleic acids of the invention. Also provided are methods for modifying the nucleic acids of the invention by, e.g., synthetic ligation reassembly, optimized directed evolution system and/or saturation mutagenesis.

The nucleic acids used in the invention can be made, isolated and/or manipulated by, e.g., cloning and expression of cDNA libraries, amplification of message or genomic DNA by PCR, and the like.

In practicing the methods of the invention, homologous genes can be modified by manipulating a template nucleic acid, as described herein. The invention can be practiced in conjunction with any method or protocol or device known in the art, which are well described in the scientific and patent literature.

The disclosure also provides an isolated nucleic acid comprising one of the sequences of the invention, or a fragment comprising at least 10, 15, 20, 25, 30, 35, 40, 50, 75, 100, 150, 200, 300, 400, or 500 consecutive bases of a nucleic acid of the invention. The isolated, nucleic acids may comprise DNA, including cDNA, genomic DNA and synthetic DNA. The DNA may be double-stranded or single-stranded and if single stranded may be the coding strand or non-coding (anti-sense) strand. Alternatively, the isolated nucleic acids may comprise RNA.

The isolated nucleic acids disclosed herein may be used to prepare one of the polypeptides for use according to the invention, or fragments comprising at least 5, 10, 15, 20, 25, 30, 35, 40, 50, 75, 100, or 150 consecutive amino acids of one of the polypeptides of the invention.

The isolated nucleic acid which encodes one of the polypeptides of the invention, but is not limited to: only the coding sequence of a nucleic acid of the invention and additional coding sequences, such as leader sequences or proprotein sequences and non-coding sequences, such as introns or non-coding sequences 5' and/or 3' of the coding sequence. Thus, as used herein, the term "polynucleotide encoding a polypeptide" encompasses a polynucleotide which includes only the coding sequence for the polypeptide as well as a polynucleotide which includes additional coding and/or non-coding sequence.

Alternatively, the nucleic acid sequences for a use according to the invention, may be mutagenized using conventional techniques, such as site directed mutagenesis, or other techniques familiar to those skilled in the art, to introduce silent changes into the polynucleotides o of the invention. As used herein, "silent changes" include, for example, changes which do not alter the amino acid sequence encoded by the polynucleotide. Such changes may be desirable in order to increase the level of the polypeptide produced by host cells containing a vector encoding the polypeptide by introducing codons or codon pairs which occur frequently in the host organism.

As used herein, the term "isolated" means that the material is removed from its original environment (*e.g*., the natural environment if it is naturally occurring). For example, a naturally-occurring polynucleotide or polypeptide present in a living animal is not isolated, but the same polynucleotide or polypeptide, separated from some or all of the coexisting materials in the natural system, is isolated. Such polynucleotides could be part of a vector and/or such polynucleotides or polypeptides could be part of a composition and still be isolated in that such vector or composition is notpart of its natural environment. As used herein, the term "purified" does not require absolute purity; rather, it is intended as a relative definition. Individual nucleic acids obtained from a library have been conventionally purified to electrophoretic homogeneity. The sequences obtained from these clones could not be obtained directly either from the library or from total human DNA. The purified nucleic acids of the invention have been purified from the remainder of the genomic DNA in the organism by at least 10⁴-10⁶ fold. However, the term "purified" also includes nucleic acids which have been purified from the remainder of the genomic DNA or from other sequences in a library or other environment by at least one order of magnitude, typically two or three orders and more typically four or five orders of magnitude.

As used herein, the term "recombinant" means that the nucleic acid is adjacent to a "backbone" nucleic acid to which it is not adjacent in its natural environment. Additionally, to be "enriched" the nucleic acids will represent 5% or more of the number of nucleic acid inserts in a population of nucleic acid backbone molecules. Backbone molecules according to the invention include nucleic acids such as expression vectors, self-replicating nucleic acids, viruses, integrating nucleic acids and other vectors or nucleic acids used to maintain or manipulate a nucleic acid insert of interest. Typically, the enriched nucleic acids represent 15% or more of the number of nucleic acid inserts in the population of recombinant backbone molecules. More typically, the enriched nucleic acids represent 50% or more of the number of nucleic acid inserts in the population of recombinant backbone molecules. The enriched nucleic acids represent 90% or more of the number of nucleic acid inserts in the population of recombinant backbone molecules.

"Recombinant" polypeptides or proteins refer to polypeptides or proteins produced by recombinant DNA techniques; i.e., produced from cells transformed by an exogenous DNA construct encoding the desired polypeptide or protein. "Synthetic" polypeptides or protein are those prepared by chemical synthesis. Solid-phase chemical peptide synthesis methods can also be used to synthesize the polypeptide or fragments of the invention. Such method have been known in the art since the early 1960's (Merrifield, R. B., J. Am. Chem. Soc., 85:2149-2154, 1963) (See also Stewart, J. M. and Young, J. D., Solid Phase Peptide Synthesis, 2nd Ed., Pierce Chemical Co., Rockford, III., pp. 11-12)) and have recently been employed in commercially available laboratory peptide design and synthesis kits (Cambridge Research Biochemicals). Such commercially available laboratory kits have generally utilized the teachings of H. M. Geysen et al, Proc. Natl. Acad. Sci., USA, 81:3998 (1984) and provide for synthesizing peptides upon the tips of a multitude of "rods" or "pins" all of which are connected to a single plate. When such a system is utilized, a plate of rods or pins is inverted and inserted into a second plate of corresponding wells or reservoirs, which contain solutions for attaching or anchoring an appropriate amino acid to the pin's or rod's tips. By repeating such a process step, i.e., inverting and inserting the rod's and pin's tips into appropriate solutions, amino acids are built into desired peptides. In addition, a number of available FMOC peptide synthesis systems are available. For example, assembly of a polypeptide or fragment can be carried out on a solid support using an Applied Biosystems, Inc. Model 431A automated peptide synthesizer. Such equipment provides ready access to the peptides of the invention, either by direct synthesis or by synthesis of a series of fragments that can be coupled using other known techniques.

The term "variant" refers to polynucleotides or polypeptides of the invention modified at one or more base pairs, codons, introns, exons, or amino acid residues (respectively) yet still retain the biological activity of an enzyme of the invention. Variants can be produced by any number of means included methods such as, for example, error-prone PCR, shuffling, oligonucleotide-directed mutagenesis, assembly PCR, sexual PCR mutagenesis, *in vivo* mutagenesis, cassette mutagenesis, recursive ensemble mutagenesis, exponential ensemble mutagenesis, site-specific mutagenesis, gene reassembly, GSSM and any combination thereof.

The term "Saturation Mutagenesis" or "Gene Site Saturation Mutagenesis" or "GSSM" includes a method that uses degenerate oligonucleotide primers to introduce point mutations into a polynucleotide, as described in detail, below.

### General Techniques

The present invention provides novel compositions and processes for enzymatically treating (e.g., "bleaching") chlorophyll-containing compositions such as plants, algae, foods or oils. The skilled artisan will recognize that compounds used in the methods of the invention (e.g., catalytic, starting or intermediate compounds) can be synthesized using a variety of procedures and methodologies, which are well described in the scientific and patent literature., *e.g*., Organic Syntheses Collective Volumes, Gilman et al. (Eds) John Wiley & Sons, Inc., NY; Venuti (1989) Pharm Res. 6:867-873. The invention can be practiced in conjunction with any method or protocol known in the art, which are well described in the scientific and patent literature.

The nucleic acids used to practice this invention, whether RNA, iRNA, antisense nucleic acid, cDNA, genomic DNA, vectors, viruses or hybrids thereof, may be isolated from a variety of sources, genetically engineered, amplified, and/or expressed/ generated recombinantly. Recombinant polypeptides (e.g., enzymes of the invention) generated from these nucleic acids can be individually isolated or cloned and tested for a desired activity. Any recombinant expression system can be used, including bacterial, mammalian, yeast, insect or plant cell expression systems.

Alternatively, these nucleic acids can be synthesized *in vitro* by well-known chemical synthesis techniques, as described in, e.g., Adams (1983) J. Am. Chem. Soc. 105:661; Belousov (1997) Nucleic Acids Res. 25:3440-3444; Frenkel (1995) Free Radic. Biol. Med. 19:373-380; Blommers (1994) Biochemistry 33:7886-7896; Narang (1979) Meth. Enzymol. 68:90; Brown (1979) Meth. Enzymol. 68:109; Beaucage (1981) Tetra. Lett. 22:1859; U.S. Patent No. 4,458,066.

Techniques for the manipulation of nucleic acids, such as, e.g., subcloning, labeling probes (e.g., random-primer labeling using Klenow polymerase, nick translation, amplification), sequencing, hybridization and the like are well described in the scientific and patent literature, see, e.g., Sambrook, ed., MOLECULAR CLONING: A LABORATORY MANUAL (2ND ED.), Vols. 1-3, Cold Spring Harbor Laboratory, (1989); CURRENT PROTOCOLS IN MOLECULAR BIOLOGY, Ausubel, ed. John Wiley & Sons, Inc., New York (1997); LABORATORY TECHNIQUES IN BIOCHEMISTRY AND MOLECULAR BIOLOGY: HYBRIDIZATION WITH NUCLEIC ACID PROBES, Part I. Theory and Nucleic Acid Preparation, Tijssen, ed. Elsevier, N.Y. (1993).

Another useful means of obtaining and manipulating nucleic acids used to practice the methods of the invention is to clone from genomic samples, and, if desired, screen and re-clone inserts isolated or amplified from, e.g., genomic clones or cDNA clones. Sources of nucleic acid used in the methods of the invention include genomic or cDNA libraries contained in, e.g., mammalian artificial chromosomes (MACs), see, e.g., U.S. Patent Nos. 5,721,118; 6,025,155; human artificial chromosomes, see, e.g., Rosenfeld (1997) Nat. Genet. 15:333-335; yeast artificial chromosomes (YAC); bacterial artificial chromosomes (BAC); P1 artificial chromosomes, see, e.g., Woon (1998) Genomics 50:306-316; P1-derived vectors (PACs), see, e.g., Kern (1997) Biotechniques 23:120-124; cosmids, recombinant viruses, phages or plasmids.

The present invention provides novel compositions and processes for enzymatically treating (e.g., "bleaching") chlorophyll-containing compositions such as plants, algae, foods or oils. The skilled artisan will recognize that compounds used in the methods of the invention (e.g., catalytic, starting or intermediate compounds) can be synthesized using a variety of procedures and methodologies, which are well described in the scientific and patent literature., e.g., Organic Syntheses Collective Volumes; Gilman et al. (Eds) John Wiley & Sons, Inc., NY; Venuti (1989) Pharm Res. 6:867-873. The invention can be practiced in conjunction with any method or protocol known in the art, which are well described in the scientific and patent literature.

A nucleic acid encoding a polypeptide for use according to the invention is assembled in appropriate phase with a leader sequence capable of directing secretion of the translated polypeptide or fragment thereof.

### Transcriptional and translational control sequences

The disclosure provides nucleic acid (e.g., DNA) sequences of the invention operatively linked to expression (e.g., transcriptional or translational) control sequence(s), e.g., promoters or enhancers, to direct or modulate RNA synthesis/ expression. The expression control sequence can be in an expression vector. Exemplary bacterial promoters include lacI, lacZ, T3, T7, gpt, lambda PR, PL and trp. Exemplary eukaryotic promoters include CMV immediate early, HSV thymidine kinase, early and late SV40, LTRs from retrovirus, and mouse metallothionein I.

### Expression vectors and cloning vehicles

Expression vectors and cloning vehicles are provided comprising nucleic acids for use according to the invention, e.g., sequences encoding the enzymes of the invention. Expression vectors and cloning vehicles of the invention can comprise viral particles, baculovirus, phage, plasmids, phagemids, cosmids, fosmids, bacterial artificial chromosomes, viral DNA (e.g., vaccinia, adenovirus, foul pox virus, pseudorabies and derivatives of SV40), P1-based artificial chromosomes, yeast plasmids, yeast artificial chromosomes, and any other vectors specific for specific hosts of interest (such as *Bacillus, Aspergillus* and yeast). Vectors can include chromosomal, non-chromosomal and synthetic DNA sequences. Large numbers of suitable vectors are known to those of skill in the art, and are commercially available. The expression vector can comprise a promoter, a ribosome binding site for translation initiation and a transcription terminator. The vector may also include appropriate sequences for amplifying expression.

The expression vectors contain one or more selectable marker genes to permit selection of host cells containing the vector. Such selectable markers include genes encoding dihydrofolate reductase or genes conferring neomycin resistance for eukaryotic cell culture, genes conferring tetracycline or ampicillin resistance in *E. coli,* and the *S. cerevisiae* TRP1 gene. Promoter regions can be selected from any desired gene using chloramphenicol transferase (CAT) vectors or other vectors with selectable markers.

The nucleic acids for use according to the invention can be expressed in expression cassettes, vectors or viruses and transiently or stably expressed in plant cells and seeds. One exemplary transient expression system uses episomal expression systems, e.g., cauliflower mosaic virus (CaMV) viral RNA generated in the nucleus by transcription of an episomal mini-chromosome containing supercoiled DNA, see, e.g., Covey (1990) Proc. Natl. Acad. Sci. USA 87:1633-1637. Expression vectors capable of expressing nucleic acids and proteins in plants are well known in the art, and can include, *e.g.,* vectors from *Agrobacterium* spp., potato virus X (see, *e.g*., Angell (1997) EMBO J. 16:3675-3684), tobacco mosaic virus (see, *e.g*., Casper (1996) Gene 173:69-73), tomato bushy stunt virus (see, *e.g*., Hillman (1989) Virology 169:42-50), tobacco etch virus (see, *e.g*., Dolja (1997) Virology 234:243-252), bean golden mosaic virus (see, *e.g*., Morinaga (1993) Microbiol Immunol. 37:471-476), cauliflower mosaic virus (see, *e.g.,* Cecchini (1997) Mol. Plant Microbe Interact. 10:1094-1101), maize Ac/Ds transposable element (see, *e.g*., Rubin (1997) Mol. Cell. Biol. 17:6294-6302; Kunze (1996) Curr. Top. Microbiol. Immunol. 204:161-194), and the maize suppressor-mutator (Spm) transposable element (see, *e.g*., Schlappi (1996) Plant Mol. Biol. 32:717-725); and derivatives thereof.

The DNA sequence in the expression vector is operatively linked to an appropriate expression control sequence(s) (promoter) to direct RNA synthesis. Particular named bacterial promoters include *lacI, lacZ, T3, T7, gpt, lambda P_{R}, P_{L}* and *trp.* Eukaryotic promoters include CMV immediate early, HSV thymidine kinase, early and late SV40, LTRs from retrovirus and mouse metallothionein-I. Selection of the appropriate vector and promoter is well within the level of ordinary skill in the art. The expression vector also contains a ribosome binding site for translation initiation and a transcription terminator. The vector may also include appropriate sequences for amplifying expression. Promoter regions can be selected from any desired gene using chloramphenicol transferase (CAT) vectors or other vectors with selectable markers. In addition, the expression vectors contain one or more selectable marker genes to provide a phenotypic trait for selection of transformed host cells such as dihydrofolate reductase or neomycin resistance for eukaryotic cell culture, or such as tetracycline or ampicillin resistance in *E. coli.*

In addition, the expression vectors typically contain one or more selectable marker genes to permit selection of host cells containing the vector. Such selectable markers include genes encoding dihydrofolate reductase or genes conferring neomycin resistance for eukaryotic cell culture, genes conferring tetracycline or ampicillin resistance in *E. coli* and the *S. cerevisiae TRP1* gene.

The nucleic acid encoding one of the polypeptides used in the invention, or fragments comprising at least about 5, 10, 15, 20, 25, 30, 35, 40, 50, 75, 100, or 150 consecutive amino acids thereof is assembled in appropriate phase with a leader sequence capable of directing secretion of the translated polypeptide or fragment thereof. Optionally, the nucleic acid can encode a fusion polypeptide in which one of the polypeptides of the invention, or fragments comprising at least 5, 10, 15, 20, 25, 30, 35, 40, 50, 75, 100, or 150 consecutive amino acids thereof is fused to heterologous peptides or polypeptides, such as N-terminal identification peptides which impart desired characteristics, such as increased stability or simplified purification.

The appropriate DNA sequence may be inserted into the vector by a variety of procedures. In general, the DNA sequence is ligated to the desired position in the vector following digestion of the insert and the vector with appropriate restriction endonucleases. Alternatively, blunt ends in both the insert and the vector may be ligated. A variety of cloning techniques are disclosed in Ausubel et al. Current Protocols in Molecular Biology, John Wiley 503 Sons, Inc. 1997 and Sambrook et al., Molecular Cloning: A Laboratory Manual 2nd Ed., Cold Spring Harbor Laboratory Press (1989. Such procedures and others are deemed to be within the scope of those skilled in the art.

A variety of cloning and expression vectors for use with prokaryotic and eukaryotic hosts are described by Sambrook, et al., Molecular Cloning: A Laboratory Manual, 2nd Ed., Cold Spring Harbor, N.Y., (1989).

### Host cells and transformed cells

The disclosure also provides a transformed cell comprising a nucleic acid sequence of the invention, e.g., a sequence encoding an enzyme of the invention, or a vector of the invention. The host cell may be any of the host cells familiar to those skilled in the art, including prokaryotic cells, eukaryotic cells, such as bacterial cells, fungal cells, yeast cells, mammalian cells, insect cells, or plant cells. Exemplary bacterial cells include *E. coli, Lactococcus lactis, Streptomyces, Bacillus subtilis, Bacillus cereus, Salmonella typhimurium* or any species within the genera *Bacillus, Streptomyces* and *Staphylococcus.* Exemplary insect cells include *Drosophila* S2 and *Spodoptera* Sf9. Exemplary yeast cells include *Pichia pastoris, Saccharomyces cerevisiae* or *Schizosaccharomyces pombe.* Exemplary animal cells include CHO, COS or Bowes melanoma or any mouse or human cell line. The selection of an appropriate host is within the abilities of those skilled in the art. Techniques for transforming a wide variety of higher plant species are well known and described in the technical and scientific literature. See, e.g., Weising (1988) Ann. Rev. Genet. 22:421-477; U.S. Patent No. 5,750,870.

The vector can be introduced into the host cells using any of a variety of techniques, including transformation, transfection, transduction, viral infection, gene guns, or Ti-mediated gene transfer. Particular methods include calcium phosphate transfection, DEAE-Dextran mediated transfection, lipofection, or electroporation (Davis, L., Dibner, M., Battey, I., Basic Methods in Molecular Biology, (1986)).

The nucleic acids or vectors as disclosed herein are introduced into the cells for screening, thus, the nucleic acids enter the cells in a manner suitable for subsequent expression of the nucleic acid. The method of introduction is largely dictated by the targeted cell type. Exemplary methods include CaPO₄ precipitation, liposome fusion, lipofection (e.g., LIPOFECTIN™), electroporation, viral infection, etc. The candidate nucleic acids may stably integrate into the genome of the host cell (for example, with retroviral introduction) or may exist either transiently or stably in the cytoplasm (i.e. through the use of traditional plasmids, utilizing standard regulatory sequences, selection markers, etc.). As many pharmaceutically important screens require human or model mammalian cell targets, retroviral vectors capable of transfecting such targets can be used.

Where appropriate, the engineered host cells can be cultured in conventional nutrient media modified as appropriate for activating promoters, selecting transformants or amplifying the genes of the invention. Following transformation of a suitable host strain and growth of the host strain to an appropriate cell density, the selected promoter may be induced by appropriate means (e.g., temperature shift or chemical induction) and the cells may be cultured for an additional period to allow them to produce the desired polypeptide or fragment thereof.

Host cells containing the polynucleotides of interest, e.g., nucleic acids of the invention, can be cultured in conventional nutrient media modified as appropriate for activating promoters, selecting transformants or amplifying genes. The culture conditions, such as temperature, pH and the like, are those previously used with the host cell selected for expression and will be apparent to the ordinarily skilled artisan. The clones which are identified as having the specified enzyme activity may then be sequenced to identify the polynucleotide sequence encoding an enzyme having the enhanced activity.

The disclosure provides a method for overexpressing a recombinant enzyme in a cell comprising expressing a vector comprising a nucleic acid for use according tothe invention, e.g., a nucleic acid comprising a nucleic acid sequence with at least about 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81 %, 82%, 83%, 84%, 85%, 86%, 87%, 88%, 89%, 90%, 91%, 92%, 93%, 94%, 95%, 96%, 97%, 98%, 99%, or more sequence identity to an exemplary sequence of the invention, such as SEQ ID NO: 9 over a region of at least about 50, 75, 100, 150, 200, 250, 300, 350, 400, 450, 500, 550, 600, 650, 700, 750, 800, 850, 900, 950, 1000, 1050, 1100, 1150 or more residues, or the full length of a gene or a transcript, wherein the sequence identities are determined by analysis with a sequence comparison algorithm or by visual inspection, or, a nucleic acid that hybridizes under stringent conditions to a nucleic acid sequence of the invention. The overexpression can be effected by any means, e.g., use of a high activity promoter, a dicistronic vector or by gene amplification of the vector. Over-expression can be effected by appropriate choice of promoters, enhancers, vectors (e.g., use of replicon vectors, dicistronic vectors (see, e.g., Gurtu (1996) Biochem. Biophys. Res. Commun. 229:295-8), media, culture systems and the like. Gene amplification using selection markers, e.g., glutamine synthetase (see, e.g., Sanders (1987) Dev. Biol. Stand. 66:55-63), in cell systems are used to overexpress the polypeptides for use according to the invention.

The host cell may be any of the host cells familiar to those skilled in the art, including prokaryotic cells, eukaryotic cells, mammalian cells, insect cells, or plant cells. As representative examples of appropriate hosts, there may be mentioned: bacterial cells, such as *E. coli*, *Streptomyces, Bacillus subtilis, Salmonella typhimurium* and various species within the genera Streptomyces and Staphylococcus, fungal cells, such as yeast, insect cells such as *Drosophila* S2 and *Spodoptera Sf9,* animal cells such as CHO, COS or Bowes melanoma and adenoviruses. The selection of an appropriate host is within the abilities of those skilled in the art.

The vector may be introduced into the host cells using any of a variety of techniques, including transformation, transfection, transduction, viral infection, gene guns, or Ti-mediated gene transfer. Particular methods include calcium phosphate transfection, DEAE-Dextran mediated transfection, lipofection, or electroporation (Davis, L., Dibner, M., Battey, I., Basic Methods in Molecular Biology, (1986)).

The constructs in host cells can be used in a conventional manner to produce the gene product encoded by the recombinant sequence. Depending upon the host employed in a recombinant production procedure, the polypeptides produced by host cells containing the vector may be glycosylated or may be non-glycosylated. Polypeptides of the invention may or may not also include an initial methionine amino acid residue.

### Amplification of Nucleic Acids

In practicing the invention, nucleic acids for use according to the invention and nucleic acids encoding enzymes for use according to the invention, or modified nucleic acids for use according to the invention, can be reproduced by amplification. Amplification can also be used to clone or modify the nucleic acids of the invention. Thus, the disclosure provides amplification primer sequence pairs for amplifying nucleic acids of the invention. One of skill in the art can design amplification primer sequence pairs for any part of or the full length of these sequences.

The skilled artisan can select and design suitable oligonucleotide amplification primers. Amplification methods are also well known in the art, and include, e.g., polymerase chain reaction, PCR (see, e.g., PCR PROTOCOLS, A GUIDE TO METHODS AND APPLICATIONS, ed. Innis, Academic Press, N.Y. (1990) and PCR STRATEGIES (1995), ed. Innis, Academic Press, Inc., N.Y., ligase chain reaction (LCR) (see, e.g., Wu (1989) Genomics 4:560; Landegren (1988) Science 241:1077; Barringer (1990) Gene 89:117); transcription amplification (see, e.g., Kwoh (1989) Proc. Natl. Acad. Sci. USA 86:1173); and, self-sustained sequence replication (see, e.g., Guatelli (1990) Proc. Natl. Acad. Sci. USA 87:1874); Q Beta replicase amplification (see, e.g., Smith (1997) J. Clin. Microbiol. 35:1477-1491), automated Q-beta replicase amplification assay (see, e.g., Burg (1996) Mol. Cell. Probes 10:257-271) and other RNA polymerase mediated techniques (e.g., NASBA, Cangene, Mississauga, Ontario); see also Berger (1987) Methods Enzymol. 152:307-316; Sambrook; Ausubel; U.S. Patent Nos. 4,683,195 and 4,683,202; Sooknanan (1995) Biotechnology 13:563-564.

### Determining the degree of sequence identity

The disclosure provides nucleic acids comprising sequences having at least about 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81 %, 82%, 83%, 84%, 85%, 86%, 87%, 88%, 89%, 90%, 91%, 92%, 93%, 94%, 95%, 96%, 97%, 98%, 99%, or more, or complete (100%) sequence identity to an exemplary nucleic acid of the invention (e.g., SEQ ID NO:1, SEQ ID NO:3, SEQ ID NO:5, SEQ ID NO:7, SEQ ID NO:9, SEQ ID NO:11, SEQ ID NO:13, SEQ ID NO:15, SEQ ID NO:17 or SEQ ID NO:19) over a region of at least about 10, 20, 30, 40, 50, 60, 70, 75, 100, 150, 200, 250, 300, 350, 400, 450, 500, 550, 600, 650, 700, 750, 800, 850, 900, 950, 1000, 1050, 1100, 1150, 1200, 1250, 1300, 1350, 1400, 1450, 1500, 1550 or more, residues. A nucleic acid comprising a sequence at least about 90%, 91%, 92%, 93%, 94%, 95%, 96%, 97%, 98%, 99%, or more, or complete (100%) sequence identity to nucleic acid of SEQ ID NO:9 is used according to the invention The disclosure provides polypeptides comprising sequences having at least about 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or more, or complete (100%) sequence identity to an exemplary polypeptide disclosed herein. A polypeptide having at least 90%, 91%, 92%, 93%, 94%, 95%, 96%, 97%, 98%, 99% sequence identity to SEQ ID NO: 10 is used in the invention. The extent of sequence identity (homology) may be determined using any computer program and associated parameters, including those described herein, such as BLAST 2.2.2. or FASTA version 3.0t78, with the default parameters.

Nucleic acid sequences in a use according to the invention can comprise at least 10, 15, 20, 25, 30, 35, 40, 50, 75, 100, 150, 200, 300, 400, or 500 consecutive nucleotides of an exemplary sequence of the invention and sequences substantially identical thereto. Homologous sequences and fragments of nucleic acid sequences of the invention can refer to a sequence having at least 99%, 98%, 97%, 96%, 95%, 90%, 85%, 80%, 75%, 70%, 65%, 60%, 55%, or 50% homology to these sequences. Homology may be determined using any of the computer programs and parameters described herein, including FASTA version 3.0t78 with the default parameters. Homologous sequences also include RNA sequences in which uridines replace the thymines in the nucleic acid sequences of the invention. The homologous sequences may be obtained using any of the procedures described herein or may result from the correction of a sequencing error. It will be appreciated that the nucleic acid sequences of the invention can be represented in the traditional single character format (See the inside back cover of Stryer, Lubert. Biochemistry, 3rd Ed., W. H Freeman & Co., New York.) or in any other format which records the identity of the nucleotides in a sequence.

Various sequence comparison programs identified elsewhere in this patent specification are particularly contemplated for use in this part of the disclosure. Protein and/or nucleic acid sequence homologies may be evaluated using any of the variety of sequence comparison algorithms and programs known in the art. Such algorithms and programs include, but are by no means limited to, TBLASTN, BLASTP, FASTA, TFASTA and CLUSTALW (Pearson and Lipman, Proc. Natl. Acad. Sci. USA 85(8):2444-2448, 1988; Altschul et al., J. Mol. Biol. 215(3):403-410, 1990; Thompson et al., Nucleic Acids Res. 22(2):4673-4680, 1994; Higgins et al., Methods Enzymol. 266:383-402, 1996; Altschul et al, J. Mol. Biol. 215(3):403-410, 1990; Altschul et al., Nature Genetics 3:266-272, 1993).

Homology or identity is often measured using sequence analysis software (e.g., Sequence Analysis Software Package of the Genetics Computer Group, University of Wisconsin Biotechnology Center, 1710 University Avenue, Madison, WI 53705). Such software matches similar sequences by assigning degrees of homology to various deletions, substitutions and other modifications. The terms "homology" and "identity" in the context of two or more nucleic acids or polypeptide sequences, refer to two or more sequences or subsequences that are the same or have a specified percentage of amino acid residues or nucleotides that are the same when compared and aligned for maximum correspondence over a comparison window or designated region as measured using any number of sequence comparison algorithms or by manual alignment and visual inspection.

For sequence comparison, typically one sequence acts as a reference sequence, to which test sequences are compared. When using a sequence comparison algorithm, test and reference sequences are entered into a computer, subsequence coordinates are designated, if necessary and sequence algorithm program parameters are designated. Default program parameters can be used, or alternative parameters can be designated. The sequence comparison algorithm then calculates the percent sequence identities for the test sequences relative to the reference sequence, based on the program parameters.

A "comparison window", as used herein, includes reference to a segment of any one of the number of contiguous positions selected from the group consisting of from 20 to 600, usually about 50 to about 200, more usually about 100 to about 150 in which a sequence may be compared to a reference sequence of the same number of contiguous positions after the two sequences are optimally aligned. Methods of alignment of sequence for comparison are well-known in the art. Optimal alignment of sequences for comparison can be conducted, *e.g*., by the local homology algorithm of Smith & Waterman, Adv. Appl. Math. 2:482, 1981, by the homology alignment algorithm of Needleman & Wunsch, J. Mol. Biol 48:443, 1970, by the search for similarity method of person & Lipman, Proc. Nat'l. Acad. Sci. USA 85:2444, 1988, by computerized implementations of these algorithms (GAP, BESTFIT, FASTA and TFASTA in the Wisconsin Genetics Software Package, Genetics Computer Group, 575 Science Dr., Madison, WI), or by manual alignment and visual inspection. Other algorithms for determining homology or identity include, for example, in addition to a BLAST program (Basic Local Alignment Search Tool at the National Center for Biological Information), ALIGN, AMAS (Analysis of Multiply Aligned Sequences), AMPS (Protein Multiple Sequence Alignment), ASSET (Aligned Segment Statistical Evaluation Tool), BANDS, BESTSCOR, BIOSCAN (Biological Sequence Comparative Analysis Node), BLIMPS (BLocks IMProved Searcher), FASTA, Intervals & Points, BMB, CLUSTAL V, CLUSTAL W, CONSENSUS, LCONSENSUS, WCONSENSUS, Smith-Waterman algorithm, DARWIN, Las Vegas algorithm, FNAT (Forced Nucleotide Alignment Tool), Framealign, Framesearch, DYNAMIC, FILTER, FSAP (Fristensky Sequence Analysis Package), GAP (Global Alignment Program), GENAL, GIBBS, GenQuest, ISSC (Sensitive Sequence Comparison), LALIGN (Local Sequence Alignment), LCP (Local Content Program), MACAW (Multiple Alignment Construction & Analysis Workbench), MAP (Multiple Alignment Program), MBLKP, MBLKN, PIMA (Pattem-Induced Multi-sequence Alignment), SAGA (Sequence Alignment by Genetic Algorithm) and WHAT-IF. Such alignment programs can also be used to screen genome databases to identify polynucleotide sequences having substantially identical sequences. A number of genome databases are available, for example, a substantial portion of the human genome is available as part of the Human Genome Sequencing Project (J. Roach, http://weber.u.Washington.edu/~roach/human_ genome_ progress 2.html) (Gibbs, 1995). At least twenty-one other genomes have already been sequenced, including, for example, *M. genitalium* (Fraser *et al.*, 1995), M. jannaschii (Bult *et al.*, 1996), *H. influenzae* (Fleischmann *et al.*, 1995), E. *coli* (Blattner *et al.*, 1997) and yeast (*S. cerevisiae*) (Mewes *et al.*, 1997) and *D. melanogaster* (Adams *et al.*, 2000). Significant progress has also been made in sequencing the genomes of model organism, such as mouse, *C. elegans* and *Arabadopsis sp.* Several databases containing genomic information annotated with some functional information are maintained by different organization and are accessible via the internet

One example of a useful algorithm is BLAST and BLAST 2.0 algorithms, which are described in Altschul et al., Nuc. Acids Res. 25:3389-3402, 1977 and Altschul et al., J. Mol. Biol. 215:403-410, 1990, respectively. Software for performing BLAST analyses is publicly available through the National Center for Biotechnology Information. This algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold (Altschul *et al., supra*). These initial neighborhood word hits act as seeds for initiating searches to find longer HSPs containing them. The word hits are extended in both directions along each sequence for as far as the cumulative alignment score can be increased. Cumulative scores are calculated using, for nucleotide sequences, the parameters M (reward score for a pair of matching residues; always >0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T and X determine the sensitivity and speed of the alignment. The BLASTN program (for nucleotide sequences) uses as defaults a wordlength (W) of 11, an expectation (E) of 10, M=5, N=-4 and a comparison of both strands. For amino acid sequences, the BLASTP program uses as defaults a wordlength of 3 and expectations (E) of 10 and the BLOSUM62 scoring matrix (see Henikoff & Henikoff, Proc. Natl. Acad. Sci. USA 89:10915, 1989) alignments (B) of 50, expectation (E) of 10, M=5, N= -4 and a comparison of both strands.

The BLAST algorithm also performs a statistical analysis of the similarity between two sequences (see, *e.g*., Karlin & Altschul, Proc. Natl. Acad. Sci. USA 90:5873, 1993). One measure of similarity provided by BLAST algorithm is the smallest sum probability (P(N)), which provides an indication of the probability by which a match between two nucleotide or amino acid sequences would occur by chance. For example, a nucleic acid is considered similar to a references sequence if the smallest sum probability in a comparison of the test nucleic acid to the reference nucleic acid is less than about 0.2, for instacneless than about 0.01 and for instance less than about 0.001.

Protein and nucleic acid sequence homologies are evaluated using the Basic Local Alignment Search Tool ("BLAST") In particular, five specific BLAST programs are used to perform the following task:
(1) BLASTP and BLAST3 compare an amino acid query sequence against a protein sequence database;
(2) BLASTN compares a nucleotide query sequence against a nucleotide sequence database;
(3) BLASTX compares the six-frame conceptual translation products of a query nucleotide sequence (both strands) against a protein sequence database;
(4) TBLASTN compares a query protein sequence against a nucleotide sequence database translated in all six reading frames (both strands); and
(5) TBLASTX compares the six-frame translations of a nucleotide query sequence against the six-frame translations of a nucleotide sequence database.

The BLAST programs identify homologous sequences by identifying similar segments, which are referred to herein as "high-scoring segment pairs," between a query amino or nucleic acid sequence and a test sequence which is obtained from a protein or nucleic acid sequence database. High-scoring segment pairs are identified (i.e., aligned) by means of a scoring matrix, many of which are known in the art. The scoring matrix used is the BLOSUM62 matrix (Gonnet et al., Science 256:1443-1445, 1992; Henikoff and Henikoff, Proteins 17:49-61, 1993). The PAM or PAM250 matrices may also be used (see, e.g., Schwartz and Dayhoff, eds., 1978, Matrices for Detecting Distance Relationships: Atlas of Protein Sequence and Structure, Washington: National Biomedical Research Foundation). BLAST programs are accessible through the U.S. National Library of Medicine.

The parameters used with the above algorithms may be adapted depending on the sequence length and degree of homology studied. The parameters may be the default parameters used by the algorithms in the absence of instructions from the user.

The phrase "substantially identical" in the context of two nucleic acids or polypeptides, refers to two or more sequences that have, e.g., at least about 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or more nucleotide or amino acid residue (sequence) identity, when compared and aligned for maximum correspondence, as measured using one of the known sequence comparison algorithms or by visual inspection. Alternatively. the substantial identity exists over a region of at least about 50, 75, 100, 150, 200, 250, 300, 350, 400, 450, 500, 550, 600, 650, 700, 750, 800, 850, 900, 950, 1000, 1050, 1100, 1150 or more residues, or the full length of a gene or a transcript. The sequences are substantially identical over the entire length of a coding region.

### Computer systems and computer program products

To determine and identify sequence identities, structural homologies, motifs and the like in silico, a nucleic acid or polypeptide sequence of the invention can be stored, recorded, and manipulated on any medium which can be read and accessed by a computer.

Accordingly, the present disclosure provides computers, computer systems, computer readable mediums, computer programs products and the like recorded or stored thereon the nucleic acid and polypeptide sequences of the invention. As used herein, the words "recorded" and "stored" refer to a process for storing information on a computer medium. A skilled artisan can readily adopt any known methods for recording information on a computer readable medium to generate manufactures comprising one or more of the nucleic acid and/or polypeptide sequences of the invention.

The polypeptides of the disclosure include the polypeptide sequences disclosed herein, e.g., the exemplary sequences disclosed, and sequences substantially identical thereto, and fragments of any of the preceding sequences. Substantially identical, or homologous, polypeptide sequences refer to a polypeptide sequence having at least 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or more, or complete (100%) sequence identity to an exemplary sequence disclosed herein.

Homology may be determined using any of the computer programs and parameters described herein, including FASTA version 3.0t78 with the default parameters or with any modified parameters. The homologous sequences may be obtained using any of the procedures described herein or may result from the correction of a sequencing error. The polypeptide fragments comprise at least about 10, 15, 20, 25, 30, 35, 40, 45, 50, 75, 100, 150, 200, 250, 300, 350, 400, 450, 500 or more consecutive amino acids of the polypeptides of the invention. It will be appreciated that the polypeptide codes as set forth in amino acid sequences of the invention, can be represented in the traditional single character format or three letter format (See the inside back cover of Stryer, Lubert. Biochemistry, 3rd Ed., W. H Freeman & Co., New York.) or in any other format which relates the identity of the polypeptides in a sequence.

A nucleic acid or polypeptide sequence as disclosed herein can be stored, recorded and manipulated on any medium which can be read and accessed by a computer. As used herein, the words "recorded" and "stored" refer to a process for storing information on a computer medium. A skilled artisan can readily adopt any of the presently known methods for recording information on a computer readable medium to generate manufactures comprising one or more of the nucleic acid sequences of the invention, one or more of the polypeptide sequences of the invention. Disclosed herein is a computer readable medium having recorded thereon at least 2, 5, 10, 15, or 20 or more nucleic acid sequences.

Disclosed herein is a computer readable medium having recorded thereon one or more of the nucleic acid sequences of the invention. Further disclosed is a computer readable medium having recorded thereon one or more of the polypeptide sequences of the invention. Also disclosed is a computer readable medium having recorded thereon at least 2, 5, 10, 15, or 20 or more of the sequences as set forth above.

As used herein, the terms "computer," "computer program" and "processor" are used in their broadest general contexts and incorporate all such devices, as described in detail, below. A "coding sequence of" or a "sequence encodes" a particular polypeptide or protein, is a nucleic acid sequence which is transcribed and translated into a polypeptide or protein when placed under the control of appropriate regulatory sequences.

Computer readable media include magnetically readable media, optically readable media, electronically readable media and magnetic/optical media. For example, the computer readable media may be a hard disk, a floppy disk, a magnetic tape, CD-ROM, Digital Versatile Disk (DVD), Random Access Memory (RAM), or Read Only Memory (ROM) as well as other types of other media known to those skilled in the art.

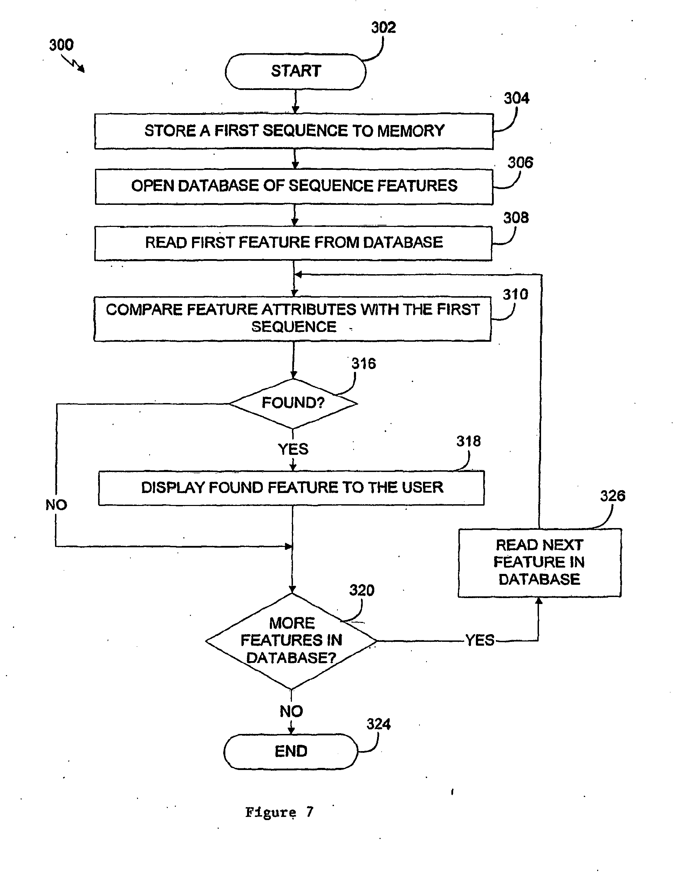

Further disclosed are systems (*e.g*., internet based systems), particularly computer systems which store and manipulate the sequence information described herein. One example of a computer system 100 is illustrated in block diagram form in Figure 7. As used herein, "a computer system" refers to the hardware components, software components and data storage components used to analyze a nucleotide sequence of a nucleic acid sequence of the invention, or a polypeptide sequence of the invention. The computer system 100 typically includes a processor for processing, accessing and manipulating the sequence data. The processor 105 can be any well-known type of central processing unit, such as, for example, the Pentium III from Intel Corporation, or similar processor from Sun, Motorola, Compaq, AMD or International Business Machines.

Typically the computer system 100 is a general purpose system that comprises the processor 105 and one or more internal data storage components 110 for storing data and one or more data retrieving devices for retrieving the data stored on the data storage components. A skilled artisan can readily appreciate that any one of the currently available computer systems are suitable.

The computer system 100 includes a processor 105 connected to a bus which is connected to a main memory 115 (implemented as RAM) and one or more internal data storage devices 110, such as a hard drive and/or other computer readable media having data recorded thereon. The computer system 100 further includes one or more data retrieving device 118 for reading the data stored on the internal data storage devices 110.

The data retrieving device 118 may represent, for example, a floppy disk drive, a compact disk drive, a magnetic tape drive, or a modem capable of connection to a remote data storage system (e.g., via the internet) etc. The internal data storage device 110 is a removable computer readable medium such as a floppy disk, a compact disk, a magnetic tape, etc. containing control logic and/or data recorded thereon. The computer system 100 may advantageously include or be programmed by appropriate software for reading the control logic and/or the data from the data storage component once inserted in the data retrieving device.

The computer system 100 includes a display 120 which is used to display output to a computer user. It should also be noted that the computer system 100 can be linked to other computer systems 125a-c in a network or wide area network to provide centralized access to the computer system 100.

Software for accessing and processing the nucleotide sequences of a nucleic acid sequence of the invention, or a polypeptide sequence of the invention, (such as search tools, compare tools and modeling tools etc.) may reside in main memory 115 during execution.

The computer system 100 may further comprise a sequence comparison algorithm for comparing a nucleic acid sequence of the invention, or a polypeptide sequence of the invention, stored on a computer readable medium to a reference nucleotide or polypeptide sequence(s) stored on a computer readable medium. A "sequence comparison algorithm" refers to one or more programs which are implemented (locally or remotely) on the computer system 100 to compare a nucleotide sequence with other nucleotide sequences and/or compounds stored within a data storage means. For example, the sequence comparison algorithm may compare the nucleotide sequences of a nucleic acid sequence of the invention, or a polypeptide sequence of the invention, stored on a computer readable medium to reference sequences stored on a computer readable medium to identify homologies or structural motifs.

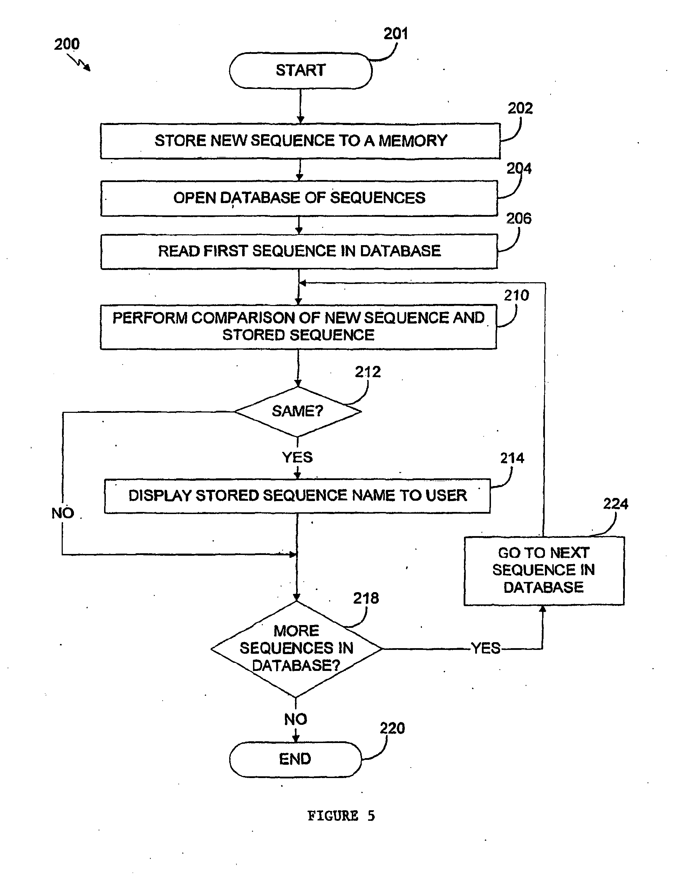

Figure 5 is a flow diagram illustrating a process 200 for comparing a new nucleotide or protein sequence with a database of sequences in order to determine the homology levels between the new sequence and the sequences in the database. The database of sequences can be a private database stored within the computer system 100, or a public database such as GENBANK that is available through the Internet.

The process 200 begins at a start state 201 and then moves to a state 202 wherein the new sequence to be compared is stored to a memory in a computer system 100. As discussed above, the memory could be any type of memory, including RAM or an internal storage device.

The process 200 then moves to a state 204 wherein a database of sequences is opened for analysis and comparison. The process 200 then moves to a state 206 wherein the first sequence stored in the database is read into a memory on the computer. A comparison is then performed at a state 210 to determine if the first sequence is the same as the second sequence. It is important to note that this step is not limited to performing an exact comparison between the new sequence and the first sequence in the database. Well-known methods are known to those of skill in the art for comparing two nucleotide or protein sequences, even if they are not identical. For example, gaps can be introduced into one sequence in order to raise the homology level between the two tested sequences. The parameters that control whether gaps or other features are introduced into a sequence during comparison are normally entered by the user of the computer system.

Once a comparison of the two sequences has been performed at the state 210, a determination is made at a decision state 210 whether the two sequences are the same. Of course, the term "same" is not limited to sequences that are absolutely identical. Sequences that are within the homology parameters entered by the user will be marked as "same" in the process 200.

If a determination is made that the two sequences are the same, the process 200 moves to a state 214 wherein the name of the sequence from the database is displayed to the user. This state notifies the user that the sequence with the displayed name fulfills the homology constraints that were entered. Once the name of the stored sequence is displayed to the user, the process 200 moves to a decision state 218 wherein a determination is made whether more sequences exist in the database. If no more sequences exist in the database, then the process 200 terminates at an end state 220. However, if more sequences do exist in the database, then the process 200 moves to a state 224 wherein a pointer is moved to the next sequence in the database so that it can be compared to the new sequence. In this manner, the new sequence is aligned and compared with every sequence in the database.

It should be noted that if a determination had been made at the decision state 212 that the sequences were not homologous, then the process 200 would move immediately to the decision state 218 in order to determine if any other sequences were available in the database for comparison.

Accordingly, a computer system comprising a processor, a data storage device having stored thereon a nucleic acid sequencedisclosed herein, or a polypeptide sequence disclosed herein, a data storage device having retrievably stored thereon reference nucleotide sequences or polypeptide sequences to be compared to a nucleic acid sequence disclosed herein, or a polypeptide sequence disclosed hereinand a sequence comparer for conducting the comparison is herein disclosed. The sequence comparer may indicate a homology level between the sequences compared or identify structural motifs in the above described nucleic acid code a nucleic acid sequence disclosed herein, or a polypeptide sequence disclosed herein, or it may identify structural motifs in sequences which are compared to these nucleic acid codes and polypeptide codes. The data storage device may have stored thereon the sequences of at least 2, 5, 10, 15, 20, 25, 30 or 40 or more of the nucleic acid sequences of the invention, or the polypeptide sequences of the invention.

Further disclosed is a method for determining the level of homology between a nucleic acid sequence of the invention, or a polypeptide sequence of the invention and a reference nucleotide sequence. The method including reading the nucleic acid code or the polypeptide code and the reference nucleotide or polypeptide sequence through the use of a computer program which determines homology levels and determining homology between the nucleic acid code or polypeptide code and the reference nucleotide or polypeptide sequence with the computer program. The computer program may be any of a number of computer programs for determining homology levels, including those specifically enumerated herein, (*e.g*., BLAST2N with the default parameters or with any modified parameters). The method may be implemented using the computer systems described above. The method may also be performed by reading at least 2, 5, 10, 15, 20, 25, 30 or 40 or more of the above described nucleic acid sequences of the invention, or the polypeptide sequences disclosed herein through use of the computer program and determining homology between the nucleic acid codes or polypeptide codes and reference nucleotide sequences or polypeptide sequences.

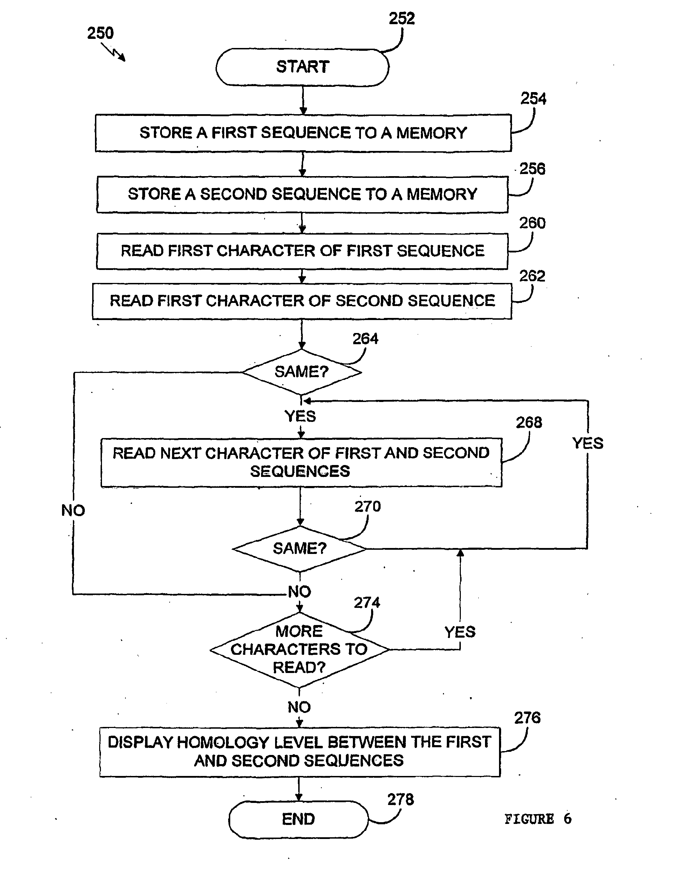

Figure 6 is a flow diagram illustrating a process 250 in a computer for determining whether two sequences are homologous. The process 250 begins at a start state 252 and then moves to a state 254 wherein a first sequence to be compared is stored to a memory. The second sequence to be compared is then stored to a memory at a state 256. The process 250 then moves to a state 260 wherein the first character in the first sequence is read and then to a state 262 wherein the first character of the second sequence is read. It should be understood that if the sequence is a nucleotide sequence, then the character would normally be either A, T, C, G or U. If the sequence is a protein sequence, then it is i in the single letter amino acid code so that the first and sequence sequences can be easily compared.

A determination is then made at a decision state 264 whether the two characters are the same. If they are the same, then the process 250 moves to a state 268 wherein the next characters in the first and second sequences are read. A determination is then made whether the next characters are the same. If they are, then the process 250 continues this loop until two characters are not the same. If a determination is made that the next two characters are not the same, the process 250 moves to a decision state 274 to determine whether there are any more characters either sequence to read.

If there are not any more characters to read, then the process 250 moves to a state 276 wherein the level of homology between the first and second sequences is displayed to the user. The level of homology is determined by calculating the proportion of characters between the sequences that were the same out of the total number of sequences in the first sequence. Thus, if every character in a first 100 nucleotide sequence aligned with a every character in a second sequence, the homology level would be 100%.

Alternatively, the computer program may be a computer program which compares the nucleotide sequences of a nucleic acid sequence as set forth in the invention, to one or more reference nucleotide sequences in order to determine whether the nucleic acid code of the invention, differs from a reference nucleic acid sequence at one or more positions. Optionally such a program records the length and identity of inserted, deleted or substituted nucleotides with respect to the sequence of either the reference polynucleotide or a nucleic acid sequence of the invention. The computer program may be a program which determines whether a nucleic acid sequence of the invention, contains a single nucleotide polymorphism (SNP) with respect to a reference nucleotide sequence.

Accordingly, disclosed is a method for determining whether a nucleic acid sequence disclosed herein, differs at one or more nucleotides from a reference nucleotide sequence comprising the steps of reading the nucleic acid code and the reference nucleotide sequence through use of a computer program which identifies differences between nucleic acid sequences and identifying differences between the nucleic acid code and the reference nucleotide sequence with the computer program. The computer program is a program which identifies single nucleotide polymorphisms. The method may be implemented by the computer systems described above and the method illustrated in Figure 6. The method may also be performed by reading at least 2, 5, 10, 15, 20, 25, 30, or 40 or more of the nucleic acid sequences of the invention and the reference nucleotide sequences through the use of the computer program and identifying differences between the nucleic acid codes and the reference nucleotide sequences with the computer program.

The computer based system may further comprise an identifier for identifying features within a nucleic acid sequence disclosed herein or a polypeptide sequence disclosed herein.

An "identifier" refers to one or more programs which identifies certain features within a nucleic acid sequence of the invention, or a polypeptide sequence of the invention. The identifier may comprise a program which identifies an open reading frame in a nucleic acid sequencedisclosed herein.

Figure 7 is a flow diagram illustrating an identifier process 300 for detecting the presence of a feature in a sequence. The process 300 begins at a start state 302 and then moves to a state 304 wherein a first sequence that is to be checked for features is stored to a memory 115 in the computer system 100. The process 300 then moves to a state 306 wherein a database of sequence features is opened. Such a database would include a list of each feature's attributes along with the name of the feature. For example, a feature name could be "Initiation Codon" and the attribute would be "ATG". Another example would be the feature name "TAATAA Box" and the feature attribute would be "TAATAA". An example of such a database is produced by the University of Wisconsin Genetics Computer Group. Alternatively, the features may be structural polypeptide motifs such as alpha helices, beta sheets, or functional polypeptide motifs such as enzymatic catalytic domains (CDs), or, active sites, helix-turn-helix motifs or other motifs known to those skilled in the art.

Once the database of features is opened at the state 306, the process 300 moves to a state 308 wherein the first feature is read from the database. A comparison of the attribute of the first feature with the first sequence is then made at a state 310. A determination is then made at a decision state 316 whether the attribute of the feature was found in the first sequence. If the attribute was found, then the process 300 moves to a state 318 wherein the name of the found feature is displayed to the user.

The process 300 then moves to a decision state 320 wherein a determination is made whether move features exist in the database. If no more features do exist, then the process 300 terminates at an end state 324. However, if more features do exist in the database, then the process 300 reads the next sequence feature at a state 326 and loops back to the state 310 wherein the attribute of the next feature is compared against the first sequence. It should be noted, that if the feature attribute is not found in the first sequence at the decision state 316, the process 300 moves directly to the decision state 320 in order to determine if any more features exist in the database.

Accordingly, further disclosed is a method of identifying a feature within a nucleic acid sequence of the invention, or a polypeptide sequence of the invention, comprising reading the nucleic acid code(s) or polypeptide code(s) through the use of a computer program which identifies features therein and identifying features within the nucleic acid code(s) with the computer program. A computer program comprises a computer program which identifies open reading frames. The method may be performed by reading a single sequence or at least 2, 5, 10, 15, 20, 25, 30, or 40 of the nucleic acid sequences of the invention, or the polypeptide sequences of the invention, through the use of the computer program and identifying features within the nucleic acid codes or polypeptide codes with the computer program.

A nucleic acid sequence disclosed herein, or a polypeptide sequence disclosed herein, may be stored and manipulated in a variety of data processor programs in a variety of formats. For example, a nucleic acid sequence of the invention, or a polypeptide sequence of the invention, may be stored as text in a word processing file, such as Microsoft WORD™ or WORDPERFECT™ or as an ASCII file in a variety of database programs familiar to those of skill in the art, such as DB2™, SYBASE™, or ORACLE™. In addition, many computer programs and databases may be used as sequence comparison algorithms, identifiers, or sources of reference nucleotide sequences or polypeptide sequences to be compared to a nucleic acid sequence of the invention, or a polypeptide sequence of the invention. The following list is intended not to limit the invention but to provide guidance to programs and databases which are useful with the nucleic acid sequences disclosed herein, or the polypeptide sequences disclosed herein.

The programs and databases which may be used include, but are not limited to: MacPattem (EMBL), DiscoveryBase (Molecular Applications Group), GeneMine (Molecular Applications Group), Look (Molecular Applications Group), MacLook (Molecular Applications Group), BLAST and BLAST2 (NCBI), BLASTN and BLASTX (Altschul et al, J. Mol. Biol. 215: 403, 1990), FASTA (Pearson and Lipman, Proc. Natl. Acad. Sci. USA, 85: 2444, 1988), FASTDB (Brutlag et al. Comp. App. Biosci. 6:237-245, 1990), Catalyst (Molecular Simulations Inc.), Catalyst/SHAPE (Molecular Simulations Inc.), Cerius².DBAccess (Molecular Simulations Inc.), HypoGen (Molecular Simulations Inc.), Insight II, (Molecular Simulations Inc.), Discover (Molecular Simulations Inc.), CHARMm (Molecular Simulations Inc.), Felix (Molecular Simulations Inc.), DelPhi, (Molecular Simulations Inc.), QuanteMM, (Molecular Simulations Inc.), Homology (Molecular Simulations Inc.), Modeler (Molecular Simulations Inc.), ISIS (Molecular Simulations Inc.), Quanta/Protein Design (Molecular Simulations Inc.), WebLab (Molecular Simulations Inc.), WebLab Diversity Explorer (Molecular Simulations Inc.), Gene Explorer (Molecular Simulations Inc.), SeqFold (Molecular Simulations Inc.), the MDL Available Chemicals Directory database, the MDL Drug Data Report data base, the Comprehensive Medicinal Chemistry database, Derwents's World Drug Index database, the BioByteMasterFile database, the Genbank database and the Genseqn database. Many other programs and data bases would be apparent to one of skill in the art given the present disclosure.

Motifs which may be detected using the above programs include sequences encoding leucine zippers, helix-turn-helix motifs, glycosylation sites, ubiquitination sites, alpha helices and beta sheets, signal sequences encoding signal peptides which direct the secretion of the encoded proteins, sequences implicated in transcription regulation such as homeoboxes, acidic stretches, enzymatic active sites (catalytic domains (CDs)), substrate binding sites and enzymatic cleavage sites.

### Hybridization of nucleic acids

The present disclosure provides isolated, synthetic or recombinant nucleic acids that hybridize under stringent conditions to an exemplary sequence disclosed herein (e.g., SEQ ID NO:1, SEQ ID NO:3, SEQ ID NO:5, SEQ ID NO:7, SEQ ID NO:9, SEQ ID NO:11, SEQ ID NO:13, SEQ ID NO:15, SEQ ID NO:17 or SEQ ID NO:19). The stringent conditions can be highly stringent conditions, medium stringent conditions and/or low stringent conditions, including the high and reduced stringency conditions described herein. It is the stringency of the wash conditions that set forth the conditions which determine whether a nucleic acid is within the scope of the invention, as discussed below.

Nucleic acids as defined by their ability to hybridize under stringent conditions can be between about five residues and the full length of nucleic acid of the invention; e.g., they can be at least 5, 10, 15, 20, 25, 30, 35, 40, 50, 55, 60, 65, 70, 75, 80, 90, 100, 150, 200, 250, 300, 350, 400, 450, 500, 550, 600, 650, 700, 750, 800, 850, 900, 950, 1000, or more, residues in length. Nucleic acids shorter than full length are also included. These nucleic acids can be useful as, e.g., hybridization probes, labeling probes, PCR oligonucleotide probes, iRNA (single or double stranded), antisense or sequences encoding antibody binding peptides (epitopes), motifs, active sites (catalytic domains (CDs)) and the like.

Nucleic acids are defined by their ability to hybridize under high stringency comprises conditions of about 50% formamide at about 37°C to 42°C. , Alternatively, nucleic acids are defined by their ability to hybridize under reduced stringency comprising conditions in about 35% to 25% formamide at about 30°C to 35°C.

Alternatively, nucleic acids are defined by their ability to hybridize under high stringency comprising conditions at 42°C in 50% formamide, 5X SSPE, 0.3% SDS, and a repetitive sequence blocking nucleic acid, such as cot-1 or salmon sperm DNA (e.g., 200 n/ml sheared and denatured salmon sperm DNA). Nucleic acids of the present disclosure are defined by their ability to hybridize under reduced stringency conditions comprising 35% formamide at a reduced temperature of 35°C.

. See Sambrook, Tijssen and Ausubel for a description of SSC buffer and equivalent conditions.

These methods may be used to isolate nucleic acids of the invention. For example, the preceding methods may be used to isolate nucleic acids having a sequence with at least about 97%, at least 95%, at least 90%, at least 85%, at least 80%, at least 75%, at least 70%, at least 65%, at least 60%, at least 55%, or at least 50% homology to a nucleic acid sequence selected from the group consisting of one of the sequences of the invention, or fragments comprising at least about 10, 15, 20, 25, 30, 35, 40, 50, 75, 100, 150, 200, 300, 400, or 500 consecutive bases thereof and the sequences complementary thereto. Homology may be measured using the alignment algorithm. For example, the homologous polynucleotides may have a coding sequence which is a naturally occurring allelic variant of one of the coding sequences described herein. Such allelic variants may have a substitution, deletion or addition of one or more nucleotides when compared to the nucleic acids of the invention.

Additionally, the above procedures may be used to isolate nucleic acids which encode polypeptides having at least about 99%, 95%, at least 90%, at least 85%, at least 80%, at least 75%, at least 70%, at least 65%, at least 60%, at least 55%, or at least 50% homology to a polypeptide of the invention, or fragments comprising at least 5, 10, 15, 20, 25, 30, 35, 40, 50, 75, 100, or 150 consecutive amino acids thereof as determined using a sequence alignment algorithm (e.g., such as the FASTA version 3.0t78 algorithm with the default parameters).

### Oligonucleotides probes and methods for using them

The disclosure also provides nucleic acid probes that can be used, e.g., for identifying nucleic acids encoding a polypeptide with an enzyme activity or fragments thereof or for identifying genes or other nucleic acids encoding polypeptides having a chlorophyllase enzyme activity or enzymes involved in the catabolism of chlorophyll. The probe comprises at least 10 consecutive bases of a nucleic acid of the invention. Alternatively, a probe can be at least about 5, 6, 7, 8, 9, 10, 11, 12, 13, 14, 15, 16, 17, 18, 19, 20, 21, 22, 23, 24, 25, 30, 35, 40, 45, 50, 60, 70, 80, 90, 100, 110, 120, 130, 150 or about 10 to 50, about 20 to 60 about 30 to 70, consecutive bases of a sequence as set forth in a nucleic acid of the invention. The probes identify a nucleic acid by binding and/or hybridization.

Many methods for using the labeled probes to detect the presence of complementary nucleic acids in a sample are familiar to those skilled in the art. These include Southern Blots, Northern Blots, colony hybridization procedures and dot blots. Protocols for each of these procedures are provided in Ausubel et al. Current Protocols in Molecular Biology, John Wiley 503 Sons, Inc. (1997) and Sambrook et al., Molecular Cloning: A Laboratory Manual 2nd Ed., Cold Spring Harbor Laboratory Press (1989.

### Inhibiting Expression of Enzymes

The disclosure provides nucleic acids complementary to (e.g., antisense sequences to) the nucleic acids disclosed herein, e.g., nucleic acids encoding polypeptides having an enzyme activity involved in chlorophyll catabolism or having an esterase (e.g., chlorophyllase) activity. Antisense sequences are capable of inhibiting the transport, splicing or transcription of enzyme-encoding genes. The inhibition can be effected through the targeting of genomic DNA or messenger RNA. The transcription or function of targeted nucleic acid can be inhibited, for example, by hybridization and/or cleavage.

### Antisense Oligonucleotides

Disclosed herein are antisense oligonucleotides capable of binding enzyme message or a gene which can inhibit a target gene or message to, e.g., inhibit a polypeptide involved in chlorophyll catabolism or having an esterase (e.g., chlorophyllase) activity by targeting mRNA. Strategies for designing antisense oligonucleotides are well described in the scientific and patent literature, and the skilled artisan can design such oligonucleotides using the novel reagents of the invention. For example, gene walking/ RNA mapping protocols to screen for effective antisense oligonucleotides are well known in the art, see, e.g., Ho (2000) Methods Enzymol. 314:168-183, describing an RNA mapping assay, which is based on standard molecular techniques to provide an easy and reliable method for potent antisense sequence selection. See also Smith (2000) Eur. J. Pharm. Sci. 11:191-198.

. Antisense oligonucleotides having phosphorothioate linkages can also be used, as described in WO 97/03211; WO 96/39154; Mata (1997) Toxicol Appl Pharmacol 144:189-197; Antisense Therapeutics, ed. Agrawal (Humana Press, Totowa, N.J., 1996). Antisense oligonucleotides having synthetic DNA backbone analogues provided by the invention can also include phosphoro-dithioate, methylphosphonate, phosphoramidate, alkyl phosphotriester, sulfamate, 3'-thioacetal, methylene(methylimino), 3'-N-carbamate, and morpholino carbamate nucleic acids, as described above.

Combinatorial chemistry methodology can be used to create vast numbers of oligonucleotides that can be rapidly screened for specific oligonucleotides that have appropriate binding affinities and specificities toward any target, such as the sense and antisense sequences of the invention (see, e.g., Gold (1995) J. of Biol. Chem. 270:13581-13584).

### Modification of Nucleic Acids

The present disclosure provides methods of generating variants of the nucleic acids for use according to the invention, e.g., those encoding a polypeptide involved in chlorophyll catabolism or having an esterase (e.g., chlorophyllase) activity, e.g., enzymes described herein. These methods can be repeated or used in various combinations to generate polypeptides involved in chlorophyll catabolism or having an esterase (e.g., chlorophyllase) activity having an altered or different activity or an altered or different stability from that of an enzyme encoded by the template nucleic acid. These methods also can be repeated or used in various combinations, e.g., to generate variations in gene/ message expression, message translation or message stability. The genetic composition of a cell is altered by, e.g., modification of a homologous gene ex vivo, followed by its reinsertion into the cell.

A nucleic acid can be altered by any means. For example, random or stochastic methods, or, non-stochastic, or "directed evolution," methods, see, e.g., U.S. Patent No. 6,361,974. Methods for random mutation of genes are well known in the art, see, e.g., U.S. Patent No. 5,830,696. For example, mutagens can be used to randomly mutate a gene. Mutagens include, e.g., ultraviolet light or gamma irradiation, or a chemical mutagen, e.g., mitomycin, nitrous acid, photoactivated psoralens, alone or in combination, to induce DNA breaks amenable to repair by recombination. Other chemical mutagens include, for example, sodium bisulfite, nitrous acid, hydroxylamine, hydrazine or formic acid. Other mutagens are analogues of nucleotide precursors, e.g., nitrosoguanidine, 5-bromouracil, 2-aminopurine, or acridine. These agents can be added to a PCR reaction in place of the nucleotide precursor thereby mutating the sequence. Intercalating agents such as proflavine, acriflavine, quinacrine and the like can also be used.

Any technique in molecular biology can be used, e.g., random PCR mutagenesis, see, e.g., Rice (1992) Proc. Natl. Acad. Sci. USA 89:8467-5471; or, combinatorial multiple cassette mutagenesis, see, e.g., Crameri (1995) Biotechniques 18:194-196. Alternatively, nucleic acids, e.g., genes, can be reassembled after random, or "stochastic," fragmentation, see, e.g., U.S. Patent Nos. 6,291,242; 6,287,862; 6,287,861; 5,955,358; 5,830,721; 5,824,514; 5,811,238; 5,605,793.

The following publications describe a variety of recursive recombination procedures and/or methods which can be incorporated into the methods of the invention: Stemmer (1999) "Molecular breeding of viruses for targeting and other clinical properties" Tumor Targeting 4:1-4; Ness (1999) Nature Biotechnology 17:893-896; Chang (1999) "Evolution of a cytokine using DNA family shuffling" Nature Biotechnology 17:793-797; Minshull (1999) "Protein evolution by molecular breeding" Current Opinion in Chemical Biology 3:284-290; Christians (1999) "Directed evolution of thymidine kinase for AZT phosphorylation using DNA family shuffling" Nature Biotechnology 17:259-264; Crameri (1998) "DNA shuffling of a family of genes from diverse species accelerates directed evolution" Nature 391:288-291; Crameri (1997) "Molecular evolution of an arsenate detoxification pathway by DNA shuffling," Nature Biotechnology 15:436-438; Zhang (1997) "Directed evolution of an effective fucosidase from a galactosidase by DNA shuffling and screening" Proc. Natl. Acad. Sci. USA 94:4504-4509; Patten et al. (1997) "Applications of DNA Shuffling to Pharmaceuticals and Vaccines" Current Opinion in Biotechnology 8:724-733; Crameri et al. (1996) "Construction and evolution of antibody-phage libraries by DNA shuffling" Nature Medicine 2:100-103; Gates et al. (1996) "Affinity selective isolation of ligands from peptide libraries through display on a lac repressor 'headpiece dimer" Journal of Molecular Biology 255:373-386; Stemmer (1996) "Sexual PCR and Assembly PCR" In: The Encyclopedia of Molecular Biology. VCH Publishers, New York. pp.447-457; Crameri and Stemmer (1995) "Combinatorial multiple cassette mutagenesis creates all the permutations of mutant and wildtype cassettes" BioTechniques 18:194-195; Stemmer et al. (1995) "Single-step assembly of a gene and entire plasmid form large numbers of oligodeoxyribonucleotides" Gene, 164:49-53; Stemmer (1995) "The Evolution of Molecular Computation" Science 270: 1510; Stemmer (1995) "Searching Sequence Space" Bio/Technology 13:549-553; Stemmer (1994) "Rapid evolution of a protein in vitro by DNA shuffling" Nature 370:389-391; and Stemmer (1994) "DNA shuffling by random fragmentation and reassembly: In vitro recombination for molecular evolution." Proc. Natl. Acad. Sci. USA 91:10747-10751.

Mutational methods of generating diversity include, for example, site-directed mutagenesis (Ling et al. (1997) "Approaches to DNA mutagenesis: an overview" Anal Biochem. 254(2): 157-178; Dale et al. (1996) "Oligonucleotide-directed random mutagenesis using the phosphorothioate method" Methods Mol. Biol. 57:369-374; Smith (1985) "In vitro mutagenesis" Ann. Rev. Genet. 19:423-462; Botstein & Shortle (1985) "Strategies and applications of in vitro mutagenesis" Science 229:1193-1201; Carter (1986) "Site-directed mutagenesis" Biochem. J. 237:1-7; and Kunkel (1987) "The efficiency of oligonucleotide directed mutagenesis" in Nucleic Acids & Molecular Biology (Eckstein, F. and Lilley, D. M. J. eds., Springer Verlag, Berlin)); mutagenesis using uracil containing templates (Kunkel (1985) "Rapid and efficient site-specific mutagenesis without phenotypic selection" Proc. Natl. Acad. Sci. USA 82:488-492; Kunkel et al. (1987) "Rapid and efficient site-specific mutagenesis without phenotypic selection" Methods in Enzymol. 154, 367-382; and Bass et al. (1988) "Mutant Trp repressors with new DNA-binding specificities" Science 242:240-245); oligonucleotide-directed mutagenesis (Methods in Enzymol. 100: 468-500 (1983); Methods in Enzymol. 154: 329-350 (1987); Zoller (1982) "Oligonucleotide-directed mutagenesis using M13-derived vectors: an efficient and general procedure for the production of point mutations in any DNA fragment" Nucleic Acids Res. 10:6487-6500; Zoller & Smith (1983) "Oligonucleotide-directed mutagenesis of DNA fragments cloned into M13 vectors" Methods in Enzymol. 100:468-500; and Zoller (1987) Oligonucleotide-directed mutagenesis: a simple method using two oligonucleotide primers and a single-stranded DNA template" Methods in Enzymol. 154:329-350); phosphorothioate-modified DNA mutagenesis (Taylor (1985) "The use of phosphorothioate-modified DNA in restriction enzyme reactions to prepare nicked DNA" Nucl. Acids Res. 13: 8749-8764; Taylor (1985) "The rapid generation of oligonucleotide-directed mutations at high frequency using phosphorothioate-modified DNA" Nucl. Acids Res. 13: 8765-8787 (1985); Nakamaye (1986) "Inhibition of restriction endonuclease Nci I cleavage by phosphorothioate groups and its application to oligonucleotide-directed mutagenesis" Nucl. Acids Res. 14: 9679-9698; Sayers (1988) "Y-T Exonucleases in phosphorothioate-based oligonucleotide-directed mutagenesis" Nucl. Acids Res. 16:791-802; and Sayers et al. (1988) "Strand specific cleavage of phosphorothioate-containing DNA by reaction with restriction endonucleases in the presence of ethidium bromide" Nucl. Acids Res. 16: 803-814); mutagenesis using gapped duplex DNA (Kramer et al. (1984) "The gapped duplex DNA approach to oligonucleotide-directed mutation construction" Nucl. Acids Res. 12: 9441-9456; Kramer & Fritz (1987) Methods in Enzymol. "Oligonucleotide-directed construction of mutations via gapped duplex DNA" 154:350-367; Kramer (1988) "Improved enzymatic in vitro reactions in the gapped duplex DNA approach to oligonucleotide-directed construction of mutations" Nucl. Acids Res. 16: 7207; and Fritz (1988) "Oligonucleotide-directed construction of mutations: a gapped duplex DNA procedure without enzymatic reactions in vitro" Nucl. Acids Res. 16: 6987-6999).

Additional protocols that can be used to practice the invention include point mismatch repair (Kramer (1984) "Point Mismatch Repair" Cell 38:879-887), mutagenesis using repair-deficient host strains (Carter et al. (1985) "Improved oligonucleotide site-directed mutagenesis using M13 vectors" Nucl. Acids Res. 13: 4431-4443; and Carter (1987) "Improved oligonucleotide-directed mutagenesis using M13 vectors" Methods in Enzymol. 154: 382-403), deletion mutagenesis (Eghtedarzadeh (1986) "Use of oligonucleotides to generate large deletions" Nucl. Acids Res. 14: 5115), restriction-selection and restriction-selection and restriction-purification (Wells et al. (1986) "Importance of hydrogen-bond formation in stabilizing the transition state of subtilisin" Phil. Trans. R. Soc. Lond. A 317: 415-423), mutagenesis by total gene synthesis (Nambiar et al. (1984) "Total synthesis and cloning of a gene coding for the ribonuclease S protein" Science 223: 1299-1301; Sakamar (1988) "Total synthesis and expression of a gene for the a-subunit of bovine rod outer segment guanine nucleotide-binding protein (transducin)" Nucl. Acids Res. 14: 6361-6372; Wells et al. (1985) "Cassette mutagenesis: an efficient method for generation of multiple mutations at defined sites" Gene 34:315-323; and Grundstrom et al. (1985) "Oligonucleotide-directed mutagenesis by microscale 'shot-gun gene synthesis" Nucl. Acids Res. 13: 3305-3316), double-strand break repair (Mandecki (1986); Arnold (1993) "Protein engineering for unusual environments" Current Opinion in Biotechnology 4:450-455. "Oligonucleotide-directed double-strand break repair in plasmids of Escherichia coli: a method for site-specific mutagenesis" Proc. Natl. Acad. Sci. USA, 83:7177-7181). Additional details on many of the above methods can be found in Methods in Enzymology Volume 154, which also describes useful controls for trouble-shooting problems with various mutagenesis methods.

Protocols that can be used to practice the disclosure are described, e.g., in U.S. Patent Nos. 5,605,793 to Stemmer (Feb. 25, 1997), "Methods for In Vitro Recombination;" U.S. Pat. No. 5,811,238 to Stemmer et al. (Sep. 22, 1998) "Methods for Generating Polynucleotides having Desired Characteristics by Iterative Selection and Recombination;" U.S. Pat. No. 5,830,721 to Stemmer et al. (Nov. 3, 1998), "DNA Mutagenesis by Random Fragmentation and Reassembly;" U.S. Pat. No. 5,834,252 to Stemmer, et al. (Nov. 10, 1998) "End-Complementary Polymerase Reaction;" U.S. Pat. No. 5,837,458 to Minshull, et al. (Nov. 17, 1998), "Methods and Compositions for Cellular and Metabolic Engineering;" WO 95/22625, Stemmer and Crameri, "Mutagenesis by Random Fragmentation and Reassembly;" WO 96/33207 by Stemmer and Lipschutz "End Complementary Polymerase Chain Reaction;" WO 97/20078 by Stemmer and Crameri "Methods for Generating Polynucleotides having Desired Characteristics by Iterative Selection and Recombination;" WO 97/35966 by Minshull and Stemmer, "Methods and Compositions for Cellular and Metabolic Engineering;" WO 99/41402 by Punnonen et al. "Targeting of Genetic Vaccine Vectors;" WO 99/41383 by Punnonen et al. "Antigen Library Immunization;" WO 99/41369 by Punnonen et al. "Genetic Vaccine Vector Engineering;" WO 99/41368 by Punnonen et al. "Optimization of Immunomodulatory Properties of Genetic Vaccines;" EP 752008 by Stemmer and Crameri, "DNA Mutagenesis by Random Fragmentation and Reassembly;" EP 0932670 by Stemmer "Evolving Cellular DNA Uptake by Recursive Sequence Recombination;" WO 99/23107 by Stemmer et al., "Modification of Virus Tropism and Host Range by Viral Genome Shuffling;" WO 99/21979 by Apt et al., "Human Papillomavirus Vectors;" WO 98/31837 by del Cardayre et al. "Evolution of Whole Cells and Organisms by Recursive Sequence Recombination;" WO 98/27230 by Patten and Stemmer, "Methods and Compositions for Polypeptide Engineering;" WO 98/27230 by Stemmer et al., "Methods for Optimization of Gene Therapy by Recursive Sequence Shuffling and Selection," WO 00/00632, "Methods for Generating Highly Diverse Libraries," WO 00/09679, "Methods for Obtaining in Vitro Recombined Polynucleotide Sequence Banks and Resulting Sequences," WO 98/42832 by Arnold et al., "Recombination of Polynucleotide Sequences Using Random or Defined Primers," WO 99/29902 by Arnold et al., "Method for Creating Polynucleotide and Polypeptide Sequences," WO 98/41653 by Vind, "An in Vitro Method for Construction of a DNA Library," WO 98/41622 by Borchert et al., "Method for Constructing a Library Using DNA Shuffling," and WO 98/42727 by Pati and Zarling, "Sequence Alterations using Homologous Recombination."

Protocols that can be used to practice the disclosure (providing details regarding various diversity generating methods) are described, e.g., in U.S. Patent application serial no. (USSN) 09/407,800, "SHUFFLING OF CODON ALTERED GENES" by Patten et al. filed Sep. 28, 1999; "EVOLUTION OF WHOLE CELLS AND ORGANISMS BY RECURSIVE SEQUENCE RECOMBINATION" by del Cardayre et al., United States Patent No. 6,379,964; "OLIGONUCLEOTIDE MEDIATED NUCLEIC ACID RECOMBINATION" by Crameri et al., United States Patent Nos. 6,319,714; 6,368,861; 6,376,246; 6,423,542; 6,426,224 and PCT/US00/01203; "USE OF CODON-VARIED OLIGONUCLEOTIDE SYNTHESIS FOR SYNTHETIC SHUFFLING" by Welch et al., United States Patent No. 6,436,675; "METHODS FOR MAKING CHARACTER STRINGS, POLYNUCLEOTIDES & POLYPEPTIDES HAVING DESIRED CHARACTERISTICS" by Selifonov et al., filed Jan. 18, 2000, (PCT/US00/01202) and, e.g. "METHODS FOR MAKING CHARACTER STRINGS, POLYNUCLEOTIDES & POLYPEPTIDES HAVING DESIRED CHARACTERISTICS" by Selifonov et al., filed Jul. 18, 2000 (U.S. Ser. No. 09/618,579); "METHODS OF POPULATING DATA STRUCTURES FOR USE IN EVOLUTIONARY SIMULATIONS" by Selifonov and Stemmer, filed Jan. 18, 2000 (PCT/US00/01138); and "SINGLE-STRANDED NUCLEIC ACID TEMPLATE-MEDIATED RECOMBINATION AND NUCLEIC ACID FRAGMENT ISOLATION" by Affholter, filed Sep. 6, 2000 (U.S. Ser. No. 09/656,549); and United States Patent Nos. 6,177,263; 6,153,410.

Non-stochastic, or "directed evolution," methods include, e.g., Gene Site Saturation Mutagenesis (GSSM), synthetic ligation reassembly (SLR), or a combination thereof are used to modify the nucleic acids of the invention to generate polypeptides involved in chlorophyll catabolism or having an esterase (e.g., chlorophyllase) activity with new or altered properties (e.g., activity under highly acidic or alkaline conditions, high or low temperatures, and the like). Polypeptides encoded by the modified nucleic acids can be screened for an activity before testing for glucan or other polysaccharide hydrolysis or other activity. Any testing modality or protocol can be used, e.g., using a capillary array platform. See, e.g., U.S. Patent Nos. 6,361,974; 6,280,926; 5,939,250.

### Gene Site Saturafion Mutagenesis (GSSM)

Codon primers containing a degenerate N;N,G/T sequence are used to introduce point mutations into a polynucleotide, e.g., a nucleic acid of the invention, so as to generate a set of progeny polypeptides in which a full range of single amino acid substitutions is represented at each amino acid position, e.g., an amino acid residue in an enzyme active site (catalytic domains (CDs)) or ligand binding site targeted to be modified. These oligonucleotides can comprise a contiguous first homologous sequence, a degenerate N,N,G/T sequence, and, optionally, a second homologous sequence. The downstream progeny translational products from the use of such oligonucleotides include all possible amino acid changes at each amino acid site along the polypeptide, because the degeneracy of the N,N,G/T sequence includes codons for all 20 amino acids. One such degenerate oligonucleotide (comprised of, e.g., one degenerate N,N,G/T cassette) is used for subjecting each original codon in a parental polynucleotide template to a full range of codon substitutions.

Also disclosed are the use of proprietary codon primers (containing a degenerate N,N,N sequence) to introduce point mutations into a polynucleotide, so as to generate a set of progeny polypeptides in which a full range of single amino acid substitutions is represented at each amino acid position (Gene Site Saturation Mutagenesis™ (GSSM™)). The oligos used are comprised contiguously of a first homologous sequence, a degenerate N,N,N sequence and in one aspect but not necessarily a second homologous sequence. The downstream progeny translational products from the use of such oligos include all possible amino acid changes at each amino acid site along the polypeptide, because the degeneracy of the N,N,N sequence includes codons for all 20 amino acids.

### Synthetic Ligation Reassembly (SLR)

The disclosure provides a non-stochastic gene modification system termed "synthetic ligation reassembly," or simply "SLR," a "directed evolution process," to generate polypeptides, e.g., enzymes of the invention, with new or altered properties.

SLR is a method of ligating oligonucleotide fragments together non-stochastically. This method differs from stochastic oligonucleotide shuffling in that the nucleic acid building blocks are not shuffled, concatenated or chimerized randomly, but rather are assembled non-stochastically. See, e.g., U.S. Patent Application Serial No. (USSN) 09/332,835 entitled "Synthetic Ligation Reassembly in Directed Evolution" and filed on June 14, 1999 ("USSN 09/332,835"). SLR comprises the following steps: (a) providing a template polynucleotide, wherein the template polynucleotide comprises sequence encoding a homologous gene; (b) providing a plurality of building block polynucleotides, wherein the building block polynucleotides are designed to cross-over reassemble with the template polynucleotide at a predetermined sequence, and a building block polynucleotide comprises a sequence that is a variant of the homologous gene and a sequence homologous to the template polynucleotide flanking the variant sequence; (c) combining a building block polynucleotide with a template polynucleotide such that the building block polynucleotide cross-over reassembles with the template polynucleotide to generate polynucleotides comprising homologous gene sequence variations.

### Optimized Directed Evolution System

The invention provides a non-stochastic gene modification system termed "optimized directed evolution system" to generate polypeptides, e.g., enzymes or antibodies of the invention, with new or altered properties. Optimized directed evolution is directed to the use of repeated cycles of reductive reassortment, recombination and selection that allow for the directed molecular evolution of nucleic acids through recombination. Optimized directed evolution allows generation of a large population of evolved chimeric sequences, wherein the generated population is significantly enriched for sequences that have a predetermined number of crossover events.

### Determining Crossover Events

Described herein is a system and software that receive a desired crossover probability density function (PDF), the number of parent genes to be reassembled, and the number of fragments in the reassembly as inputs. The output of this program is a "fragment PDF" that can be used to determine a recipe for producing reassembled genes, and the estimated crossover PDF of those genes. The processing described herein is performed in MATLAB™ (The Mathworks, Natick, Massachusetts) a programming language and development environment for technical computing.

### Iterative Processes

In practicing the invention, these processes can be iteratively repeated. For example, a nucleic acid (or, the nucleic acid) responsible for an altered or new phenotype is identified, re-isolated (e.g., using a nucleic acid of the invention), again modified, re-tested for activity. This process can be iteratively repeated until a desired phenotype is engineered. For example, an entire biochemical anabolic or catabolic pathway can be engineered into a cell, including, e.g., new or altered biosynthetic or (e.g., chlorophyll) degradative pathway.

Similarly, if it is determined that a particular oligonucleotide has no affect at all on the desired trait (e.g., a new or altered biosynthetic or (e.g., chlorophyll) degradative pathway phenotype), it can be removed as a variable by synthesizing larger parental oligonucleotides that include the sequence to be removed. Since incorporating the sequence within a larger sequence prevents any crossover events, there will no longer be any variation of this sequence in the progeny polynucleotides. This iterative practice of determining which oligonucleotides are most related to the desired trait, and which are unrelated, allows more efficient exploration all of the possible protein variants that might be provide a particular trait or activity.

### In vivo shuffling

*In vivo* shuffling of molecules is use in methods of the invention that provide variants of polypeptides for use according to the invention, e.g., antibodies, enzymes and the like. *In vivo* shuffling can be performed utilizing the natural property of cells to recombine multimers. While recombination *in vivo* has provided the major natural route to molecular diversity, genetic recombination remains a relatively complex process that involves 1) the recognition of homologies; 2) strand cleavage, strand invasion, and metabolic steps leading to the production of recombinant chiasma; and finally 3) the resolution of chiasma into discrete recombined molecules. The formation of the chiasma requires the recognition of homologous sequences.

### Producing sequence variants

The disclosure also provides additional methods for making sequence variants of the nucleic acid sequences in a use according to the invention. The dislosurealso provides additional methods for isolating polypeptides. The disclosure provides for variants of coding sequences (e.g., a gene, cDNA or message), which can be altered by any means, including, e.g., random or stochastic methods, or, non-stochastic, or "directed evolution," methods, as described above.

The isolated variants may be naturally occurring. Variant can also be created *in vitro.* Variants may be created using genetic engineering techniques such as site directed mutagenesis, random chemical mutagenesis, Exonuclease III deletion procedures, and standard cloning techniques. Alternatively, such variants, fragments, analogs, or derivatives may be created using chemical synthesis or modification procedures. Other methods of making variants are also familiar to those skilled in the art. These include procedures in which nucleic acid sequences obtained from natural isolates are modified to generate nucleic acids which encode polypeptides having characteristics which enhance their value in industrial or laboratory applications. In such procedures, a large number of variant sequences having one or more nucleotide differences with respect to the sequence obtained from the natural isolate are generated and characterized. These nucleotide differences can result in amino acid changes with respect to the polypeptides encoded by the nucleic acids from the natural isolates.

For example, variants may be created using error prone PCR. In error prone PCR, PCR is performed under conditions where the copying fidelity of the DNA polymerase is low, such that a high rate of point mutations is obtained along the entire length of the PCR product. Error prone PCR is described, e.g., in Leung, D.W., et al., Technique, 1:11-15, 1989) and Caldwell, R. C. & Joyce G.F., PCR Methods Applic., 2:28-33, 1992. Briefly, in such procedures, nucleic acids to be mutagenized are mixed with PCR primers, reaction buffer, MgCl₂, MnCl₂, Taq polymerase and an appropriate concentration of dNTPs for achieving a high rate of point mutation along the entire length of the PCR product. For example, the reaction may be performed using 20 fmoles of nucleic acid to be mutagenized, 30 pmole of each PCR primer, a reaction buffer comprising 50mM KCI, 10mM Tris HCl (pH 8.3) and 0.01% gelatin, 7mM MgCl2, 0.5mM MnCl₂, 5 units of Taq polymerase, 0.2mM dGTP, 0.2mM dATP, 1 mM dCTP, and 1 mM dTTP. PCR may be performed for 30 cycles of 94°C for 1 min, 45°C for 1 min, and 72°C for 1 min. However, it will be appreciated that these parameters may be varied as appropriate. The mutagenized nucleic acids are cloned into an appropriate vector and the activities of the polypeptides encoded by the mutagenized nucleic acids are evaluated.

Variants may also be created using oligonucleotide directed mutagenesis to generate site-specific mutations in any cloned DNA of interest. Oligonucleotide mutagenesis is described, e.g., in Reidhaar-Olson (1988) Science 241:53-57. Briefly, in such procedures a plurality of double stranded oligonucleotides bearing one or more mutations to be introduced into the cloned DNA are synthesized and inserted into the cloned DNA to be mutagenized. Clones containing the mutagenized DNA are recovered and the activities of the polypeptides they encode are assessed.

Another method for generating variants is assembly PCR. Assembly PCR involves the assembly of a PCR product from a mixture of small DNA fragments. A large number of different PCR reactions occur in parallel in the same vial, with the products of one reaction priming the products of another reaction. Assembly PCR is described in, e.g., U.S. Patent No. 5,965,408.

Still another method of generating variants is sexual PCR mutagenesis. In sexual PCR mutagenesis, forced homologous recombination occurs between DNA molecules of different but highly related DNA sequence *in vitro,* as a result of random fragmentation of the DNA molecule based on sequence homology, followed by fixation of the crossover by primer extension in a PCR reaction. Sexual PCR mutagenesis is described, e.g., in Stemmer (1994) Proc. Natl. Acad. Sci. USA 91:10747-10751. Briefly, in such procedures a plurality of nucleic acids to be recombined are digested with DNase to generate fragments having an average size of 50-200 nucleotides. Fragments of the desired average size are purified and resuspended in a PCR mixture. PCR is conducted under conditions which facilitate recombination between the nucleic acid fragments. For example, PCR may be performed by resuspending the purified fragments at a concentration of 10-30ng/µl in a solution of 0.2mM of each dNTP, 2.2mM MgCl₂, 50mM KCL, 10mM Tris HCl, pH 9.0, and 0.1% Triton X-100. 2.5 units of Taq polymerase per 100:l of reaction mixture is added and PCR is performed using the following regime: 94°C for 60 seconds, 94°C for 30 seconds, 50-55°C for 30 seconds, 72°C for 30 seconds (30-45 times) and 72°C for 5 minutes. However, it will be appreciated that these parameters may be varied as appropriate. For instance, oligonucleotides may be included in the PCR reactions. Alternatively, the Klenow fragment of DNA polymerase I may be used in a first set of PCR reactions and Taq polymerase may be used in a subsequent set of PCR reactions. Recombinant sequences are isolated and the activities of the polypeptides they encode are assessed.

Variants may also be created by *in vivo* mutagenesis. For instance, random mutations in a sequence of interest are generated by propagating the sequence of interest in a bacterial strain, such as an E. coli strain, which carries mutations in one or more of the DNA repair pathways. Such "mutator" strains have a higher random mutation rate than that of a wild-type parent. Propagating the DNA in one of these strains will eventually generate random mutations within the DNA. Mutator strains suitable for use for *in vivo* mutagenesis are described in PCT Publication No. WO 91/16427, published October 31, 1991, entitled "Methods for Phenotype Creation from Multiple Gene Populations".

Variants may also be generated using cassette mutagenesis. In cassette mutagenesis a small region of a double stranded DNA molecule is replaced with a synthetic oligonucleotide "cassette" that differs from the native sequence. The oligonucleotide often contains completely and/or partially randomized native sequence.

Recursive ensemble mutagenesis may also be used to generate variants. Recursive ensemble mutagenesis is an algorithm for protein engineering (protein mutagenesis) developed to produce diverse populations of phenotypically related mutants whose members differ in amino acid sequence. This method uses a feedback mechanism to control successive rounds of combinatorial cassette mutagenesis. Recursive ensemble mutagenesis is described in Arkin, A.P. and Youvan, D.C., PNAS, USA, 89:7811-7815, 1992.

For instance, variants are created using exponential ensemble mutagenesis. Exponential ensemble mutagenesis is a process for generating combinatorial libraries with a high percentage of unique and functional mutants, wherein small groups of residues are randomized in parallel to identify, at each altered position, amino acids which lead to functional proteins. Exponential ensemble mutagenesis is described in Delegrave, S. and Youvan, D.C., Biotechnology Research, 11:1548-1552, 1993. Random and site-directed mutagenesis are described in Arnold, F.H., Current Opinion in Biotechnology, 4.450-455, 1993.

For instance, the variants are created using shuffling procedures wherein portions of a plurality of nucleic acids which encode distinct polypeptides are fused together to create chimeric nucleic acid sequences which encode chimeric polypeptides as described in U.S. Patent No. 5,965,408, filed July 9, 1996, entitled, "Method of DNA Reassembly by Interrupting Synthesis" and U.S. Patent No. 5,939,250, filed May 22, 1996, entitled, "Production of Enzymes Having Desired Activities by Mutagenesis.

The variants of the polypeptides in a use according to the invention may be variants in which one or more of the amino acid residues of the polypeptides of the sequences of the invention are substituted with a conserved or non-conserved amino acid residue (in one aspect a conserved amino acid residue) and such substituted amino acid residue may or may not be one encoded by the genetic code.

The disclosure provides alternative embodiments of the polypeptides of the invention (and the nucleic acids that encode them) comprising at least one conservative amino acid substitution, as discussed herein (e.g., conservative amino acid substitutions are those that substitute a given amino acid in a polypeptide by another amino acid of like characteristics). The disclosure provides polypeptides (and the nucleic acids that encode them) wherein any, some or all amino acids residues are substituted by another amino acid of like characteristics, e.g., a conservative amino acid substitution.

Conservative substitutions are those that substitute a given amino acid in a polypeptide by another amino acid of like characteristics. Typically seen as conservative substitutions are the following replacements: replacements of an aliphatic amino acid such as Alanine, Valine, Leucine and Isoleucine with another aliphatic amino acid; replacement of a Serine with a Threonine or vice versa; replacement of an acidic residue such as Aspartic acid and Glutamic acid with another acidic residue; replacement of a residue bearing an amide group, such as Asparagine and Glutamine, with another residue bearing an amide group; exchange of a basic residue such as Lysine and Arginine with another basic residue; and replacement of an aromatic residue such as Phenylalanine, Tyrosine with another aromatic residue. Alternatively, these conservative substitutions can also be synthetic equivalents of these amino acids.

Other variants are those in which one or more of the amino acid residues of a polypeptide of the invention includes a substituent group.

Still other variants are those in which the polypeptide is associated with another compound, such as a compound to increase the half-life of the polypeptide (for example, polyethylene glycol).

Additional variants are those in which additional amino acids are fused to the polypeptide, such as a leader sequence, a secretory sequence, a proprotein sequence or a sequence which facilitates purification, enrichment, or stabilization of the polypeptide.

The fragments, derivatives and analogs retain the same biological function or activity as the polypeptides that are used in the invention. The fragment, derivative, or analog includes a proprotein, such that the fragment, derivative, or analog can be activated by cleavage of the proprotein portion to produce an active polypeptide.

### Optimizing codons to achieve high levels of protein expression in host cells

The disclosure provides methods for modifying nucleic acids encoding polypeptides involved in chlorophyll catabolism or having an esterase (e.g., chlorophyllase) activity by modifying codon usage. The disclosure provides methods for modifying codons in a nucleic acid encoding a polypeptide to increase or decrease its expression in a host cell. The disclosure also provides nucleic acids encoding polypeptides involved in chlorophyll catabolism or having an esterase (e.g., chlorophyllase) activity modified to increase its expression in a host cell, enzymes so modified, and methods of making the modified polypeptides involved in chlorophyll catabolism or having an esterase (e.g., chlorophyllase) activity. The method comprises identifying a "non-preferred" or a "less preferred" codon in enzyme-encoding nucleic acid and replacing one or more of these non- preferred or less preferred codons with a "preferred codon" encoding the same amino acid as the replaced codon and at least one non- preferred or less preferred codon in the nucleic acid has been replaced by a preferred codon encoding the same amino acid. A preferred codon is a codon over-represented in coding sequences in genes in the host cell and a non- preferred or less preferred codon is a codon under-represented in coding sequences in genes in the host cell.

Host cells for expressing the nucleic acids, expression cassettes and vectors of the invention include bacteria, yeast, fungi, plant cells, insect cells and mammalian cells. Thus, the invention provides methods for optimizing codon usage in all of these cells, codon-altered nucleic acids and polypeptides made by the codon-altered nucleic acids. Exemplary host cells include gram negative bacteria, such as *Escherichia coli;* gram positive bacteria, such as *Streptomyces, Lactobacillus gasseri, Lactococcus lactis, Lactococcus cremoris, Bacillus* sp., *Bacillus subtilis, Bacillus cereus.* Exemplary host cells also include eukaryotic organisms, e.g., various yeast, such as *Saccharomyces* sp., including *Saccharomyces cerevisiae, Schizosaccharomyces pombe, Pichia pastoris,* and *Kluyveromyces lactis, Hansenula polymorpha, Aspergillus niger,* and mammalian cells and cell lines and insect cells and cell lines. Thus, the disclosure also includes nucleic acids and polypeptides optimized for expression in these organisms and species, e.g., the nucleic acids are codon-optimized for expression in a host cell, e.g., a *Pichia* sp., e.g., *P. pastoris, a Saccharomyces* sp., or a *Bacillus* sp., a *Streptomyces* sp., and the like.

For example, the codons of a nucleic acid encoding a polypeptide of the invention or a similar enzyme isolated from a bacterial cell are modified such that the nucleic acid (encoding the enzyme) is optimally expressed in a bacterial cell different from the bacteria from which the enzyme (e.g., a polypeptide of the invention) was derived, a yeast, a fungi, a plant cell, an insect cell or a mammalian cell. Methods for optimizing codons are well known in the art, see, e.g., U.S. Patent No. 5,795,737; Baca (2000) int. J. Parasitol. 30:113-118; Hale (1998) Protein Expr. Purif. 12:185-188; Narum (2001) Infect. lmmun. 69:7250-7253. See also Narum (2001) Infect. Immun. 69:7250-7253, describing optimizing codons in mouse systems; Outchkourov (2002) Protein Expr. Purif. 24:18-24, describing optimizing codons in yeast; Feng (2000) Biochemistry 39:15399-15409, describing optimizing codons in *E. coli;* Humphreys (2000) Protein Expr. Purif. 20:252-264, describing optimizing codon usage that affects secretion in E. coli; Gao (2004) Biotechnol Prog. 20:443-448, describing "UpGene", an application of a web-based DNA codon optimization algorithm.

### Transgenic non-human animals

The disclosure provides transgenic non-human animals comprising a nucleic acid, a polypeptide, an expression cassette or vector or a transfected or transformed cell of the invention. The disclosure also provides methods of making and using these transgenic non-human animals.

. Transgenic non-human animals can be designed and generated using any method known in the art; see, e.g., U.S. Patent Nos. 6,211,428; 6,187,992; 6,156,952; 6,118,044; 6,111,166; 6,107,541; 5,959,171; 5,922,854; 5,892,070; 5,880,327; 5,891,698; 5,639,940; 5,573,933; 5,387,742; 5,087,571, describing making and using transformed cells and eggs and transgenic mice, rats, rabbits, sheep, pigs and cows. See also, e.g., Pollock (1999) J. Immunol. Methods 231:147-157, describing the production of recombinant proteins in the milk of transgenic dairy animals; Baguisi (1999) Nat. Biotechnol. 17:456-461, demonstrating the production of transgenic goats. "Knockout animals" can also be used to practice the methods of the disclosed herein

### Transgenic Plants and Seeds

The disclosure provides transgenic plants and seeds comprising a nucleic acid, a polypeptide (e.g., a polypeptide involved in chlorophyll catabolism or having an esterase (e.g., chlorophyllase) activity), an expression cassette or vector or a transfected or transformed celldisclosed herein. The disclosure also provides plant products, e.g., oils, seeds, leaves, extracts and the like, comprising a nucleic acid and/or a polypeptide disclosed herein. The transgenic plant can be dicotyledonous (a dicot) or monocotyledonous (a monocot). The disclosure also provides methods of making and using these transgenic plants and seeds. The transgenic plant or plant cell expressing a polypeptide disclosed herein may be constructed in accordance with any method known in the art. See, for example, U.S. Patent No. 6,309,872.

The disclosure also provides "knockout plants" where insertion of gene sequence by, e.g., homologous recombination, has disrupted the expression of the endogenous gene. Means to generate "knockout" plants are well-known in the art, see, e.g., Strepp (1998) Proc Natl. Acad. Sci. USA 95:4368-4373; Miao (1995) Plant J 7:359-365. See discussion on transgenic plants, below.

The nucleic acids of the invention can be used to confer desired traits on essentially any plant, e.g., on starch-producing plants, such as potato, wheat, rice, barley, and the like. Nucleic acids of the invention can be used to manipulate metabolic pathways of a plant in order to optimize or alter host's expression of a polypeptide of the invention or a homologous enzyme in the host. This can change enzyme (e.g., chlorophyllase) activity or biosynthetic pathway product (a chlorophyll degradative pathway) in the plant. Alternatively, an enzyme or nucleic acid of the invention can be used in production of a transgenic plant to produce a compound not naturally produced by that plant. This can lower production costs or create a novel product.

Making transgenic plants or seeds comprises incorporating sequences of the invention and, optionally, marker genes into a target expression construct (e.g., a plasmid), along with positioning of the promoter and the terminator sequences. This can involve transferring the modified gene into the plant through a suitable method. For example, a construct may be introduced directly into the genomic DNA of the plant cell using techniques such as electroporation and microinjection of plant cell protoplasts, or the constructs can be introduced directly to plant tissue using ballistic methods, such as DNA particle bombardment. For example, see, e.g., Christou (1997) Plant Mol. Biol. 35:197-203; Pawlowski (1996) Mol. Biotechnol. 6:17-30; Klein (1987) Nature 327:70-73; Takumi (1997) Genes Genet. Syst. 72:63-69, discussing use of particle bombardment to introduce transgenes into wheat; and Adam (1997) supra, for use of particle bombardment to introduce YACs into plant cells.

The disclosure provides for the transformation of monocotyledonous plants using the nucleic acids of the invention, including important cereals, see Hiei (1997) Plant Mol. Biol. 35:205-218. See also, e.g., Horsch, Science (1984) 233:496; Fraley (1983) Proc. Natl. Acad. Sci USA 80:4803; Thykjaer (1997) supra; Park (1996) Plant Mol. Biol. 32:1135-1148, discussing T-DNA integration into genomic DNA. See also D'Halluin, U.S. Patent No. 5,712,135, describing a process for the stable integration of a DNA comprising a gene that is functional in a cell of a cereal, or other monocotyledonous plant.

The third step can involve selection and regeneration of whole plants capable of transmitting the incorporated target gene to the next generation. Such regeneration techniques rely on manipulation of certain phytohormones in a tissue culture growth medium, typically relying on a biocide and/or herbicide marker that has been introduced together with the desired nucleotide sequences. Plant regeneration from cultured protoplasts is described in Evans et al., Protoplasts Isolation and Culture, Handbook of Plant Cell Culture, pp. 124-176, MacMillilan Publishing Company, New York, 1983; and Binding, Regeneration of Plants, Plant Protoplasts, pp. 21-73, CRC Press, Boca Raton, 1985. Regeneration can also be obtained from plant callus, explants, organs, or parts thereof. Such regeneration techniques are described generally in Klee (1987) Ann. Rev. of Plant Phys. 38:467-486. To obtain whole plants from transgenic tissues such as immature embryos, they can be grown under controlled environmental conditions in a series of media containing nutrients and hormones, a process known as tissue culture. Once whole plants are generated and produce seed, evaluation of the progeny begins.

After the expression cassette is stably incorporated in transgenic plants, it can be introduced into other plants by sexual crossing. Any of a number of standard breeding techniques can be used, depending upon the species to be crossed. Since transgenic expression of the nucleic acids of the invention leads to phenotypic changes, plants comprising the recombinant nucleic acids of the invention can be sexually crossed with a second plant to obtain a final product. Thus, the seed disclosed herein can be derived from a cross between two transgenic plants of the invention, or a cross between a plant of the invention and another plant. The desired effects (e.g., expression of the polypeptides of the invention to produce a plant in which flowering or seeding behavior is altered) can be enhanced when both parental plants express the polypeptides of the invention. The desired effects can be passed to future plant generations by standard propagation means.

The nucleic acids and polypeptides disclosed herein are expressed in or inserted in any plant or seed. Transgenic plants of the invention can be dicotyledonous or monocotyledonous. Examples of monocot transgenic plants of the invention are grasses, such as meadow grass (blue grass, Poa), forage grass such as festuca, lolium, temperate grass, such as *Agrostis,* and cereals, e.g., wheat, oats, rye, barley, rice, sorghum, and maize (corn). Examples of dicot transgenic plants of the invention are tobacco, legumes, such as lupins, potato, sugar beet, pea, bean and soybean, and cruciferous plants (family *Brassicaceae),* such as cauliflower, rape seed, and the closely related model organism *Arabidopsis thaliana.* Thus, the transgenic plants and seeds of the invention include a broad range of plants, including, but not limited to, species from the genera *Anacardium, Arachis, Asparagus, Atropa, Avena, Brassica, Citrus, Citrullus, Capsicum, Carthamus, Cocos, Coffea, Cucumis, Cucurbita, Daucus, Elaeis, Fragaria, Glycine, Gossypium, Helianthus, Heterocallis, Hordeum, Hyoscyamus, Lactuca, Linum, Lolium, Lupinus, Lycopersicon, Malus, Manihot, Majorana, Medicago, Nicotiana, Olea, Oryza, Panieum, Pannisetum, Persea, Phaseolus, Pistachia, Pisum, Pyrus, Prunus, Raphanus, Ricinus, Secale, Senecio, Sinapis, Solanum, Sorghum, Theobromus, Trigonella, Triticum, Vicia, Vitis, Vigna,* and *Zea*.

Alternatively the nucleic acids are expressed in plants which contain fiber cells, including, e.g., cotton, silk cotton tree (Kapok, *Ceiba penfandra*), desert willow, creosote bush, winterfat, balsa, ramie, kenaf, hemp, roselle, jute, sisal abaca and flax. In alternative embodiments, the transgenic plants of the invention can be members of the genus *Gossypium,* including members of any *Gossypium* species, such as *G. arboreum; G. herbaceum, G*. *barbadense,* and *G. hirsutum.*

The disclosure also provides for transgenic plants to be used for producing large amounts of the polypeptides (e.g., enzymes or antibody) of the invention. For example, see Palmgren (1997) Trends Genet. 13:348; Chong (1997) Transgenic Res. 6:289-296 (producing human milk protein beta-casein in transgenic potato plants using an auxin-inducible, bidirectional mannopine synthase (mas1',2') promoter with *Agrobacterium tumefaciens-mediated* leaf disc transformation methods).

Using known procedures, one of skill can screen for plants of the invention by detecting the increase or decrease of transgene mRNA or protein in transgenic plants. Means for detecting and quantitation of mRNAs or proteins are well known in the art.

### Polypeptides and peptides

The disclosure provides isolated, synthetic or recombinant polypeptides having a sequence identity (e.g., at least about 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 99.5% or more, or complete (100%) sequence identity) to an exemplary sequence of the invention, e.g., proteins having a sequence as set forth in SEQ ID NO:2, SEQ ID NO:4, SEQ ID NO:6, SEQ ID NO:8, SEQ ID NO:10, SEQ ID NO:12, SEQ ID NO:14, SEQ ID NO:16, SEQ ID N0:18 or SEQ ID NO:20.

A polypeptide that is used in the invention has an esterase activity, such as a chlorophyllase (a chlase) activity, or, has an enzyme activity comprising enzymatic modification of a chlorophyll molecule, e.g., wherein the enzymatic modification comprises catabolism of the chlorophyll molecule. For instance, the esterase activity comprises a chlorophyll chlorophyllido-hydrolyase activity.

Alternatively an isolated, synthetic or recombinant polypeptide or peptide including at least 10, 15, 20, 25, 30, 35, 40, 45, 50, 55, 60, 65, 70, 75, 80, 85, 90, 95 or 100 or more consecutive bases of a polypeptide or peptide sequence disclosed herein, sequences substantially identical thereto, and the sequences complementary thereto. The peptide can be, e.g., an immunogenic fragment, a motif (e.g., a binding site), a signal sequence, a prepro sequence or a catalytic domains (CDs) or active site.

The disclosure also provides chimeric polypeptides (and the nucleic acids encoding them) comprising at least two enzymes disclosed herein or subsequences thereof, e.g., active sites, or catalytic domains (CDs). A chimeric protein disclosed herein (e.g., a fusion protein, or, other heterodimer, e.g., two domains joined by other means, e.g., a linker, or, electrostatically) can comprise one polypeptide (e.g., active site or catalytic domain peptide) disclosed herein and another polypeptide (e.g., active site or catalytic domain peptide) disclosed herein or other polypeptide. For example, a chimeric protein disclosed hererin can have any activity of a polypeptide involved chlorophyll catabolism or having an esterase (e.g., chlorophyllase) activity, e.g., as described herein. The chimeric protein disclosed herein comprises a fusion of domains, e.g., a single domain can exhibit one or any combination of activities.

The polypeptides disclosed herein include enzymes in an active or inactive form. For example, the polypeptides disclosed herein include proproteins before "maturation" or processing of prepro sequences, e.g., by a proprotein-processing enzyme, such as a proprotein convertase to generate an "active" mature protein. The polypeptides disclosed herein include enzymes inactive for other reasons, e.g., before "activation" by a post-translational processing event, e.g., an endo- or exo-peptidase or proteinase action, a phosphorylation event, an amidation, a glycosylation or a sulfation, a dimerization event, and the like. The polypeptides disclosed herein include all active forms, including active subsequences, e.g., catalytic domains or active sites, of the enzymes.

Methods for identifying "prepro" domain sequences and signal sequences are well known in the art, see, e.g., Van de Ven (1993) Crit. Rev. Oncog. 4(2):115-136. For example, to identify a prepro sequence, the protein is purified from the extracellular space and the N-terminal protein sequence is determined and compared to the unprocessed form.

The disclosure includes polypeptides with or without a signal sequence and/or a prepro sequence. The disclosure includes polypeptides with heterologous signal sequences and/or prepro sequences. The prepro sequence (including a sequence of the invention used as a heterologous prepro domain) can be located on the amino terminal or the carboxy terminal end of the protein. The disclosure also includes isolated or recombinant signal sequences, prepro sequences and catalytic domains (e.g., "active sites") comprising sequences disclosed hererin.

The percent sequence identity can be over the full length of the polypeptide, or, the identity can be over a region of at least about 50, 60, 70, 80, 90, 100, 150, 200, 250, 300, 350, 400, 450, 500, 550, 600, 650, 700 or more residues. Polypeptides of the invention can also be shorter than the full length of exemplary polypeptides. Alternatively, herein provided are polypeptides (peptides, fragments) ranging in size between about 5 and the full length of a polypeptide, e.g., an enzyme, of the invention; exemplary sizes being of about 5, 10, 15, 20, 25, 30, 35, 40, 45, 50, 55, 60, 65, 70, 75, 80, 85, 90, 100, 125, 150, 175, 200, 250, 300, 350, 400, 450, 500, 550, 600, 650, 700, or more residues, e.g., contiguous residues of an exemplary enzymes disclosed herein.

Peptides of the present disclosure (e.g., a subsequence of an exemplary polypeptide of the invention) can be useful as, e.g., labeling probes, antigens, toleragens, motifs, enzyme active sites (e.g., "catalytic domains" of enzymes of the invention), binding sites of enzymes of the invention, signal sequences and/or prepro domains.

Polypeptides and peptides in a use according to the invention can be isolated from natural sources, be synthetic, or be recombinantly generated polypeptides. Peptides and proteins can be recombinantly expressed *in vitro* or *in vivo.* The peptides and polypeptides in a use according to the invention can be made and isolated using any method known in the art. Polypeptide and peptides that are used according to the invention can also be synthesized, whole or in part, using chemical methods well known in the art. See e.g., Caruthers (1980) Nucleic Acids Res. Symp. Ser. 215-223; Horn (1980) Nucleic Acids Res. Symp. Ser. 225-232; Banga, A.K., Therapeutic Peptides and Proteins, Formulation, Processing and Delivery Systems (1995) Technomic Publishing Co., Lancaster, PA. For example, peptide synthesis can be performed using various solid-phase techniques (see e.g., Roberge (1995) Science 269:202; Merrifield (1997) Methods Enzymol. 289:3-13) and automated synthesis may be achieved, e.g., using the ABI 431A Peptide Synthesizer (Perkin Elmer) in accordance with the instructions provided by the manufacturer.

The peptides and polypeptides in a use according to the invention can also be glycosylated. The glycosylation can be added post-translationally either chemically or by cellular biosynthetic mechanisms, wherein the later incorporates the use of known glycosylation motifs, which can be native to the sequence or can be added as a peptide or added in the nucleic acid coding sequence. The glycosylation can be O-linked or N-linked.

The peptides and polypeptides as defined above, include all "mimetic" and "peptidomimetic" forms. The terms "mimetic" and "peptidomimetic" refer to a synthetic chemical compound which has substantially the same structural and/or functional characteristics of the polypeptides. The mimetic can be either entirely composed of synthetic, non-natural analogues of amino acids, or, is a chimeric molecule of partly natural peptide amino acids and partly non-natural analogs of amino acids. The mimetic can also incorporate any amount of natural amino acid conservative substitutions as long as such substitutions also do not substantially alter the mimetic's structure and/or activity. As with polypeptides which are conservative variants, routine experimentation will determine whether a mimetic is within the scope of the invention, i.e., that its structure and/or function is not substantially altered from an exemplary polypeptide of the invention. For instance, a mimetic composition is used in a composition, cell system or process of the invention (e.g., a host cell having a plasmid expressing at least one enzyme of the invention).

The disclosure also provides methods for modifying the polypeptides of the invention by either natural processes, such as post-translational processing (e.g., phosphorylation, acylation, etc), or by chemical modification techniques, and the resulting modified polypeptides. Modifications can occur anywhere in the polypeptide, including the peptide backbone, the amino acid side-chains and the amino or carboxyl termini. See, e.g., Creighton, T.E., Proteins - Structure and Molecular Properties 2nd Ed., W.H. Freeman and Company, New York (1993); Posttranslational Covalent Modification of Proteins, B.C. Johnson, Ed., Academic Press, New York, pp. 1-12 (1983).

Solid-phase chemical peptide synthesis methods can also be used to synthesize the polypeptide or fragments of the invention. Such method have been known in the art since the early 1960's (Merrifield, R. B., J. Am. Chem. Soc., 85:2149-2154, 1963) (See also Stewart, J. M. and Young, J. D., Solid Phase Peptide Synthesis, 2nd Ed., Pierce Chemical Co., Rockford, III., pp. 11-12)) and have recently been employed in commercially available laboratory peptide design and synthesis kits (Cambridge Research Biochemicals). Such commercially available laboratory kits have generally utilized the teachings of H. M. Geysen et al, Proc. Natl. Acad. Sci., USA, 81:3998 (1984) and provide for synthesizing peptides upon the tips of a multitude of "rods" or "pins" all of which are connected to a single plate. When such a system is utilized, a plate of rods or pins is inverted and inserted into a second plate of corresponding wells or reservoirs, which contain solutions for attaching or anchoring an appropriate amino acid to the pin's or rod's tips. By repeating such a process step, i.e., inverting and inserting the rod's and pin's tips into appropriate solutions, amino acids are built into desired peptides. In addition, a number of available FMOC peptide synthesis systems are available. For example, assembly of a polypeptide or fragment can be carried out on a solid support using an Applied Biosystems, Inc. Model 431A™ automated peptide synthesizer. Such equipment provides ready access to the peptides of the invention, either by direct synthesis or by synthesis of a series of fragments that can be coupled using other known techniques.

The disclosure includes polypeptides with and without signal. The polypeptide comprising a signal sequence can be a polypeptide disclosed herein or another polypeptide.

The disclosure includes immobilized polypeptides in a use according to the invention, including enzymes, antibodies and fragments thereof. The disclosure provides methods for inhibiting polypeptide activity, e.g., using dominant negative mutants or antibodies of the invention. The disclosure includes heterocomplexes, e.g., fusion proteins, heterodimers, etc., comprising the enzymes of the invention.

Polypeptides in a use according to the invention can have enzyme activity under various conditions, e.g., extremes in pH and/or temperature, oxidizing agents, and the like. Disclosed are methods leading to alternative enzyme preparations with different catalytic efficiencies and stabilities, e.g., towards temperature, oxidizing agents and changing wash conditions. Enzyme variants can be produced using techniques of site-directed mutagenesis and/or random mutagenesis. Directed evolution can be used to produce a great variety of enzyme variants with alternative specificities and stability.

The polypeptides or fragments having homology to one of the polypeptides of the invention, or a fragment comprising at least about 5, 10, 15, 20, 25, 30, 35, 40, 50, 75, 100, or 150 consecutive amino acids thereof may be obtained by isolating the nucleic acids encoding them using the techniques described above.

Alternatively, the homologous polypeptides or fragments may be obtained through biochemical enrichment or purification procedures. The sequence of potentially homologous polypeptides or fragments may be determined by activity assays, gel electrophoresis and/or microsequencing. The sequence of the prospective homologous polypeptide or fragment can be compared to one of the polypeptides of the invention, or a fragment comprising at least about 5, 10, 15, 20, 25, 30, 35, 40, 50, 75, 100, or 150 consecutive amino acids thereof using any of the programs described above.

Also disclosed is an assay for identifying fragments or variants of the invention, which retain the enzymatic function of the polypeptides of the invention. For example the fragments or variants of polypeptides of the invention may be used to catalyze biochemical reactions, which indicate that the fragment or variant retains the enzymatic activity of a polypeptide of the invention.

The assay for determining if fragments of variants retain the enzymatic activity of the polypeptides of the invention includes the steps of: contacting the polypeptide fragment or variant with a substrate molecule under conditions which allow the polypeptide fragment or variant to function and detecting either a decrease in the level of substrate or an increase in the level of the specific reaction product of the reaction between the polypeptide and substrate.

The polypeptides disclosed herein or fragments comprising at least 5, 10, 15, 20, 25, 30, 35, 40, 50, 75, 100, or 150 consecutive amino acids thereof may be used in a variety of applications. For example, the polypeptides or fragments thereof may be used to catalyze biochemical reactions. Accordingly, there is provided a process for utilizing the polypeptides of the invention or polynucleotides encoding such polypeptides for hydrolyzing ester linkages. In such procedures, a substance containing an ester linkage (e.g., a chlorophyll) is contacted with one of the polypeptides of the invention, or sequences substantially identical thereto under conditions which facilitate the hydrolysis of the ester linkage.

### Signal sequences, prepro, binding domains and catalytic domains

The disclosure provides enzyme signal sequences (e.g., signal peptides (SPs)), prepro domains, binding domains and catalytic domains (CDs) (e.g., active sites). The SPs, prepro domains and/or CDs of the invention can be isolated or recombinant peptides or can be part of a fusion protein, e.g., as a heterologous domain in a chimeric protein. The disclosure provides nucleic acids encoding these catalytic domains (CDs), prepro domains and signal sequences (SPs, e.g., a peptide having a sequence comprising/ consisting of amino terminal residues of a polypeptide of the invention). The present disclosure provides a signal sequence comprising a peptide comprising/ consisting of a sequence as set forth in residues 1 to 10, 1 to 11, 1 to 12, 1 to 13, 1 to 14, 1 to 15, 1 to 16, 1 to 17, 1 to 18, 1 to 19, 1 to 20, 1 to 21, 1 to 22, 1 to 23, 1 to 24, 1 to 25, 1 to 26, 1 to 27, 1 to 28, 1 to 28, 1 to 30, 1 to 31, 1 to 32, 1 to 33, 1 to 34, 1 to 35, 1 to 36, 1 to 37, 1 to 38, 1 to 39, 1 to 40, 1 to 41, 1 to 42, 1 to 43, 1 to 44, 1 to 45, 1 to 46, 1 to 47, 1 to 48, 1 to 49, 1 to 50, 1 to 51, or 1 to 52 or more, of a polypeptide of the invention.

The disclosure also provides chimeric polypeptides (and the nucleic acids encoding them) comprising at least two enzymes of the invention or subsequences thereof, e.g., catalytic domains (CDs) or active sites. For example, a chimeric protein of the invention can have any combination of activities. The chimeric protein comprises a fusion of domains, e.g., a single domain can exhibit one or any combination of activities (e.g., as a recombinant chimeric protein).

The disclosure also provides isolated, synthetic or recombinant signal sequences comprising/ consisting of a signal sequence disclosed herein, e.g., exemplary signal sequences as set forth in Table 1, below, and polypeptides comprising these signal sequences. The polypeptide can be another enzyme disclosed herein, or another type of enzyme or polypeptide. For example, to aid in reading Table 1, the invention provides an isolated, synthetic or recombinant signal sequence as set forth by the amino terminal amino acid residues 1 to 21 ("NH₂- MSRVCLPLTLTLALTLSARA") of SEQ ID NO: 2, encoded, e.g., by SEQ ID NO:1, etc.:

**Table 1**

| SEQ ID NO: | Signal sequence position (AA= Amino Acid | **Signal sequence** |
|---|---|---|
| 1, 2 11, 12 13, 14 15, 16 | AA1-20 | MSRVCLPLTLTLALTLSARA |
| 17, 18 19, 20 3, 4 | AA1-25 | MKKYKTGLVLSGGGTRGFAHLGVIA |
| 5, 6 | AA1 - 25 | MRRIVFLYILALLCVSCANRNPSVS |
| 7, 8 | AA1-51 | MTRKKIGLALSGGAARGFAHLGVLKVFAEHGIPVDFVAGTSAGSFAGAAFA |
| 9, 10 | AA1-23 | MFNKALPAAAAVAGLFLSTSAMA |

The signal sequences (SPs) and/or prepro sequences disclosed herein can be isolated peptides, or, sequences joined to another enzyme of the invention, or a heterologous protein, e.g., as a fusion (chimeric) protein. Further provided are polypeptides comprising signal sequences disclosed herein. Polypeptides comprising signal sequences SPs and/or prepro disclosed herein comprise sequences heterologous to enzymes disclosed herein (e.g., a fusion protein comprising an SP and/or prepro of the invention and/or sequences from another protein). The disclosure provides an enzyme disclosed herein with heterologous SPs and/or prepro sequences, e.g., sequences with a yeast signal sequence. Enzymes of the invention can comprise a heterologous SP and/or prepro in a vector, e.g., a pPIC series vector (Invitrogen, Carlsbad, CA). SPs and/or prepro sequences of the invention are identified following identification of novel polypeptides. The pathways by which proteins are sorted and transported to their proper cellular location are often referred to as protein targeting pathways. One of the most important elements in all of these targeting systems is a short amino acid sequence at the amino terminus of a newly synthesized polypeptide called the signal sequence. This signal sequence directs a protein to its appropriate location in the cell and is removed during transport or when the protein reaches its final destination. Most lysosomal, membrane, or secreted proteins have an amino-terminal signal sequence that marks them for translocation into the lumen of the endoplasmic reticulum. More than 100 signal sequences for proteins in this group have been determined. The signal sequences can vary in length from 13 to 36 amino acid residues. Various methods of recognition of signal sequences are known to those of skill in the art. For example, novel signal peptides are identified by a method referred to as SignalP. SignalP uses a combined neural network which recognizes both signal peptides and their cleavage sites. (Nielsen, et al., "Identification of prokaryotic and eukaryotic signal peptides and prediction of their cleavage sites." Protein Engineering, vol. 10, no. 1, p. 1-6 (1997).

It should be understood that an enzyme may not have SPs and/or prepro sequences, or one or more "domains." The disclosure provides an enzyme lacking all or part of an SP and/or a prepro domain. The disclosure provides a nucleic acid sequence encoding a signal sequence (SP) and/or prepro from one enzyme of the invention operably linked to a nucleic acid sequence of a different enzyme of the invention or, optionally, a signal sequence (SPs) and/or prepro domain from a different type of protein may be desired.

The disclosure also provides isolated or recombinant polypeptides comprising signal sequences (SPs), prepro domain and/or catalytic domains (CDs) of the invention and heterologous sequences. The heterologous sequences are sequences not naturally associated (e.g., to enzymes of the invention) with an SP, prepro domain and/or CD. The sequence to which the SP, prepro domain and/or CD are not naturally associated can be on the SP's, prepro domain and/or CD's amino terminal end, carboxy terminal end, and/or on both ends of the SP and/or CD. The present disclosure provides an isolated or recombinant polypeptide comprising (or consisting of) a polypeptide comprising a signal sequence (SP), prepro domain and/or catalytic domain (CD) of the invention with the proviso that it is not associated with any sequence to which it is naturally associated. Similarly the disclosure provides isolated or recombinant nucleic acids encoding these polypeptides. Thus, the isolated or recombinant nucleic acid disclosed herein comprises coding sequence for a signal sequence (SP), prepro domain and/or catalytic domain (CD) of the invention and a heterologous sequence (i.e., a sequence not naturally associated with the a signal sequence (SP), prepro domain and/or catalytic domain (CD) of the invention). The heterologous sequence can be on the 3' terminal end, 5' terminal end, and/or on both ends of the SP, prepro domain and/or CD coding sequence.

### Hybrid (chimeric) Enzymes and Peptide Libraries

The present disclosure provides hybrid enzymes of the invention and fusion proteins, including peptide libraries, comprising sequences of disclosed herein. The peptide libraries of the invention can be used to isolate peptide modulators (e.g., activators or inhibitors) of targets, such as enzyme of the invention, their substrates, etc. The peptide libraries of the invention can be used to identify formal binding partners of targets, such as ligands, e.g., cytokines, hormones and the like. The present disclosure provides chimeric proteins comprising a signal sequence (SP), prepro domain and/or catalytic domain (CD) of the invention or a combination thereof and a heterologous sequence (see above).

"Amino acid" or "amino acid sequence" as used herein refer to an oligopeptide, peptide, polypeptide, or protein sequence, or to a fragment, portion, or subunit of any of these and to naturally occurring or synthetic molecules.

"Amino acid" or "amino acid sequence" include an oligopeptide, peptide, polypeptide, or protein sequence, or to a fragment, portion, or subunit of any of these, and to naturally occurring or synthetic molecules. The term "polypeptide" as used herein, refers to amino acids joined to each other by peptide bonds or modified peptide bonds, i.e., peptide isosteres and may contain modified amino acids other than the 20 gene-encoded amino acids. The polypeptides may be modified by either natural processes, such as post-translational processing, or by chemical modification techniques which are well known in the art. (*See* Creighton, T.E., Proteins - Structure and Molecular Properties 2nd Ed., W.H. Freeman and Company, New York (1993); Posttranslational Covalent Modification of Proteins, B.C. Johnson, Ed., Academic Press, New York, pp. 1-12 (1983)).

Enzymes disclosed herein comprise epitopes or purification tags, signal sequences or other fusion sequences, etc. Enzymes can be fused to a random peptide to form a fusion polypeptide.

The microorganisms from which the polynucleotide may be prepared include prokaryotic microorganisms, such as *Eubacteria* and *Archaebacteria* and lower eukaryotic microorganisms such as fungi, some algae and protozoa: Polynucleotides may be isolated from environmental samples in which case the nucleic acid may be recovered without culturing of an organism or recovered from one or more cultured organisms. Such microorganisms may be extremophiles, such as hyperthermophiles, psychrophiles, psychrotrophs, halophiles, barophiles and acidophiles. Polynucleotides encoding enzymes isolated from extremophilic microorganisms can be used. Such enzymes may function at temperatures above 100°C in terrestrial hot springs and deep sea thermal vents, at temperatures below 0°C in arctic waters, in the saturated salt environment of the Dead Sea, at pH values around 0 in coal deposits and geothermal sulfur-rich springs, or at pH values greater than 11 in sewage sludge. For example, several esterases and lipases cloned and expressed from extremophilic organisms show high activity throughout a wide range of temperatures and pHs.

Polynucleotides selected and isolated as hereinabove described are introduced into a suitable host cell. A suitable host cell is any cell which is capable of promoting recombination and/or reductive reassortment. The selected polynucleotides are already in a vector which includes appropriate control sequences. The host cell can be a higher eukaryotic cell, such as a mammalian cell, or a lower eukaryotic cell, such as a yeast cell, or the host cell can be a prokaryotic cell, such as a bacterial cell. Introduction of the construct into the host cell can be effected by calcium phosphate transfection, DEAE-Dextran mediated transfection, or electroporation (Davis *et al.,* 1986).

As representative examples of appropriate hosts, there may be mentioned: bacterial cells, such as *E. coli, Streptomyces, Salmonella typhimurium;* fungal cells, such as yeast; insect cells such as *Drosophila S2* and *Spodoptera Sf9;* animal cells such as CHO, COS or Bowes melanoma; adenoviruses; and plant cells. The selection of an appropriate host is deemed to be within the scope of those skilled in the art from the teachings herein.

With particular references to various mammalian cell culture systems that can be employed to express recombinant protein, examples of mammalian expression systems include the COS-7 lines of monkey kidney fibroblasts, described in "SV40-transformed simian cells support the replication of early SV40 mutants" (Gluzman, 1981) and other cell lines capable of expressing a compatible vector, for example, the C127, 3T3, CHO, HeLa and BHK cell lines. Mammalian expression vectors will comprise an origin of replication, a suitable promoter and enhancer and also any necessary ribosome binding sites, polyadenylation site, splice donor and acceptor sites, transcriptional termination sequences and 5' flanking nontranscribed sequences. DNA sequences derived from the SV40 splice and polyadenylation sites may be used to provide the required nontranscribed genetic elements.

Therefore, the disclosure relates to a method for producing a biologically active hybrid polypeptide and screening such a polypeptide for enhanced activity by:
1) introducing at least a first polynucleotide in operable linkage and a second polynucleotide in operable linkage, the at least first polynucleotide and second polynucleotide sharing at least one region of partial sequence homology, into a suitable host cell;
2) growing the host cell under conditions which promote sequence reorganization resulting in a hybrid polynucleotide in operable linkage;
3) expressing a hybrid polypeptide encoded by the hybrid polynucleotide;
4) screening the hybrid polypeptide under conditions which promote identification of enhanced biological activity; and
5) isolating the a polynucleotide encoding the hybrid polypeptide.

Methods for screening for various enzyme activities are known to those of skill in the art and are discussed throughout the present specification. Such methods may be employed when isolating the polypeptides and polynucleotides of the invention.

### Screening Methodologies and "On-line" Monitoring Devices

In practicing the methods of the invention, a variety of apparatus and methodologies can be used to in conjunction with the polypeptides and nucleic acids that are used in the invention, e.g., to screen polypeptides for enzyme activity, to screen compounds as potential modulators, e.g., activators or inhibitors of activity, for antibodies that bind to a polypeptide disclosed herein, for nucleic acids that hybridize to a nucleic acid disclosed herein, to screen for cells expressing a polypeptide of the invention and the like. In addition to the array formats described in detail below for screening samples, alternative formats can also be used to practice the methods of the invention. Such formats include, for example, mass spectrometers, chromatographs, e.g., high-throughput HPLC and other forms of liquid chromatography, and smaller formats, such as 1536-well plates, 384-well plates and so on. High throughput screening apparatus can be adapted and used to practice the methods of the invention, see, e.g., U.S. Patent Application No. 20020001809.

### Capillary Arrays

Nucleic acids or polypeptides that are used. in the invention can be immobilized to or applied to an array. Arrays can be used to screen for or monitor libraries of compositions (e.g., small molecules, antibodies, nucleic acids, etc.) for their ability to bind to or modulate the activity of a nucleic acid or a polypeptide of the invention. Capillary arrays, such as the GIGAMATRIX™, Diversa Corporation, San Diego, CA; and arrays described in, e.g., U.S. Patent Application No. 20020080350 A1; WO 0231203 A; WO 0244336 A, provide an alternative apparatus for holding and screening samples. The capillary array includes a plurality of capillaries formed into an array of adjacent capillaries, wherein each capillary comprises at least one wall defining a lumen for retaining a sample. The lumen may be cylindrical, square, hexagonal or any other geometric shape so long as the walls form a lumen for retention of a liquid or sample. The capillaries of the capillary array can be held together in close proximity to form a planar structure. The capillaries can be bound together, by being fused (e.g., where the capillaries are made of glass), glued, bonded, or clamped side-by-side. Additionally, the capillary array can include interstitial material disposed between adjacent capillaries in the array, thereby forming a solid planar device containing a plurality of through-holes.

A capillary array can be formed of any number of individual capillaries, for example, a range from 100 to 4,000,000 capillaries. Further, a capillary array having about 100,000 or more individual capillaries can be formed into the standard size and shape of a Microtiter® plate for fitment into standard laboratory equipment. The lumens are filled manually or automatically using either capillary action or microinjection using a thin needle. Samples of interest may subsequently be removed from individual capillaries for further analysis or characterization. For example, a thin, needle-like probe is positioned in fluid communication with a selected capillary to either add or withdraw material from the lumen.

In a single-pot screening assay, the assay components are mixed yielding a solution of interest, prior to insertion into the capillary array. The lumen is filled by capillary action when at least a portion of the array is immersed into a solution of interest. Chemical or biological reactions and/or activity in each capillary are monitored for detectable events. A detectable event is often referred to as a "hit", which can usually be distinguished from "non-hit" producing capillaries by optical detection. Thus, capillary arrays allow for massively parallel detection of "hits".

In a multi-pot screening assay, a polypeptide or nucleic acid, e.g., a ligand, can be introduced into a first component, which is introduced into at least a portion of a capillary of a capillary array. An air bubble can then be introduced into the capillary behind the first component. A second component can then be introduced into the capillary, wherein the second component is separated from the first component by the air bubble. The first and second components can then be mixed by applying hydrostatic pressure to both sides of the capillary array to collapse the bubble. The capillary array is then monitored for a detectable event resulting from reaction or non-reaction of the two components.

In a binding screening assay, a sample of interest can be introduced as a first liquid labeled with a detectable particle into a capillary of a capillary array, wherein the lumen of the capillary is coated with a binding material for binding the detectable particle to the lumen. The first liquid may then be removed from the capillary tube, wherein the bound detectable particle is maintained within the capillary, and a second liquid may be introduced into the capillary tube. The capillary is then monitored for a detectable event resulting from reaction or non-reaction of the particle with the second liquid.

### Arrays, or "Biochips"

Nucleic acids or polypeptides that are used in the invention can be immobilized to or applied to an array. Arrays can be used to screen for or monitor libraries of compositions (e.g., small molecules, antibodies, nucleic acids, etc.) for their ability to bind to or modulate the activity of a nucleic acid or a polypeptide of the invention. For example, a monitored parameter is transcript expression of a gene, e.g., a gene of the invention (a nucleic acid encoding a polypeptide of the invention). One or more, or, all the transcripts of a cell can be measured by hybridization of a sample comprising transcripts of the cell, or, nucleic acids representative of or complementary to transcripts of a cell, by hybridization to immobilized nucleic acids on an array, or "biochip." By using an "array" of nucleic acids on a microchip, some or all of the transcripts of a cell can be simultaneously quantified. Alternatively, arrays comprising genomic nucleic acid can also be used to determine the genotype of a newly engineered strain made by the methods disclosed herein. Polypeptide arrays" can also be used to simultaneously quantify a plurality of proteins. The present invention can be practiced with any known "array," also referred to as a "microarray" or "nucleic acid array" or "polypeptide array" or "antibody array" or "biochip," or variation thereof. Arrays are generically a plurality of "spots" or "target elements," each target element comprising a defined amount of one or more biological molecules, e.g., oligonucleotides, immobilized onto a defined area of a substrate surface for specific binding to a sample molecule, e.g., mRNA transcripts.

Any known array and/or method of making and using arrays can be incorporated in whole or in part, or variations thereof, as described, for example, in U.S. Patent Nos. 6,277,628; 6,277,489; 6,261,776; 6,258,606; 6,054,270; 6,048,695; 6,045,996; 6,022,963; 6,013,440; 5,965,452; 5,959,098; 5,856,174; 5,830,645; 5,770,456; 5,632,957; 5,556,752; 5,143,854; 5,807,522; 5,800,992; 5,744,305; 5,700,637; 5,556,752; 5,434,049; see also, e.g., WO 99/51773; WO 99/09217; WO 97/46313; WO 96/17958; see also, e.g., Johnston (1998) Curr. Biol. 8:R171-R174; Schummer (1997) Biotechniques 23:1087-1092; Kern (1997) Biotechniques 23:120-124; Solinas-Toldo (1997) Genes, Chromosomes & Cancer 20:399-407; Bowtell (1999) Nature Genetics Supp. 21:25-32. See also published U.S. patent applications Nos. 20010018642; 20010019827; 20010016322; 20010014449; 20010014448; 20010012537; 20010008765.

The terms "array" or "microarray" or "biochip" or "chip" as used herein is a plurality of target elements, each target element comprising a defined amount of one or more polypeptides (including antibodies) or nucleic acids immobilized onto a defined area of a substrate surface, as discussed in further detail, below.

### Antibodies and Antibody-based screening methods

The disclosure provides isolated or recombinant antibodies that specifically bind to a polypeptide of the invention. These antibodies can be used to isolate, identify or quantify a polypeptide that is used in the invention or related polypeptides. These antibodies can be used to isolate other polypeptides within the scope the invention or other related polypeptides. The antibodies can be designed to bind to an active site of a polypeptide of the invention.

The antibodies can be used in immunoprecipitation, staining, immunoaffinity columns, and the like. If desired, nucleic acid sequences encoding for specific antigens can be generated by immunization followed by isolation of polypeptide or nucleic acid, amplification or cloning and immobilization of polypeptide onto an array of the invention. Alternatively, the methods of the invention can be used to modify the structure of an antibody produced by a cell to be modified, e.g., an antibody's affinity can be increased or decreased. Furthermore, the ability to make or modify antibodies can be a phenotype engineered into a cell by the methods of the invention.

Methods of immunization, producing and isolating antibodies (polyclonal and monoclonal) are known to those of skill in the art and described in the scientific and patent literature, see, e.g., Coligan, CURRENT PROTOCOLS IN IMMUNOLOGY, Wiley/Greene, NY (1991); Stites (eds.) BASIC AND CLINICAL IMMUNOLOGY (7th ed.) Lange Medical Publications, Los Altos, CA ("Stites"); Goding, MONOCLONAL ANTIBODIES: PRINCIPLES AND PRACTICE (2d ed.) Academic Press, New York, NY (1986); Kohler (1975) Nature 256:495; Harlow (1988) ANTIBODIES, A LABORATORY MANUAL, Cold Spring Harbor Publications, New York. Antibodies also can be generated *in vitro,* e.g., using recombinant antibody binding site expressing phage display libraries, in addition to the traditional in vivo methods using animals. See, e.g., Hoogenboom (1997) Trends Biotechnol. 15:62-70; Katz (1997) Annu. Rev. Biophys. Biomol. Struct. 26:27-45.

### Kits

The disclosure provides kits comprising the compositions, e.g., nucleic acids, expression cassettes, vectors, cells, transgenic seeds or plants or plant parts, polypeptides (e.g., enzymes involved in chlorophyll catabolism or having an esterase (e.g., chlorophyllase) activity) and/or antibodies of the disclosure. The kits also can contain instructional material teaching the methodologies and industrial uses of the invention, as described herein. The kits are designed to accommodate industrial scale levels of processing, e.g., of foods, feeds, oils and the like.

### Whole cell engineering and measuring metabolic parameters

The methods disclosed herein provide whole cell evolution, or whole cell engineering, of a cell to develop a new cell strain having a new phenotype, e.g., a modified chlorophyll catabolism pathway, or, a new or modified enzyme (e.g., chlorophyllase) activity, by modifying the genetic composition of the cell. The genetic composition can be modified by addition to the cell of a nucleic acid of the invention, e.g., a coding sequence for an enzyme disclosed herein. See, e.g., WO0229032; WO0196551.

To detect the new phenotype, at least one metabolic parameter of a modified cell is monitored in the cell in a "real time" or "on-line" time frame. A plurality of cells, such as a cell culture, is monitored in "real time" or "on-line." A plurality of metabolic parameters is monitored in "real time" or "on-line." Metabolic parameters can be monitored using an enzyme disclosed herein.

Metabolic flux analysis (MFA) is based on a known biochemistry framework. A linearly independent metabolic matrix is constructed based on the law of mass conservation and on the pseudo-steady state hypothesis (PSSH) on the intracellular metabolites. In practicing the methods disclosed herein, metabolic networks are established, including the:
- identity of all pathway substrates, products and intermediary metabolites
- identity of all the chemical reactions interconverting the pathway metabolites, the stoichiometry of the pathway reactions,
- identity of all the enzymes catalyzing the reactions, the enzyme reaction kinetics,
- the regulatory interactions between pathway components, e.g. allosteric interactions, enzyme-enzyme interactions etc,
- intracellular compartmentalization of enzymes or any other supramolecular organization of the enzymes, and,
- the presence of any concentration gradients of metabolites, enzymes or effector molecules or diffusion barriers to their movement.

Once the metabolic network for a given strain is built, mathematic presentation by matrix notion can be introduced to estimate the intracellular metabolic fluxes if the on-line metabolome data is available. Metabolic phenotype relies on the changes of the whole metabolic network within a cell. Metabolic phenotype relies on the change of pathway utilization with respect to environmental conditions, genetic regulation, developmental state and the genotype, etc. The methods disclosed herein, after the on-line MFA calculation, the dynamic behavior of the cells, their phenotype and other properties are analyzed by investigating the pathway utilization. For example, if the glucose supply is increased and the oxygen decreased during the yeast fermentation, the utilization of respiratory pathways will be reduced and/or stopped, and the utilization of the fermentative pathways will dominate. Control of physiological state of cell cultures will become possible after the pathway analysis. The methods disclosed herein can help determine how to manipulate the fermentation by determining how to change the substrate supply, temperature, use of inducers, etc. to control the physiological state of cells to move along desirable direction. In practicing the methods disclosed herein, the MFA results can also be compared with transcriptome and proteome data to design experiments and protocols for metabolic engineering or gene shuffling, etc.

In practicing the methods disclosed herein, any modified or new phenotype can be conferred and detected, including new or improved characteristics in the cell. Any aspect of metabolism or growth can be monitored.

### Enzymes

The disclosure provides novel compositions and methods for enzymatically treating, e.g., decoloring or "bleaching," algal, animal (e.g., fish) and/or plant preparations, feeds, foods or oils comprising chlorophyll (the chlorophyll can be in the preparations, feeds, foods or oils naturally, as a contaminant, as an undesired composition in a processed product, etc). In one aspect, chlorophyll-containing or chlorophyll-contaminated compositions, e.g., algal, animal or plant preparations, feeds, foods or oils are enzymatically treated using a chlorophyllase or equivalent enzyme. Any polypeptide having an activity that can modify a chlorophyll or chlorophyll metabolite, can be used in such a composition or method.

### Chlorophyllases

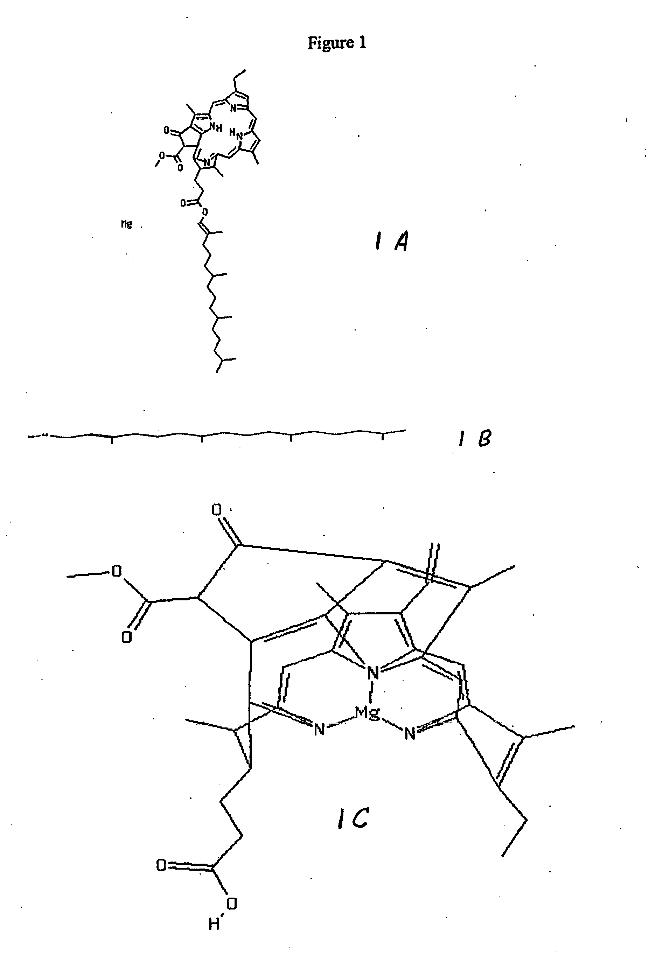

The polypeptides and/or peptides can have esterase activity, e.g., a chlorophyllase or a similar activity. The polypeptides and/or peptides can include catalytic antibodies, enzymes, active sites, and the like. These polypeptides and/or peptides having esterase (e.g., chlorophyllase) activity can be used in compositions or methods disclosed herein. For example, in one aspect, compositions and methods enzymatically treat chlorophyll-containing or chlorophyll-contaminated compositions by hydrolyzing chlorophyll (Figure 1A) to phytol (Figure 1B) and chlorophyllide (Figure 1C).

Any chlorophyllase, chlase or chlorophyll chlorophyllido-hydrolyase or polypeptide having a similar activity (e.g., chlorophyll-chlorophyllido hydrolase 1 or chlase 1, or, chlorophyll-chlorophyllido hydrolase 2 or chlase 2, see, e.g., NCBl P59677_1 and P59678, respectively) can be used in a composition or method disclosed herein. Any polypeptide (e.g., enzyme or catalytic antibody) that catalyses the hydrolysis of a chlorophyll ester bond to yield chlorophyllide and phytol can be used in a composition or method disclosed herein. Any isolated, recombinant or synthetic or chimeric (a combination of synthetic and recombinant) polypeptide (e.g., enzyme or catalytic antibody) can be used, e.g., a chlorophyllase, chlase or chlorophyll chlorophyllido-hydrolyase or polypeptide having a similar activity can be used in a composition or method disclosed herein see, e.g., Marchler-Bauer (2003) Nucleic Acids Res. 31: 383-387.

The compositions and methods disclosed herein can be practiced with enzymes as described in WO 0229022. For example, , the compositions and methods can comprise recombinant expression of enzymes, e.g., chlorophyllases, such as chlorophyllase-encoding polynucleotides. Recombinant nucleic acid is expressed in whole cells, cell extracts or *in vitro.* The enzyme-encoding polynucleotide is modified to result in production of altered levels of enzyme (e.g., chlorophyllase) in a transformed host cell.

The compositions and methods disclosed herein can be practiced with known enzymes, such as chlorophyllases (including chlases and chlorophyll chlorophyllido-hydrolyases) and related polypeptides are well known in the art. For example, the *Arabidopsis thaliana* chlorophyllase can be used as described, e.g., in NCBI entry NM_123753 (where the enzyme having a sequence as set forth in SEQ ID NO:22 is encoded, e.g., by SEQ ID NO:21):
SEQ ID NO:21
SEQ ID NO:22

The *Ginkgo biloba* chlorophyllase can be used as described, e.g., in NCBI entry AY292526:
SEQ ID NO:23
SEQ ID NO:24

The Brassica *oleracea* chlorophyllase can be used as described, e.g., in NCBI entry AF337546:
SEQ ID NO:25
SEQ ID NO:26

The *Citrus sinensis* chlorophyllase can be used as described, e.g., in NCBI entry Q9MV14:
SEQ lD NO:27

### Enzyme preparations

Enzymes used in the methods of the invention can be formulated or modified, e.g., chemically modified, e.g., to enhance oil solubility, stability, activity or for immobilization. For example, enzymes used in the methods of the invention can be formulated to be amphipathic or more lipophilic. For example, enzymes used in the methods of the invention can be encapsulated, e.g., in liposomes or gels, e.g., alginate hydrogels or alginate beads or equivalents. Enzymes used in the methods of the invention can be formulated in micellar systems, e.g., a ternary micellar (TMS) or reverse micellar system (RMS) medium. Enzymes used in the methods of the invention can be formulated as described in Yi (2002) J. of Molecular Catalysis B: Enzymatic, Vol. 19, No. 0, pgs 319-325. For example, amphipathic enzyme, e.g., chlorophyllase, in the form of a ternary micellar (TMS) or reverse micellar system (RMS) medium can be encapsulated in alginate hydrogels. An enzyme, e.g., a chlorophyllase, is prepared in aqueous buffer and retained in a hydrogel, e.g., TMS/alginate and RMS/alginate. One approach to encapsulating enzyme, e.g., chlorophyllase, can be emulsification and/or internal gelation of the enzyme-TMS or -RMS system.

The enzymatic reactions of the methods of the invention can be done *in vitro,* including, e.g. capillary arrays, as discussed below, or, in whole cell systems. Enzyme reactions of the methods of the invention are done in one reaction vessel or multiple vessels. For instance, the enzymatic reactions of the methods of the invention are done in a vegetable oil refining apparatus.

The compositions and methods of the invention can be practiced with immobilized enzymes, e.g., immobilized chlorophyllase. The enzyme can be immobilized on any organic or inorganic support. Exemplary inorganic supports include alumina, celite, Dowex-1-chloride, glass beads and silica gel. Exemplary organic supports include alginate hydrogels or alginate beads or equivalents.

In various aspects of the invention, immobilization of chlorophyllase can be optimized by physical adsorption on various inorganic supports, including alumina, celite, Dowex-1-chloride, glass beads and silica gel. Enzymes used to practice the invention can be immobilized in different media, including water, Tris-HCI buffer solution and a ternary micellar system containing Tris-HCI buffer solution, hexane and surfactant. The highest immobilization efficiency (84.56%) and specific activity (0.34 mumol hydrolyzed chlorophyll mg protein-1 per min) were obtained when chlorophyllase was suspended in Tris-HCI buffer solution and adsorbed onto silica gel.

### Industrial and Medical Applications

The polypeptides, e.g., enzymes of the invention involved in chlorophyll catabolism or having an esterase (e.g., chlorophyllase) activity, can be used in a variety of medical and industrial applications, as described herein. The compositions and methods of the invention can be used in conjunction with any industrial use or pharmaceutical or medical application for the treatment of chlorophyll-containing materials, e.g., vegetable preparations, oil-comprising materials. For example, the compositions and methods of the invention can be used with processes for converting a non-hydratable phospholipid to a hydratable form, oil degumming, processing of oils from plants, fish, algae and the like, to name just a few applications. For example, the methods of the invention can be used with the processing of fats and oils as described, e.g., in JP Patent Application Publication H6-306386, describing converting phospholipids present in the oils and fats into water-soluble substances containing phosphoric acid groups.

The compositions and methods of the invention can be used in conjunction with methods for processing plant oils, such as those derived from or isolated from rice bran, soy, canola, palm, cottonseed, corn, palm kernel, coconut, peanut, sesame, sunflower. The compositions and methods of the invention can be used in conjunction with methods for processing essential oils, e.g., those from fruit seed oils, e.g., grapeseed, apricot, borage, etc. The compositions and methods of the invention can be used in conjunction with methods for processing oils and phospholipids in different forms, including crude forms, degummed, gums, wash water, clay, silica, soapstock, and the like. The compositions and methods of the invention can be used in conjunction with methods for processing high phosphorous oils (e.g., a soy bean oil), fish oils, animal oils, plant oils, algae oils and the like.

The compositions and methods of the invention can be used in conjunction with methods for processing and making edible oils, biodiesel oils, liposomes for pharmaceuticals and cosmetics, structured phospholipids and structured lipids. The compositions and methods of the invention can be used in conjunction with methods for oil extraction. The compositions and methods of the invention can be used in conjunction with methods for making various soaps.

The methods can further comprise modifying pH (e.g., increasing pH) to promote aqueous separation of chlorophyllide. Thus, the compositions and methods of the invention can also comprise a caustic neutralization processes, e.g., with caustic-neutralized pH conditions. The compositions and methods of the invention comprise a neutralization step, e.g., in treating "chemically refined oils", e.g., using chlorophyllases and/or in the separation chlorophyllide. The compositions and methods of the invention can comprise modifying pH to promote aqueous separation of chlorophyllide.

The compositions and methods of the invention comprise use of adsorbent-free or reduced adsorbent silica refining devices and processes, which are known in the art, e.g., using TriSyl Silica Refining Processes (Grace Davison, Columbia, MD), or, SORBSIL R™ silicas (INEOS Silicas, Joliet, IL).

### Enzymatic treatment, or "bleaching" or decoloring processes

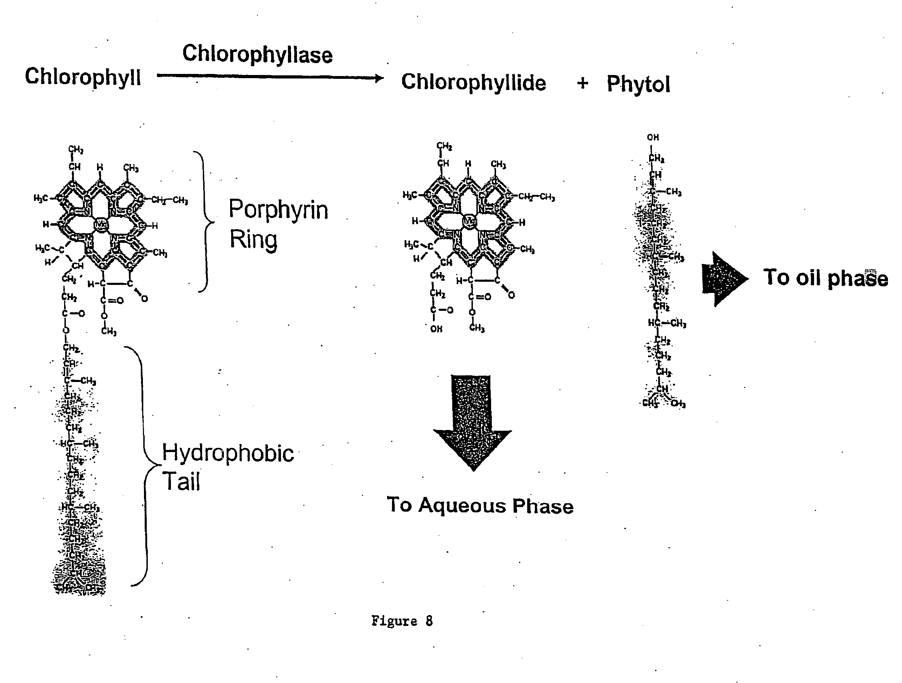

The invention provides novel compositions and methods for enzymatically treating, e.g., decoloring or "bleaching," algal, animal (e.g., fish) and/or plant preparations, feeds, foods or oils, as illustrated in Figures 8 to 16. In one aspect, chlorophyll-containing or chlorophyll-contaminated foods or oils are treated. For example, vegetable oils, including oils processed from oilseeds, such as canola (rapeseed) oil or soybean oil, or oil fruits, such as palm oil, are processes using the compositions and/or methods of the invention.

At least one step in this exemplary method involves use of an enzyme, e.g., a chlorophyllase enzyme that can hydrolyze chlorophyll to phytol and chlorophyllide. Alternatively, one, several or all steps use an enzyme. The reaction can be *in vitro* or *in vivo.*

Figure 8 illustrates the reaction of an exemplary esterase of the invention in chlorophyll degradation - the chlorophyllase (chlase) catalyzes hydrolysis of an ester bond in chlorophyll to yield chlorophyllide and phytol, where the chlorophyllide enters the aqueous phase due to a hydrophilic porphyrin ring, and the phytol separates into an oil (hydrophobic) phase. In a process of the invention the hydrophilic porphyrin ring is separated with gum/water fraction using any one of the many well-known methods.

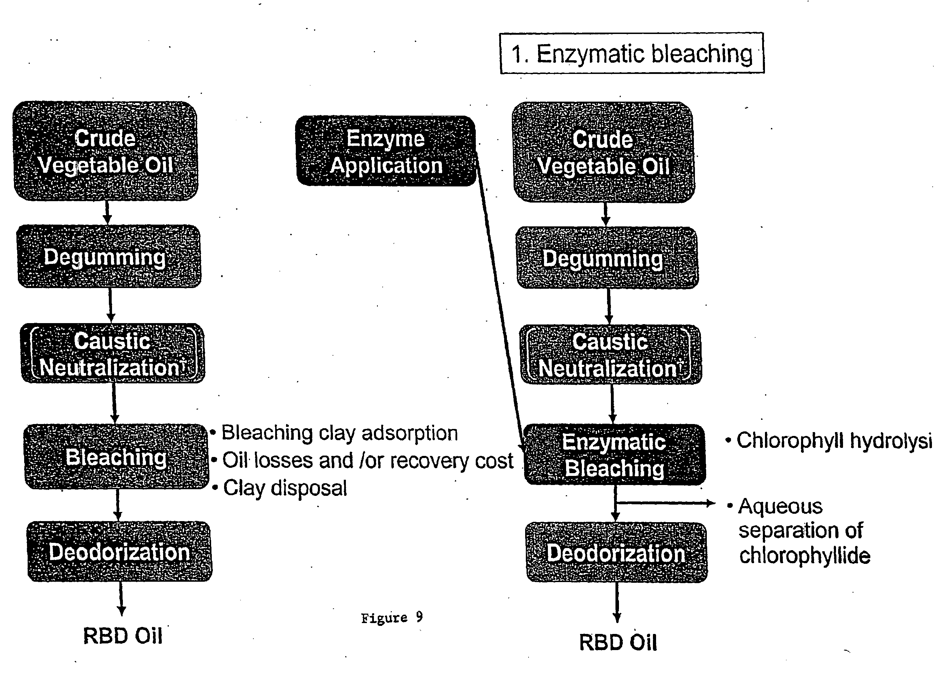

Figure 9 illustrates and compares traditional versus an exemplary enzymatic decoloring (bleaching) process of the invention, where the enzymatic bleaching process can incorporate an esterase of the invention. In the traditional method crude vegetable oil is degummed, (optionally, caustic neutralized), bleached using, e.g., clay adsorption with subsequent clay disposal, and deodorization to produce "refined, bleached and deodorized" or RBD oil. In this exemplary enzymatic bleaching process of the invention, the crude vegetable oil is degummed, (optionally, caustic neutralized), bleached using, e.g., a polypeptide of the invention, such as a chlorophyllase of the invention, with subsequent aqueous separation of the chlorophyllide, followed by deodorization to produce a "refined, bleached and deodorized" or RBD oil. The need for the degumming depends on phosphorus content and other factors (all known in the art). Soy and canola are typically degummed.

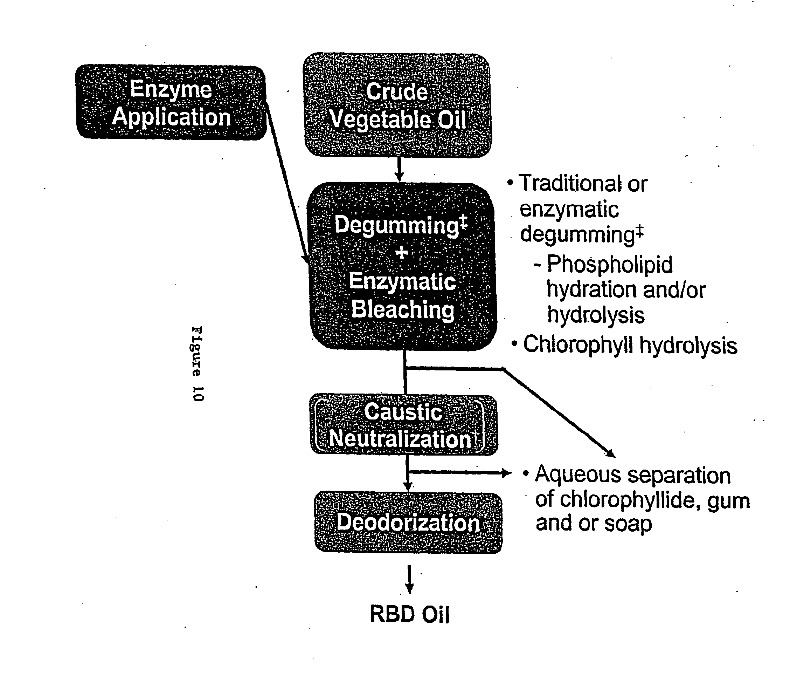

Figure 10 illustrates an exemplary enzymatic decoloring (bleaching) process of the invention - a combined degumming-bleaching ("decoloring") process. In this exemplary enzymatic bleaching process of the invention, the crude vegetable oil is degummed and enzymatically bleached using a polypeptide of the invention, such as an esterase, e.g., a chlorophyllase, of the invention in one step, or "one pot." The degumming can be a "traditional" or an enzymatic degumming, e.g., involving phospholipid(s) and/or hydrolysis. The exemplary process of the invention comprises a subsequent aqueous separation step to remove the reaction product chlorophyllide, gum and/or soap. This is followed by deodorization to produce a "refined, bleached and deodorized" or RBD oil.

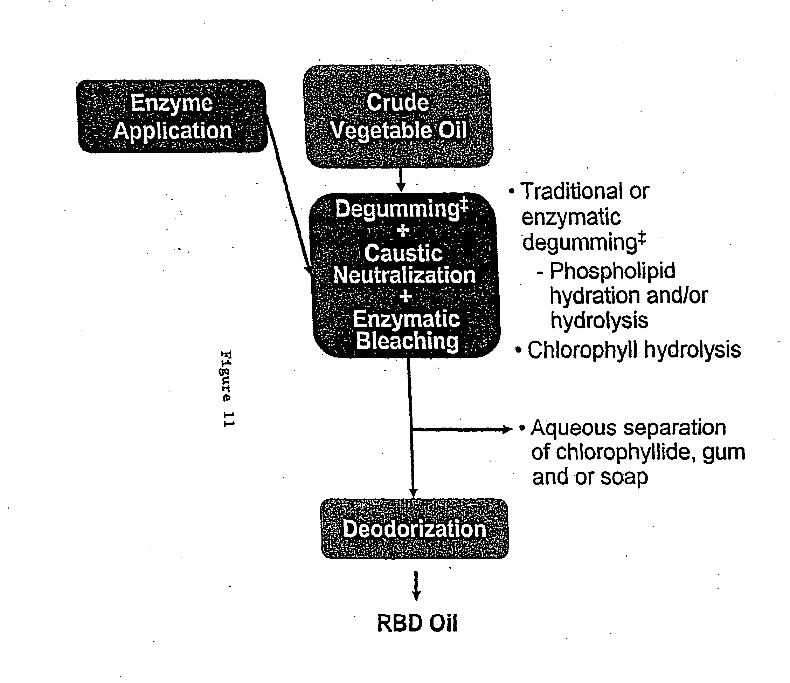

Figure 11 illustrates an exemplary enzymatic decoloring (bleaching) process of the invention that combines degumming, enzymatic bleaching ("decoloring") and caustic neutralization steps. In this exemplary enzymatic bleaching process of the invention, the crude vegetable oil is degummed, neutralized and enzymatically bleached using a polypeptide of the invention, such as an esterase, e.g., a chlorophyllase, of the invention in one step, or "one pot." The degumming can be a "traditional" or an enzymatic degumming, e.g., involving phospholipid(s) and/or hydrolysis. The exemplary process of the invention comprises a subsequent aqueous separation step to remove the reaction product chlorophyllide, gum and/or soap.

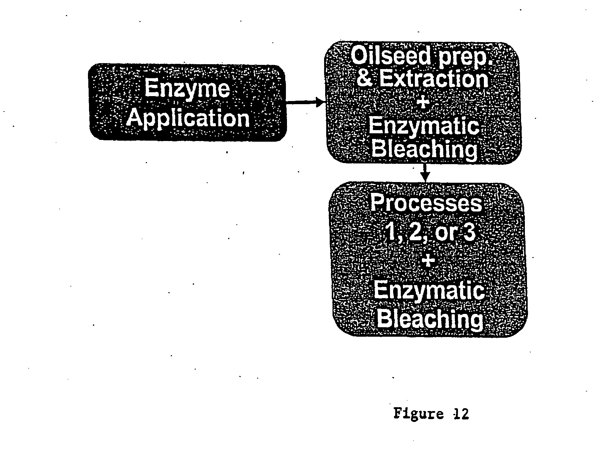

Figure 12 illustrates an exemplary enzymatic decoloring (bleaching) process of the invention that comprises application of a polypeptide of the invention, such as an esterase, e.g., a chlorophyllase to an oilseed preparation, followed by a subsequent aqueous separation step (to remove, e.g., the reaction product chlorophyllide, or gums and/or soaps), followed by the processes illustrated in Figures 9, 10, or 11.

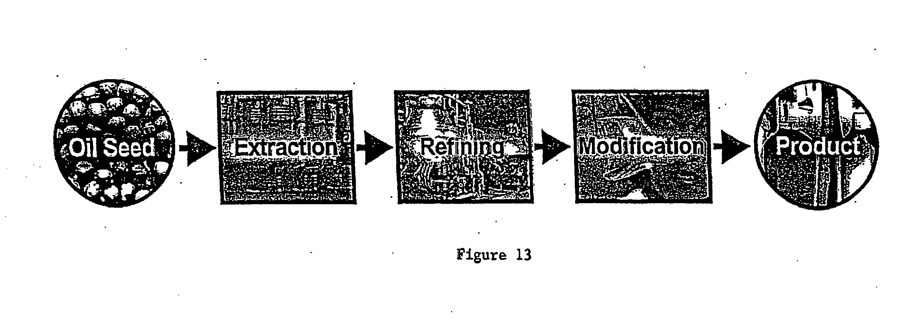

Figure 13 illustrates a general oilseed refining scheme comprising extraction, refining and modification of an oilseed, where in addition to a polypeptide of the invention, such as an esterase, e.g., a chlorophyllase to an oilseed in one or several or all of these steps, other polypeptides and/or chemicals are also added, e.g., cellulase, hemicellulase, protease, pectinase, phospholipase A, B, C and/or D, esterase (e.g., a selective esterase), a lipase (e.g., 1,3 lipase), a selective lipase, a known chlorophyllase or other enzyme involved in chlorophyll catabolism, and the like.

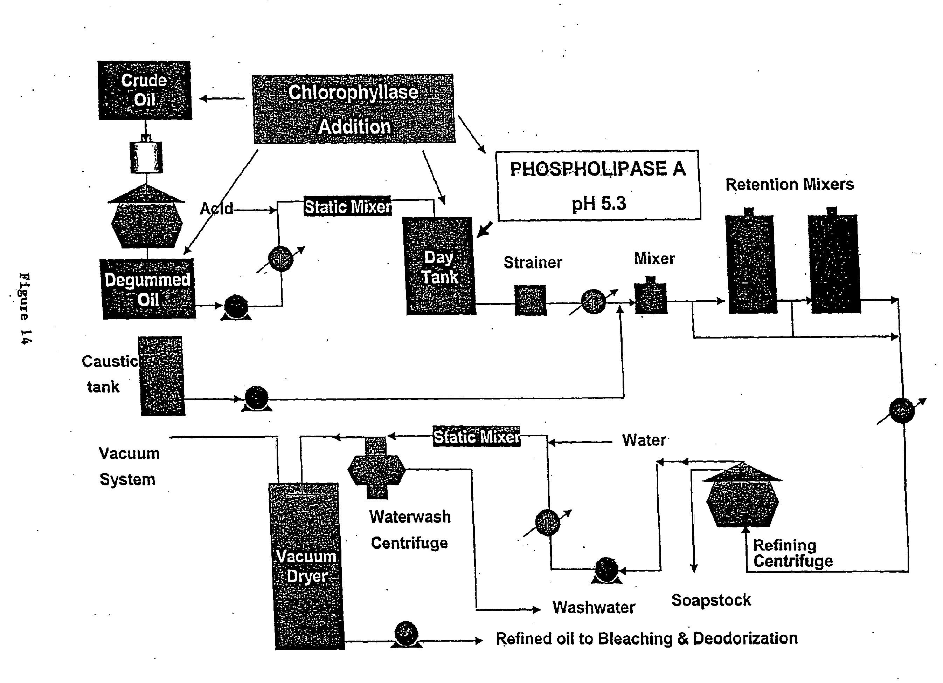

Figure 14 illustrates an exemplary industrial process of the invention - a biodegumming process, comprising use of a phospholipase A and at least one polypeptide of the invention having chlorophyllase enzyme activity. The at least one polypeptide of the invention having chlorophyllase activity can be added to one or several or all of the following steps: added to the crude oil, in the degumming process or in the degummed oil, a storage or holding tank, with the phospholipase A (e.g., in "the day tank" of the figure) and/or the caustic tank.

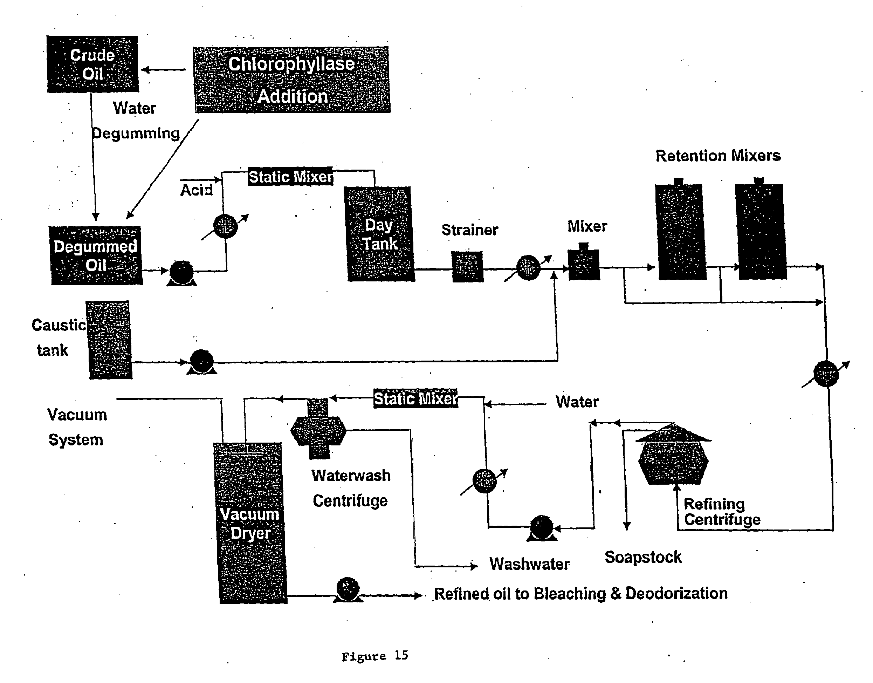

Figure 15 illustrates another exemplary industrial process of the invention comprising use of at least one polypeptide of the invention having chlorophyllase enzyme activity. The at least one polypeptide of the invention having chlorophyllase activity can be added to one or several or all of the following steps: added to the crude oil, in the degumming process or in the degummed oil, a storage or holding tank, a caustic tank and/or a retention mixer.

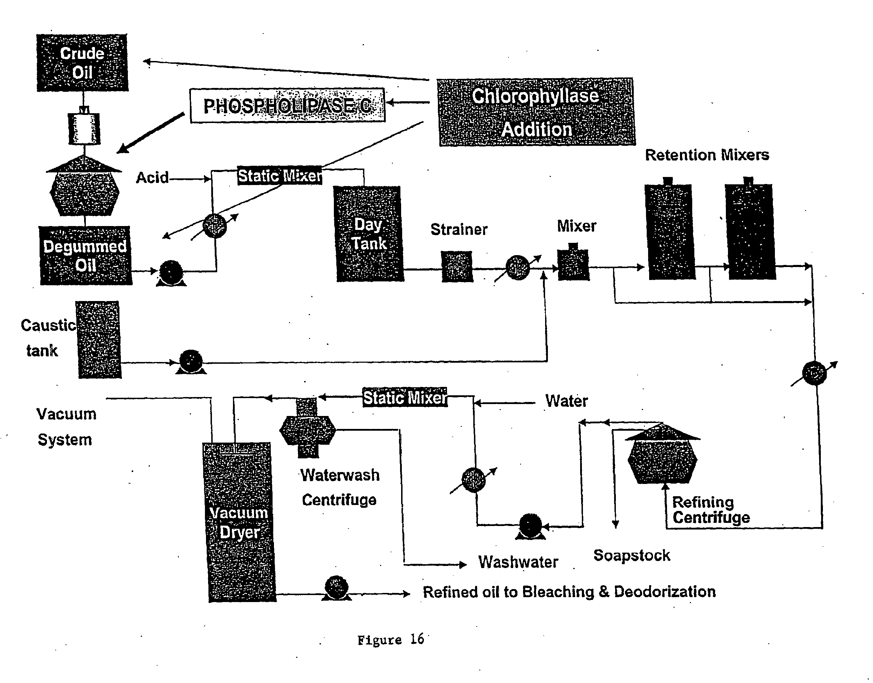

Figure 16 illustrates another exemplary industrial process of the invention comprising use of at least one polypeptide of the invention having chlorophyllase enzyme activity. In this exemplary process, phospholipase C (PLC) is added into the degumming process or in the degummed oil with the chlorophyllase enzyme of the invention. The at least one polypeptide of the invention having chlorophyllase activity can be added to one or several or all of the following steps: added to the crude oil, in the degumming process or in the degummed oil (with a PLC), a storage or holding tank, a caustic tank and/or a retention mixer.

### Oil degumming and Vegetable oil processing

The compositions and methods of the invention can be used in various vegetable oil processing steps, such as in vegetable oil extraction, particularly, in the removal of "phospholipid gums" in a process called "oil degumming,".

The compositions and methods of the invention can be used in methods for processing vegetable oils from various sources, such as rice bran, soybeans, rapeseed, peanuts and other nuts, sesame, sunflower, palm and corn. The methods can used in conjunction with processes based on extraction with as hexane, with subsequent refining of the crude extracts to edible oils. The first step in the refining sequence is the so-called "degumming" process, which serves to separate phosphatides by the addition of water. The material precipitated by degumming is separated and further processed to mixtures of lecithins. The commercial lecithins, such as soybean lecithin and sunflower lecithin, are semi-solid or very viscous materials. They consist of a mixture of polar lipids, mainly phospholipids, and oil, mainly triglycerides. The compositions and methods of the invention can be used before or after any step in a process, or before or after any combination of steps, or before or after all of the steps, in a process, e.g., prior to, during or following mechanical and/or chemical extraction, degumming and/or bleaching and the like.

The compositions and methods of the invention can be used in (i.e., in conjunction with) any "degumming" procedure, including water degumming, ALCON oil degumming (e.g., for soybeans), safinco degumming, "super degumming," UF degumming, TOP degumming, uni-degumming, dry degumming and ENZYMAX™ degumming. See, e.g., U.S. Patent Nos. 6,355,693; 6,162,623; 6,103,505; 6,001,640; 5,558,781; 5,264,367. Compositions and methods of the invention can be used in any oil processing method, e.g., degumming or equivalent processes. For example, compositions and methods of the invention can be used in processes as described in U.S. Patent Nos. 5,558,781; 5,288,619; 5,264,367; 6,001,640; 6,376,689; WO 0229022; oil degumming as described, e.g., in WO 98/18912; processes as described in JP Application No.: H5-132283 (filed April 25, 1993); EP Application number: 82870032.8, and the like. Various "degumming" procedures incorporated by the methods of the invention are described in Bockisch, M. (1998) In Fats and Oils Handbook, The extraction of Vegetable Oils (Chapter 5), 345-445, AOCS Press, Champaign, Illinois. The compositions and methods of the invention can be used in the industrial application of enzymatic degumming of triglyceride oils as described, e.g., in EP 513 709.

Compositions and methods of the invention are used to treat Vegetable oils, e.g., crude oils, such as rice bran, soy, canola, flower and the like. This improves the efficiency of the degumming process. The methods of the invention result in the improved separation of chlorophyll from the oil phase, e.g., during centrifugation. The improved separation of these phases can result in more efficient removal of chlorophylls from the oil, including both hydratable and nonhydratable oils.

The compositions and methods of the invention can be used in the industrial application of enzymatic degumming as described, e.g., in CA 1102795, which describes a method of isolating polar lipids from cereal lipids by the addition of at least 50% by weight of water. This method is a modified degumming in the sense that it utilizes the principle of adding water to a crude oil mixture.

The disclosure provides enzymatic processes comprising use of compositions and methods of the invention comprising hydrolysis of hydrated phospholipids in oil at a temperature of about 20°C to 40°C, at an alkaline pH, e.g., a pH of about pH 8 to pH 10, using a reaction time of about 3 to 10 minutes.

In various exemplary processes of the invention, a number of distinct steps comprise the degumming process preceding the core bleaching and deodorization refining processes. These steps include heating, mixing, holding, separating and drying. Following the heating step, water and often acid are added and mixed to allow the insoluble phospholipid "gum" to agglomerate into particles which may be separated. While water separates many of the phosphatides in degumming, portions of the phospholipids are non-hydratable phosphatides (NHPs) present as calcium or magnesium salts. Degumming processes address these NHPs by the addition of acid. Following the hydration of phospholipids, the oil is mixed, held and separated by centrifugation. Finally, the oil is dried and stored, shipped or refined. The resulting gums are either processed further for lecithin products or added back into the meal. As noted above, the compositions and methods of the invention can be used before or after any of these steps, or before or after any combination of steps, or before or after all of the steps, in any processing method.

Upon completion of an enzyme treatment of the invention, the treated liquid (e.g., oil) is separated with an appropriate means such as a centrifugal separator and the processed oil is obtained. Compounds produced by enzyme modification of chlorophyll are partially or completely transferred into the aqueous phase and removed from the oil phase. Upon completion of the enzyme treatment, if necessary, the processed oil can be additionally washed with water or organic or inorganic acid such as, e.g., acetic acid, phosphoric acid, succinic acid, and the like, or with salt solutions.

In one exemplary process for ultra-filtration degumming, an enzyme used in a method of the invention is bound to a filter or the enzyme is added to an oil prior to filtration. Enzymes used in compositions or methods of the invention can be immobilized to any substrate, e.g., filters, fibers, columns, beads, colloids, gels, hydrogels, meshes and the like.

Compositions and methods of the invention can be used to improve oil extraction, oil degumming and caustic neutralization (e.g., vegetable oils). A composition or method of the invention and at least one plant cell wall degrader (e.g., a cellulase, a hemicellulase or the like, to soften walls and increase yield at extraction) is used in a process of the invention. In an exemplary method, to improve oil extraction and oil degumming, a phospholipase, e.g., a phospholipase C, or another hydrolase (e.g., a cellulase, a hemicellulase, an esterase, a protease and/or a phosphatase) is used. For example, during a crushing step associated with oil production (including but not limited to soybean, canola, sunflower, rice bran oil) a phospholipase or other enzyme can be used. By using enzymes prior to or in place of solvent extraction, it is possible to increase oil yield and reduce the amount of hydratable and non-hydratable phospholipids in the crude oil. The overall reduction of phospholipids in the crude oil will result in improved yields during refining with the potential for eliminating the requirement for a separate degumming step prior to bleaching and deodorization.

Compositions and methods of the invention also can be practiced using processes as described in U.S. Patent No. 5,414,100. For example, the methods or compositions further comprise chromatographic processes for deacidification of vegetable oils at ambient temperature. These processes can be retrofitted into deacidification operations using miscella refining or solvent extraction, crude vegetable oil is dissolved in a solvent such as isopropyl alcohol and passed through a column of activated alumina (aluminum oxide) at room temperature. The process, which eliminates physical contact between both oil and an alkaline reagent and oil and water, simplifies subsequent bleaching processes by also removing some color pigments. The spent alumina can be reactivated by washing it with a dilute solution of sodium hydroxide or potassium hydroxide.

Compositions and methods of the invention also can be practiced using processes as described in JP57156482, 1982 (application no. JP19810040794 19810320), describing refining vegetable fats or oils as by-products.

Compositions and methods of the invention also can be practiced using processes as described in U.S. Patent No. 5,315,021. For example, the methods or compositions of the invention can be practiced with processes for removing chlorophyll color impurities from vegetable oils. The processes can comprise dispersing a source of phosphoric acid in vegetable oil to form a mixture having a moisture content of less than 0.1% by weight which mixture is maintained at a temperature in the range of 70°C to 160°C until a precipitate containing chlorophyll color impurities is formed. This can be followed separating the precipitated material from the oil to remove the chlorophyll color impurities with the precipitated material, e.g., during conventional oil processing up to and including the removal of bleaching clay from the oil.

### Enzymatic processing of oilseeds

The compositions and methods of the invention can be used for enzymatic processing of oilseeds, including soybean, canola (rapeseed), coconut, avocado and olive paste. These processes of the invention can increase the oil yield and to improve the nutritional quality of the obtained meals. enzymatic processing of oilseeds using the enzymes and methods of the invention will provide economical and environmental benefits, as well as alternative technologies for oil extraction and processing food for human and animal consumption. Alternatively, the processes of the invention further comprise use of phospholipases, proteases, phosphatases, phytases, xylanases, amylases (e.g., □-amylases), glucanases (e.g., □-glucanases), polygalacturonases, galactolipases, cellulases, hemicellulases, pectinases and other plant cell wall degrading enzymes, as well as mixed enzyme preparations and cell lysates. Alternatively, the processes of the invention can be practiced in conjunction with other processes, e.g., enzymatic treatments, e.g., with carbohydrases, including cellulase, hemicellulase and other side degrading activities, or, chemical processes, e.g., hexane extraction of soybean oil. The enzymatic treatment can increase the oil extractability by 8-10% when the enzymatic treatment is carried out prior to the solvent extraction.

The processes of the invention can be practiced with aqueous extraction processes. The aqueous extraction methods can be environmentally cleaner alternative technologies for oil extraction. The processes of the invention can also use enzymes that hydrolyze the structural polysaccharides forming the cell wall of oilseeds, or that hydrolyze the proteins which form the cell and lipid body membranes, e.g., utilizing digestions comprising cellulase, hemicellulase, and/or protopectinase for extraction of oil from soybean cells. Methods are practiced with an enzyme of the invention as described by Kasai (2003) J. Agric. Food Chem. 51:6217-6222, who reported that the most effective enzyme to digest the cell wall was cellulase.

Proteases are used in combination with the methods of the invention. The combined effect of operational variables and enzyme activity of protease and cellulase on oil and protein extraction yields combined with other process parameters, such as enzyme concentration, time of hydrolysis, particle size and solid-to-liquid ratio has been evaluated. Methods disclosed herein are practiced with protocols as described by Rosenthal (2001) Enzyme and Microb. Tech. 28:499-509, who reported that use of protease can result in significantly higher yields of oil and protein over the control when heat treated flour is used.

Complete protein, pectin, and hemicellulose extraction are used in combination with the methods of the invention. The plant cell consists of a series of polysaccharides often associated with or replaced by proteins or phenolic compounds. Most of these carbohydrates are only partially digested or poorly utilized by the digestive enzymes. The disruption of these structures through processing or degrading enzymes can improve their nutrient availability. Methods disclosed herein are practiced with protocols as described by Ouhida (2002) J. Agric. Food Chem. 50:1933-1938, who reported that a significant degradation of the soybean cell wall cellulose (up to 20%) has been achieved after complete protein, pectin, and hemicellulose extraction.

The methods disclosed herein further comprise incorporation of various enzymatic treatments in the treatment of seeds, e.g., canola seeds, these treatments comprising use of proteases, cellulases, and hemicellulases (in various combinations with each other and with one or more enzymes of the invention). For example, the methods can comprise enzymatic treatments of canola seeds at 20 to 40 moisture during the incubation with enzymes prior to a conventional process; as described, e.g., by Sosulski (1990) Proc. Can. lnst. Food Sci. Technol. 3:656. The methods of the invention can further comprise incorporation of proteases, □-amylases, polygalacturonases (in various combinations with each other and with one or more enzymes of the invention) to hydrolyze cellular material in coconut meal and release the coconut oil, which can be recovered by centrifugation, as described, e.g., by McGlone (1986) J. of Food Sci. 51:695-697. The methods of the invention can further comprise incorporation of pectinases, □-amylases, proteases, cellulases in different combinations (with each other and with one or more enzymes of the invention) to result in significant yield improvement (~70% in the best case) during enzymatic extraction of avocado oil, as described, e.g., by Buenrostro (1986) Biotech. Letters 8(7):505-506. In processes of the invention for olive oil extraction, olive paste is treated with cellulase, hemicellulase, poligalacturonase, pectin-methyltransferase, protease and their combinations (with each other and with one or more enzymes of the invention), as described, e.g., by Montedoro (1976) Acta Vitamin. Enzymol. (Milano) 30:13.

The methods of the invention further comprise incorporation of various enzymatic treatments in the treatment of seeds, e.g., canola seeds, these treatments comprising use of proteases, cellulases, and hemicellulases (in various combinations with each other and with one or more enzymes of the invention). For example, the methods can comprise enzymatic treatments of canola seeds at 20 to 40 moisture during the incubation with enzymes prior to a conventional process; as described, e.g., by Sosulski (1990) Proc. Can. lnst. Food Sci. Technol. 3:656. The methods of the invention can further comprise incorporation of proteases, □-amylases, polygalacturonases (in various combinations with each other and with one or more enzymes of the invention) to hydrolyze cellular material in coconut meal and release the coconut oil, which can be recovered by centrifugation, as described, e.g., by McGlone (1986) J. of Food Sci. 51:695-697. The methods of the invention can further comprise incorporation of pectinases, □-amylases, proteases, cellulases in different combinations (with each other and with one or more enzymes of the invention) to result in significant yield improvement (-70% in the best case) during enzymatic extraction of avocado oil, as described, e.g., by Buenrostro (1986) Biotech. Letters 8(7):505-506. In processes of the invention for olive oil extraction, olive paste is treated with cellulase, hemicellulase, poligalacturonase, pectin-methyltransferase, protease and their combinations (with each other and with one or more enzymes of the invention), as described, e.g., by Montedoro (1976) Acta Vitamin. Enzymol. (Milano) 30:13.

The compositions and methods of the invention can be practiced with methods as described in U.S. Patent No. 6,376,689. For example, the compositions and methods of the invention can comprise a single-step acid degumming/decolorizing process that removes chlorophyll-type compounds from vegetable oils from seeds, especially frost damaged seeds which have large amounts of chlorophyll-type compounds. The methods disclosed herein further comprise a mixture of aqueous sulfuric and phosphoric acids that is blended with the oil to remove chlorophyll-type compounds from the oil. The purified oil can have less than about 5 ppm chlorophyll-type compounds, less than about 50 ppm phosphorus or less than about 1.0 weight percent free fatty acids.

### Purification of phytosterols from vegetable oils

The compositions (e.g., esterases) and methods of the invention can also be used In conjunction with methods and processes for the purification of phytosterols and triterpenes, or plant sterols, from vegetable oils. Phytosterols that can be purified using methods of the invention include □-sitosterol, campesterol, stigmasterol, stigmastanol, □-sitostanol, sitostanol, desmosterol, chalinasterol, poriferasterol, clionasterol and brassicasterol. Plant sterols are important agricultural products for health and nutritional industries. Thus, compositions (e.g., esterases) and methods of the invention can be used to make emulsifiers for cosmetic manufacturers and steroidal intermediates and precursors for the production of hormone pharmaceuticals. The compositions (e.g., esterases) and methods of the invention can be used to make (e.g., purify) analogs of phytosterols and their esters for use as cholesterol-lowering agents with cardiologic health benefits. The compositions (e.g., esterases) and methods of the invention can be used to purify plant sterols to reduce serum cholesterol levels by inhibiting cholesterol absorption in the intestinal lumen. The compositions (e.g., esterases) and methods of the invention can be used to purify plant sterols that have immunomodulating properties at extremely low concentrations, including enhanced cellular response of T lymphocytes and cytotoxic ability of natural killer cells against a cancer cell line. The compositions (e.g., esterases) and methods of the invention can be used to purify plant sterols for the treatment of pulmonary tuberculosis, rheumatoid arthritis, management of HIV-infested patients and inhibition of immune stress, e.g., in marathon runners.

The compositions (e.g., esterases) and methods of the invention can be used to purify sterol components present in the sterol fractions of commodity vegetable oils (e.g., coconut, canola, cocoa butter, corn, cottonseed, linseed, olive, palm, peanut, rice bran, safflower, sesame, soybean, sunflower oils), such as sitosterol (40.2-92.3 %), campesterol (2.6-38.6 %), stigmasterol (0-31 %) and 5-avenasterol (1.5 -29 %).

### Vegetable oil refining apparatus

The disclosure provides product of manufacture comprising a degumming system for the enzymatic treatment of chlorophyll-containing or chlorophyll-contaminated compositions comprising (a) a vegetable oil refining apparatus; and (b) a polypeptide having an chlorophyllase activity operably integrated into the vegetable oil refining apparatus, wherein the activity of the polypeptide comprises catalysis of a chlorophyll-modifying reaction, and the vegetable oil refining apparatus can react a chlorophyll-containing or chlorophyll-contaminated composition with the polypeptide to under conditions wherein the polypeptide can catalyze a chlorophyll-modifying reaction.

The products of manufacture can comprise any vegetable oil refining apparatus or combination thereof, e.g., an oil leaving expellor (e.g., from Pennwalt Corp.), or a gravitational gum separation device.

The disclosure provides product of manufacture comprising immobilized enzymes, e.g., an immobilized chlorophyllase, e.g., an esterase used in the invention. The product of manufacture, the chlorophyllase comprises a silica-immobilized chlorophyllase. The silica comprises a silica gel or equivalent. The silica comprises a TriSyl Silica or a SORBSIL RTM silica.

The products of manufacture comprise apparatus for adjusting pH, e.g., increasing pH ("caustic treatment"), and then, alternatively, neutralizing pH.

The invention will be further described with reference to the following examples; however, it is to be understood that the invention is not limited to such examples.

### EXAMPLES

### EXAMPLE 1: Exemplary Esterase Activity Assay

The following example demonstrates an exemplary esterase (chlorophyllase activity) assay for isolating and characterizing enzymes of the invention and the nucleic acids that encode them, and to determine if a polypeptide is within the scope of the invention.

Esterases were screened for activity on chlorophyll from spinach to produce chlorophyllide. In this exemplary esterase (chlorophyllase activity) assay the esterase screening format comprises:
- Plates screened in duplicate.
- Positive (CHLase) & negative controls on each plate.
- 1 mM CHL, 20% cell lysate, 20% acetone, pH 7.5, 0.01% HBT.
- 24 hr incubation time at 30°C in the dark.
- 100 mL reaction volume.
- Analysis by LC-VIS; injection of 1 mL sample.

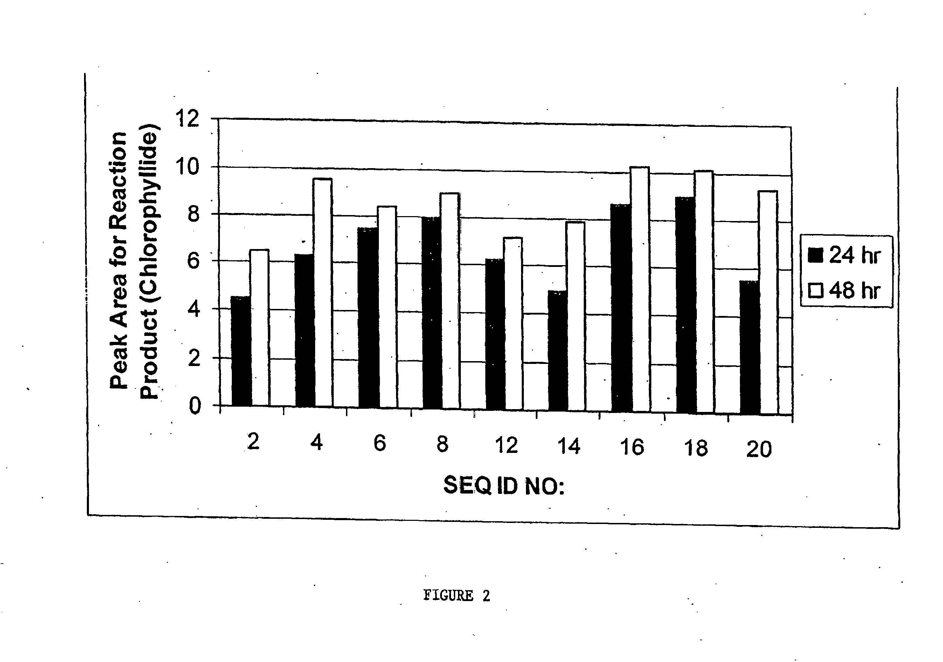

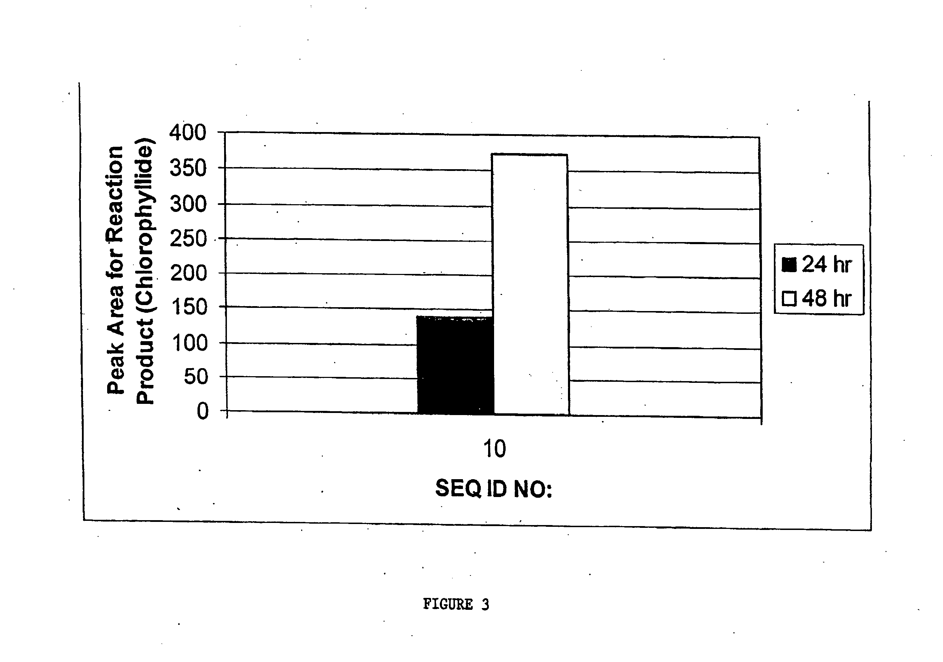

This esterase screening method used HPLC to analyze reaction products. Figure 2 and Figure 3 illustrate data showing the results of the esterase (chlorophyllase activity) activity assay using the indicated exemplary enzymes disclosed herein.

For the HPLC:
Column: Cromolith SpeedROD RP-18e 50-4, 6 mm (Cat# UM1082/086) Flow: 1.0 mL/min; Injection: 1.0 mL.

| | | | | |
|---|---|---|---|---|
| A: H2O | | | | |
| B: MEOH + 1mM | | | | |
| NH4OAc | | | | |
| C: MTBE | | | | |

| T (min) | | A | B | C |
|---|---|---|---|---|
| | 0 | 10% | 80% | 10% |
| | 2.3 | 10% | 80% | 10% |
| | 2.3 | | | |
| 1 | | 0% | 50% | 50% |
| | 4 | 0% | 50% | 50% |
| | 4.1 | 10% | 80% | 10% |
| | 7 | 10% | 80% | 10% |

| DAD signal | L (nm) | Bw | Reference I | Bw |
|---|---|---|---|---|
| 1 | 660 | 20 | 710 nm | 10 |

| Compound | Rt |
|---|---|
| CHLa | 4.20 |
| CHLb | 4.15 |
| PHPa | 4.30 |
| PHPb | 4.25 |
| CHPa | 0.85 |
| CHPb | 0.80 |
| PHBa | 1.00 |
| PHBb | 0.95 |

The data illustrated in Figure 2 illustrates increased levels of reaction product between 24 hr and 48 hr time points, were the levels of reaction product indicate chlorophyllase activity for SEQ ID NO:2, SEQ ID NO:4, SEQ ID NO:6, SEQ ID NO:8, SEQ ID NO:12, SEQ ID NO:14, SEQ ID NO:16, SEQ ID NO:18 and SEQ ID NO:20. The data illustrated in Figure 3 illustrates increased levels of reaction product between 24 hr and 48 hr time points, were the levels of reaction product indicate chlorophyllase activity for SEQ ID NO:10.
## Claims

1. Use of at least one polypeptide as set forth in SEQ ID NO: 10, or of at least one polypeptide which is a variant of SEQ ID NO:10 and the variant is at least 90%, 91 %, 92%, 93%, 94%, 95%, 96%, 97%, 98%, or 99% identical to SEQ ID NO:10, having a chlorophyllase activity in an enzymatic treatment that is a de-coloring of a chlorophyll-containing composition under conditions wherein the polypeptide can catalyze hydrolysis of the chlorophyll to generate a chlorophyllide and a phytol; and the chlorophyllide and phytol are separated.

2. Use of at least one polypeptide as set forth in SEQ ID NO: 10, or of at least one polypeptide which is a variant of SEQ ID NO:10 and the variant is at least 90%, 91%. 92%, 93%, 94%, 95%, 96%, 97%, 98%, or 99% identical to SEQ ID NO:10, having a chlorophyllase activity in an enzymatic treatment that is a de-coloring of a pheophytin-containing composition under conditions wherein the polypeptide can catalyze hydrolysis of the pheophytin to generate a pheophorbide and a phytol; and the pheophorbide and phytol are separated.

3. The use of any claim 1 or 2, wherein the polypeptide is immobilized on: an array, a cell, a metal, a resin, a polymer, a ceramic, a glass, a microelectrode, a graphitic particle, a bead, a gel, a plate, a capillary tube, an inorganic support or organic support, and optionally the support comprises: an alumina, celite, Dowex-1-chloride, glass beads, silica, silica gel, alginate hydrogel, or alginate bead.

4. The use of any of claims 1 to 3, wherein the chlorophyll- *or pheophytin-* containing composition:
(a) is derived from a plant, an animal or an algae, or a mixture thereof,
(b) comprises a plant material, plant oil or plant extract,
(c) comprises a vegetable oil or a seed oil,
(d) comprises a palm oil or a canola oil,
(e) comprises a crude oil or a refined oil,
(f) comprises an undiluted crude oil preparation, or
(g) comprises an algae preparation.

5. The use of any of claims 1 to 4, wherein at least one step of the treatment is performed in a reaction vessel, and optionally the reaction vessel comprises a gravitational gum separation device or a holding tank.

6. The use of any of claims 1 to 4, wherein at least one step of the treatment is performed in a cell extract or in a whole cell.

7. The use of any of claims 1 to 6, wherein the polypeptide is used with a lipoxygenase.

8. The use of any of claims 1 to 7, wherein the treatment further comprises modifying pH to promote aqueous separation of a chlorophyllide or pheophorbide.

9. The use of any of claims 1 to 8, wherein the treatment is combined with a caustic neutralization step.

10. The use of any of claims 1 to 9, wherein polypeptide is used with a phospholipase and optionally the phospholipase is a phospholipase C.

11. The use of any of claims 1 to 10, wherein the treatment is included in a degumming process.

12. The use of any of claims 1 to 11, wherein the treatment further comprises the removal of residual chlorophyll or pheophytin; pesticides; or a polycyclic aromatic hydrocarbons.

13. The use of any of claims 1 to 12, wherein the polypeptide is encoded by a nucleic acid as set forth in SEQ ID NO: 9.

14. The use of any of claims 1 to 13, wherein the enzymatic treatment of a chlorophyllor *pheophytin*-containing composition is combined with a degumming process and a caustic neutralization step.

15. The use of the polypeptide of any of the claims 1 to 12, wherein the polypeptide is encoded by a variant of the nucleic acid as set forth in SEQ ID NO:9 and the variant is at least 90%, 91%, 92%, 93%. 94%, 95%, 96%, 97%. 98%, or 99% identical to SEQ ID NO:9.

## Patentansprüche

1. Verwendung von mindestens einem Polypeptid gemäß SEQ ID NO: 10 oder von mindestens einem Polypeptid, bei dem es sich um eine Variante von SEQ ID NO: 10 handelt und die Variante zu mindestens 90%, 91%, 92%, 93%, 94%, 95%, 96%, 97%, 98% oder 99% mit SEQ ID NO: 10 identisch ist, das Chlorophyllaseaktivität aufweist, in einer enzymatischen Behandlung, bei der es sich um eine Entfärbung einer chlorophyllhaltigen Zusammensetzung handelt, und zwar unter Bedingungen, unter denen das Polypeptid die Hydrolyse des Chlorophylls zu einem Chlorophyllid und einem Phytol katalysieren kann, und das Chlorophyllid und das Phytol getrennt werden.

2. Verwendung von mindestens einem Polypeptid gemäß SEQ ID NO: 10 oder von mindestens einem Polypeptid, bei dem es sich um eine Variante von SEQ ID NO: 10 handelt und die Variante zu mindestens 90%, 91%, 92%, 93%, 94%, 95%, 96%, 97%, 98% oder 99% mit SEQ ID NO: 10 identisch ist, das Chlorophyllaseaktivität aufweist, in einer enzymatischen Behandlung, bei der es sich um eine Entfärbung einer phäophytinhaltigen Zusammensetzung handelt, und zwar unter Bedingungen, unter denen das Polypeptid die Hydrolyse des Phäophytins zu einem Phäophorbid und einem Phytol katalysieren kann, und das Phäophorbid und das Phytol getrennt werden.

3. Verwendung nach einem beliebigen Anspruch 1 oder 2, wobei das Polypeptid auf: einem Array, einer Zelle, einem Metall, einem Harz, einem Polymer, einem Keramikprodukt, einem Glas, einer Mikroelektrode, einem Graphitteilchen, einem Bead, einem Gel, einer Platte, einer Kapillarröhre, einem anorganischen Träger oder organischen Träger immobilisiert ist und der Träger optional Folgendes umfasst: ein Aluminiumoxid, Celite, Dowex-1-chlorid, Glas-Beads, Silika, Silikagel, Alginathydrogel oder Alginat-Bead.

4. Verwendung nach einem der Ansprüche 1 bis 3, wobei die chlorophyll- oder phäophytinhaltige Zusammensetzung:
(a) von einer Pflanze, einem Tier oder einer Alge oder einer Mischung davon stammt,
(b) ein Pflanzenmaterial, ein Pflanzenöl oder einen Pflanzenextrakt umfasst,
(c) ein vegetabiles Öl oder ein Samenöl umfasst,
(d) ein Palmöl oder ein Canolaöl umfasst,
(e) ein Rohöl oder ein raffiniertes Öl umfasst,
(f) ein unverdünntes Rohölpräparat umfasst, oder
(g) ein Algenpräparat umfasst.

5. Verwendung nach einem der Ansprüche 1 bis 4, wobei mindestens ein Behandlungsschritt in einem Reaktionsgefäß erfolgt und das Reaktionsgefäß optional eine Gummi-Schwerkraftabscheidevorrichtung oder einen Speicherbehälter umfasst.

6. Verwendung nach einem der Ansprüche 1 bis 4, wobei mindestens ein Schritt der Behandlung in einem Zellextrakt oder in einer ganzen Zelle erfolgt.

7. Verwendung nach einem der Ansprüche 1 bis 6, wobei das Polypeptid mit einer Lipoxygenase verwendet wird.

8. Verwendung nach einem der Ansprüche 1 bis 7, wobei die Behandlung weiterhin das Modifizieren des pH-Werts umfasst, um die wässrige Trennung eines Chlorophyllids oder Phäophorbids zu fördern.

9. Verwendung nach einem der Ansprüche 1 bis 8, wobei die Behandlung mit einem Laugenneutralisierungsschritt kombiniert wird.

10. Verwendung nach einem der Ansprüche 1 bis 9, wobei das Polypeptid mit einer Phospholipase verwendet wird und es sich bei der Phospholipase optional um eine Phospholipase C handelt.

11. Verwendung nach einem der Ansprüche 1 bis 10, wobei die Behandlung in einem Entschleimungsschritt beinhaltet ist.

12. Verwendung nach einem der Ansprüche 1 bis 11, wobei die Behandlung weiterhin die Entfernung von restlichem Chlorophyll oder Phäophytin; von Pestiziden; oder von polycyclischen aromatischen Kohlenwasserstoffen umfasst.

13. Verwendung nach einem der Ansprüche 1 bis 12, wobei das Polypeptid von einer Nukleinsäure gemäß SEQ ID NO: 9 codiert wird.

14. Verwendung nach einem der Ansprüche 1 bis 13, wobei die enzymatische Behandlung einer chlorophylloder phäophytinhaltigen Zusammensetzung mit einem Entschleimungsverfahren und einem Laugenneutralisierungsschritt kombiniert wird.

15. Verwendung des Polypeptids nach einem der Ansprüche 1 bis 12, wobei das Polypeptid von einer Variante der Nukleinsäure gemäß SEQ ID NO: 9 codiert wird und die Variante zu mindestens 90%, 91%, 92%, 93%, 94%, 95%, 96%, 97%, 98% oder 99% mit SEQ ID NO: 9 identisch ist.

## Revendications

1. Utilisation d'au moins un polypeptide, tel qu'indique dans la SEQ ID n° : 10, ou d'au moins un polypeptide qui est un variant de la SEQ ID n° : 10, et le variant est identique, au moins à 90%, 91%, 92%, 93%, 94%, 95%, 96%, 97%, 98% ou 99%, à la SEQ ID n° : 10, ayant une activité de chlorophyllase dans un traitement enzymatique, qui est une décoloration d'une composition contenant de la chlorophylle dans des conditions dans lesquelles le polypeptide peut catalyser l'hydrolyse de la chlorophylle pour générer un chlorophyllide et un phytol ; et les chlorophyllide et phytol sont séparés.

2. Utilisation d'au moins un polypeptide, tel qu'indique dans la SEQ ID n° : 10, ou d'au moins un polypeptide qui est un variant de la SEQ ID n° : 10, et le variant est identique, au moins à 90%, 91%, 92%, 93%, 94%, 95%, 96%, 97%, 98% ou 99%, à la SEQ ID n° : 10, ayant une activité de chlorophyllase dans un traitement enzymatique, qui est une décoloration d'une composition contenant de la phéophytine dans des conditions dans lesquelles le polypeptide peut catalyser l'hydrolyse de la phéophytine pour générer un phéophorbide et un phytol ; et les phéophorbide et phytol sont séparés.

3. Utilisation selon l'une quelconque des revendications 1 ou 2, dans laquelle le polypeptide est immobilisé sur : une matrice, une cellule, un métal, une résine, un polymère, une céramique, du verre, une microélectrode, une particule de graphite, une bille, un gel, une plaque, un tube capillaire, un support inorganique ou un support organique, et, en option, le support comprend : de l'alumine, de la célite, du chlorure de Dowex-1, des billes de verre, de la silice, du gel de silice, un hydrogel d'alginate ou une bille d'alginate.

4. Utilisation selon l'une quelconque des revendications 1 à 3, dans laquelle la composition contenant de la chlorophylle ou de la phéophytine :
(a) est dérivée d'une plante, d'un animal ou d'une algue ou bien d'un mélange de ceux-ci,
(b) comprend un matériel végétal, une huile végétale ou un extrait de plante,
(c) comprend une huile végétale ou une huile de graines,
(d) comprend de l'huile de palme ou de l'huile de colza canola,
(e) comprend une huile brute ou une huile raffinée,
(f) comprend une préparation à base d'huile brute non diluée ou
(g) comprend une préparation à base d'algues.

5. Utilisation selon l'une quelconque des revendications 1 à 4, dans laquelle au moins une étape du traitement est exécutée dans un récipient de réaction et, en option, le récipient de réaction comprend un dispositif de séparation de la gomme par gravitation ou un réservoir de stockage.

6. Utilisation selon l'une quelconque des revendications 1 à 4, dans laquelle au moins une étape du traitement est exécutée dans un extrait cellulaire ou dans une cellule entière.

7. Utilisation selon l'une quelconque des revendications 1 à 6, dans laquelle le polypeptide est utilisé avec une lipo-oxygénase.

8. Utilisation selon l'une quelconque des revendications 1 à 7, dans laquelle le traitement comprend en outre la modification du pH pour favoriser la séparation aqueuse d'un chlorophyllide ou d'un phéophorbide.

9. Utilisation selon l'une quelconque des revendications 1 à 8, dans laquelle le traitement est combiné avec une étape de neutralisation caustique.

10. Utilisation selon l'une quelconque des revendications 1 à 9, dans laquelle un polypeptide est utilisé avec une phospholipase et, en option, la phospholipase est une phospholipase C.

11. Utilisation selon l'une quelconque des revendications 1 à 10, dans laquelle le traitement est inclus dans un processus de dégommage.

12. Utilisation selon l'une quelconque des revendications 1 à 11, dans laquelle le traitement comprend en outre le retrait : de la chlorophylle ou de la phéophytine résiduelle ; de pesticides ; ou d'hydrocarbures aromatiques polycycliques.

13. Utilisation selon l'une quelconque des revendications 1 à 12, dans laquelle le polypeptide est codé par un acide nucléique tel qu'indiqué dans la SEQ ID n° : 9.

14. Utilisation selon l'une quelconque des revendications 1 à 13, dans laquelle le traitement enzymatique d'une composition contenant de la chlorophylle ou de la phéophytine est combiné avec un processus de dégommage ainsi qu'une étape de neutralisation caustique.

15. Utilisation du polypeptide selon l'une quelconque des revendications 1 à 12, dans laquelle le polypeptide est codé par un variant de l'acide nucléique tel qu'indiqué dans la SEQ ID n° : 9 et le variant est identique, au moins à 90%, 91%, 92%, 93%, 94%, 95%, 96%, 97%, 98% ou 99%, à la SEQ ID n° : 9.


<110> DIVERSA CORPORATION LAM, David WEINER, David HITCHMAN, Timothy BARTON, Nelson Robert BURK, Mark J.
<120> COMPOSITIONS AND METHODS FOR ENZYMATIC DECOLORIZATION OF CHLOROPHYLL
<130> 564462013240
<140> Not Yet Assigned
   <141> Concurrently Herewith
<150> US 60/580,447
   <151> 2004-06-16
<160> 27
<170> PatentIn version 3.1
<210> 1
   <211> 915
   <212> DNA
   <213> Unknown
<220>
   <223> Obtained from environmental sample
<400> 1
   atgtcgcgtg tctgtcttcc actcacactc acactcgcac tcacactctc tgccagagca    60
   gcggagccga ccacggtgaa gctctggccc ggcaaagcgc ccggcgagac gaaggacatc   120
   ggcccggaga agtatctcga aaccaagaag gggcagcttg acatcaagcg gctcaccaac   180
   gtgagcgaac cgaccatcac gatctattcg ccgccaaagg agaaggcgaa cgacacagtg   240
   gtgattgtcg cgcccggcgg cgggtacaac atcctcgcca tcgaacacga agggaccgat   300
   gtctgcgagt ggctgaactc gctgggcgtg accgcggtgc tgctcaagta tcgcgtgccg   360
   cggcgcccca tgcagtcgcc ggacaacctc gcgatgattc aggacgcgca gcgcgccatc   420
   agtcttgttc gcagtatgca gaaagagttg ggcattcacc caacacgtgt gggaatgctc   480
   ggcttttcgg ccggggggaa cctcaccgct tgcaccgcgt tggccgaaaa gcggatgtac   540
   gagaacatcg acaaggtgga cgaggtattc aactgcacgc cgaacttcgc catcctcgtg   600
   taccccgcgt atctcgtcga gaaggatgga acgctgcgcg ccgagttcaa agtgaagatc   660
   gattcgccgc cgatgttctt cgtgcattcg tccgacgaca acgtgagcag tgaaaacagc   720
   gtggcgcttt acctggcact caagaagaac aaggtgccgg cggaaatgca cctctacgcc   780
   agcggcgggc acggctacgg aatgcgcaag gttccgcatc cgtgcgcaag ctggcccgac   840
   cgcgccgcgg agtggatgaa agcacaccgg ctgcttgaga aggccaagcc cgagccggat   900
   gggaagaagg agtaa                                                    915
<210> 2
   <211> 304
   <212> PRT
   <213> Unknown
<220>
   <223> Obtained from environmental sample
<220>
   <221> SIGNAL
   <222> (1)...(20)
<400> 2
   Met Ser Arg Val Cys Leu Pro Leu Thr Leu Thr Leu Ala Leu Thr Leu
   1               5                   10                  15
   Ser Ala Arg Ala Ala Glu Pro Thr Thr Val Lys Leu Trp Pro Gly Lys
               20                  25                  30
   Ala Pro Gly Glu Thr Lys Asp Ile Gly Pro Glu Lys Tyr Leu Glu Thr
           35                  40                  45
   Lys Lys Gly Gln Leu Asp Ile Lys Arg Leu Thr Asn Val Ser Glu Pro
       50                  55                  60
   Thr Ile Thr Ile Tyr Ser Pro Pro Lys Glu Lys Ala Asn Asp Thr Val
   65                  70                  75                  80
   Val Ile Val Ala Pro Gly Gly Gly Tyr Asn Ile Leu Ala Ile Glu His
                   85                  90                  95
   Glu Gly Thr Asp Val Cys Glu Trp Leu Asn Ser Leu Gly Val Thr Ala
               100                 105                 110
   Val Leu Leu Lys Tyr Arg Val Pro Arg Arg Pro Met Gln Ser Pro Asp
           115                 120                 125
   Asn Leu Ala Met Ile Gln Asp Ala Gln Arg Ala Ile Ser Leu Val Arg
       130                 135                 140
   Ser Met Gln Lys Glu Leu Gly Ile His Pro Thr Arg Val Gly Met Leu
   145                 150                 155                 160
   Gly Phe Ser Ala Gly Gly Asn Leu Thr Ala Cys Thr Ala Leu Ala Glu
                   165                 170                 175
   Lys Arg Met Tyr Glu Asn Ile Asp Lys Val Asp Glu Val Phe Asn Cys
               180                 185                 190
   Thr Pro Asn Phe Ala Ile Leu Val Tyr Pro Ala Tyr Leu Val Glu Lys
           195                 200                 205
   Asp Gly Thr Leu Arg Ala Glu Phe Lys Val Lys Ile Asp Ser Pro Pro
       210                 215                 220
   Met Phe Phe Val His Ser Ser Asp Asp Asn Val Ser Ser Glu Asn Ser
   225                 230                 235                 240
   Val Ala Leu Tyr Leu Ala Leu Lys Lys Asn Lys Val Pro Ala Glu Met
                   245                 250                 255
   His Leu Tyr Ala Ser Gly Gly His Gly Tyr Gly Met Arg Lys Val Pro
               260                 265                 270
   His Pro Cys Ala Ser Trp Pro Asp Arg Ala Ala Glu Trp Met Lys Ala
           275                 280                 285
   His Arg Leu Leu Glu Lys Ala Lys Pro Glu Pro Asp Gly Lys Lys Glu
       290                 295                 300
<210> 3
   <211> 927
   <212> DNA
   <213> Unknown
<220>
   <223> Obtained from environmental sample
<400> 3
   atgtcacttg atccacaaac gaaatttgta ttagcccaat tggctgctgc cgatgctcct    60
   ccaatggaga ctttgtcacc ggaaatggct agacaggcat tcatattgcc gcaaggtgca   120
   gtggaggaag tagggaaggt agaaaatcga acgattcctg gtcctgcaac ggacatccct   180
   gtccgtgttt attaccctaa agaactccag cctgaaaatc ccgcgctagt tttctatcat   240
   ggcggcggct gggtaattgg caatttggac tctcatgacg atatttgccg tgctttaacc   300
   aacctcgcca actgcgtgac catttccgtc gactaccgcc tggctccaga gaataaattt   360
   cctgctgcgg ttgaggatgc ttatgctgct gcacaatatg tgtatgacca tgcagaagat   420
   ttcaaagtcg acaagacccg tattgccgtt ggcggtgaca gtgccggtgg aaatcttgcc   480
   gctgtcgtga cgaatctggc aaaagacaaa aactcacctt ctatttgttt ccaacttctg   540
   atttatccaa gcaccaatgc aggtggtgag ccaacagcat caatggttga gaatgcccat   600
   ggctatttct tagaaaaagg cacgatggat tggttccgtg actgctacct gaacagtgaa   660
   gaagacaaac agaatccgct agtctcaccg atgctttatg atgatttcca aggactgcct   720
   ccagcaattg tgattactgc cgagtacgat ccattgcgag atgaaggcga agcatatgcc   780
   aaaaagctgg gcgaagcagg ggttgctgtc caaaccatcc gctttgacgg tacgattcat   840
   ggctttgtca gcatgtcggc tgtcattagc caagggaagg cggcattgga aaaagcagga   900
   gaggctttaa ctaaagcctt tcaataa                                       927
<210> 4
   <211> 308
   <212> PRT
   <213> Unknown
<220>
   <223> Obtained from environmental sample
<400> 4
   Met Ser Leu Asp Pro Gln Thr Lys Phe Val Leu Ala Gln Leu Ala Ala
   1               5                   10                  15
   Ala Asp Ala Pro Pro Met Glu Thr Leu Ser Pro Glu Met Ala Arg Gln
               20                  25                  30
   Ala Phe Ile Leu Pro Gln Gly Ala Val Glu Glu Val Gly Lys Val Glu
           35                  40                  45
   Asn Arg Thr Ile Pro Gly Pro Ala Thr Asp Ile Pro Val Arg Val Tyr
       50                  55                  60
   Tyr Pro Lys Glu Leu Gln Pro Glu Asn Pro Ala Leu Val Phe Tyr His
   65                  70                  75                  80
   Gly Gly Gly Trp Val Ile Gly Asn Leu Asp Ser His Asp Asp Ile Cys
                   85                  90                  95
   Arg Ala Leu Thr Asn Leu Ala Asn Cys Val Thr Ile Ser Val Asp Tyr
               100                 105                 110
   Arg Leu Ala Pro Glu Asn Lys Phe Pro Ala Ala Val Glu Asp Ala Tyr
           115                 120                 125
   Ala Ala Ala Gln Tyr Val Tyr Asp His Ala Glu Asp Phe Lys Val Asp
       130                 135                 140
   Lys Thr Arg Ile Ala Val Gly Gly Asp Ser Ala Gly Gly Asn Leu Ala
   145                 150                 155                 160
   Ala Val Val Thr Asn Leu Ala Lys Asp Lys Asn Ser Pro Ser Ile Cys
                   165                 170                 175
   Phe Gln Leu Leu Ile Tyr Pro Ser Thr Asn Ala Gly Gly Glu Pro Thr
               180                 185                 190
   Ala Ser Met Val Glu Asn Ala His Gly Tyr Phe Leu Glu Lys Gly Thr
           195                 200                 205
   Met Asp Trp Phe Arg Asp Cys Tyr Leu Asn Ser Glu Glu Asp Lys Gln
   210 215 220
   Asn Pro Leu Val Ser Pro Met Leu Tyr Asp Asp Phe Gln Gly Leu Pro
   225 230 235 240
   Pro Ala Ile Val Ile Thr Ala Glu Tyr Asp Pro Leu Arg Asp Glu Gly
   245 250 255
   Glu Ala Tyr Ala Lys Lys Leu Gly Glu Ala Gly Val Ala Val Gln Thr
   260 265 270
   Ile Arg Phe Asp Gly Thr Ile His Gly Phe Val Ser Met Ser Ala Val
   275 280 285
   Ile Ser Gln Gly Lys Ala Ala Leu Glu Lys Ala Gly Glu Ala Leu Thr
   290 295 300
   Lys Ala Phe Gln
   305
<210> 5
   <211> 672
   <212> DNA
   <213> Unknown
<220>
   <223> Obtained from environmental sample
<400> 5
   atgagaagaa ttgttttcct ttatattctg gcgttgctct gcgtatcctg tgcgaaccgg 60
   aatccttctg tctcggaccc cgctccgtca ccgaccttgg gggagcaatc gtcagcgagt 120
   ttaccgaaga tcgtagcatt cggcgacagc ctaactgctg gctttggtct ttcccagaat 180
   gaaagttatc cagcgctttt gcaggaacga ctgagacagg acggttatga ttacgaagtg 240
   atcaatgccg gagtttcagg cgataccagc gcgggcggtg tgcgtaggat cgactgggtt 300
   ctggatgaga gcgtacgaat tctaattctt gaattgggtg ctaatgattt tcttcgtggc 360
   catccagtag ctcagactaa gaaaaacctc gccgttatca tcgaacgtgc tcaggaaaaa 420
   aacgtcaggg ttctgctagc agggatgttt gcaccgacga atactggatg ggagtaccag 480
   gggcagatcc agcaaatgtt taacgatctt tcgcgagaaa aagccgtacc actaattcca 540
   ttctttctcg agggagtagc cggcattcca actctgaatc tagctgatgg cattcatccc 600
   aatgcggctg gcacaaagat cgttgctgag aatgtgtaca agtatctaaa accgatgctc 660
   ccatctgatt aa 672
<210> 6
   <211>223
   <212> PRT
   <213> Unknown
<220>
   <223> Obtained from environmental sample
<220>
   <221> SIGNAL
   <222> (1)...(25)
<220>
   <221> DOMAIN
   <222> (44)...(212)
   <223> GDSL-like Lipase/Acylhydrolase
<400> 6
   Met Arg Arg Ile Val Phe Leu Tyr Ile Leu Ala Leu Leu Cys Val Ser
   1               5                   10                  15
   Cys Ala Asn Arg Asn Pro Ser Val Ser Asp Pro Ala Pro Ser Pro Thr
               20                  25                  30
   Leu Gly Glu Gln Ser Ser Ala Ser Leu Pro Lys Ile Val Ala Phe Gly
           35                  40                  45
   Asp Ser Leu Thr Ala Gly Phe Gly Leu Ser Gln Asn Glu Ser Tyr Pro
       50                  55                  60
   Ala Leu Leu Gln Glu Arg Leu Arg Gln Asp Gly Tyr Asp Tyr Glu Val
   65                  70                  75                  80
   Ile Asn Ala Gly Val Ser Gly Asp Thr Ser Ala Gly Gly Val Arg Arg
                   85                  90                  95
   Ile Asp Trp Val Leu Asp Glu Ser Val Arg Ile Leu Ile Leu Glu Leu
               100                 105                 110
   Gly Ala Asn Asp Phe Leu Arg Gly His Pro Val Ala Gln Thr Lys Lys
           115                 120                 125
   Asn Leu Ala Val Ile Ile Glu Arg Ala Gln Glu Lys Asn Val Arg Val
       130                 135                 140
   Leu Leu Ala Gly Met Phe Ala Pro Thr Asn Thr Gly Trp Glu Tyr Gln
   145                 150                 155                 160
   Gly Gln Ile Gln Gln Met Phe Asn Asp Leu Ser Arg Glu Lys Ala Val
                   165                 170                 175
   Pro Leu Ile Pro Phe Phe Leu Glu Gly Val Ala Gly Ile Pro Thr Leu
               180                 185                 190
   Asn Leu Ala Asp Gly Ile His Pro Asn Ala Ala Gly Thr Lys Ile Val
           195                 200                 205
   Ala Glu Asn Val Tyr Lys Tyr Leu Lys Pro Met Leu Pro Ser Asp
       210                 215                 220
<210> 7
   <211> 783
   <212> DNA
   <213> Unknown
<220>
   <223> Obtained from environmental sample
<400> 7
   atgacacgaa aaaaaatcgg cttggcttta tccggcggcg cggcgcgtgg cttcgcacat     60
   ctcggagttt tgaaagtttt cgccgaacac ggcattcccg tcgatttcgt cgccggaacg    120
   agcgccggtt cgtttgcggg cgcggctttc gcttccggtt taagcgtggc ggaaatcatc    180
   gaaatgtcaa ggaaaatcag ttggtttcgg atgaccggat tttcgtactc gccgaaaggc    240
   ttgctgtcga acgcgccgat gggagcgttt atcaatcagc attttccgcg caaaaaattt    300
   gaagaactgc cgataccgtt cgcggcaatt acgtgcgatc tcgaaaccgg cgaggaaatc    360
   gtgcttaaag aaaccggcga cgtggcgact gccgtccgtg cgagctgcgc gctgcccggc    420
   gtcttcgtgc caatcgaata cggcggacgg cgattgatag acggcggcgt cgtgtcgaac    480
   gtgccgacga gagccgtcaa aaagctcggc gcggaaatca ttattgcggt tgacgttctg    540
   gcgtgcggca caacttactg gggttcgcct tccactttgc tcggcatctt tttccaatcg     600
   gcgatgatgc ttctccgcgc cgcttcaaaa tctcatcatt accgcgcgag cgtcgtcatc    660
   acgccgcaaa tcgctcatct gcgcccggac gaaatcagca aaatggacga atttatcaaa    720
   gcgggcgaac aagccgcgct tgaaaaagtt gacgaaatca aggctttgct cgccgaacaa    780
   taa                                                                  783
<210> 8
   <211> 260
   <212> PRT
   <213> Unknown
<220>
   <223> Obtained from environmental sample
<220>
   <221> DOMAIN
   <222> (8)...(167)
   <223> Patatin-like phospholipase
<400> 8
   Met Thr Arg Lys Lys Ile Gly Leu Ala Leu Ser Gly Gly Ala Ala Arg
   1               5                   10                  15
   Gly Phe Ala His Leu Gly Val Leu Lys Val Phe Ala Glu His Gly Ile
               20                  25                  30
   Pro Val Asp Phe Val Ala Gly Thr Ser Ala Gly Ser Phe Ala Gly Ala
           35                  40                  45
   Ala Phe Ala Ser Gly Leu Ser Val Ala Glu Ile Ile Glu Met Ser Arg
       50                  55                  60
   Lys Ile Ser Trp Phe Arg Met Thr Gly Phe Ser Tyr Ser Pro Lys Gly
   65                  70                  75                  80
   Leu Leu Ser Asn Ala Pro Met Gly Ala Phe Ile Asn Gln His Phe Pro
                   85                  90                  95
   Arg Lys Lys Phe Glu Glu Leu Pro Ile Pro Phe Ala Ala Ile Thr Cys
               100                 105                 110
   Asp Leu Glu Thr Gly Glu Glu Ile Val Leu Lys Glu Thr Gly Asp Val
           115                 120                 125
   Ala Thr Ala Val Arg Ala Ser Cys Ala Leu Pro Gly Val Phe Val Pro
       130                 135                 140
   Ile Glu Tyr Gly Gly Arg Arg Leu Ile Asp Gly Gly Val Val Ser Asn
   145                 150                 155                 160
   Val Pro Thr Arg Ala Val Lys Lys Leu Gly Ala Glu Ile Ile Ile Ala
                   165                 170                 175
   Val Asp Val Leu Ala Cys Gly Thr Thr Tyr Trp Gly Ser Pro Ser Thr
               180                 185                 190
   Leu Leu Gly Ile Phe Phe Gln Ser Ala Met Met Leu Leu Arg Ala Ala
           195                 200                 205
   Ser Lys Ser His His Tyr Arg Ala Ser Val Val Ile Thr Pro Gln Ile
       210                 215                 220
   Ala His Leu Arg Pro Asp Glu Ile Ser Lys Met Asp Glu Phe Ile Lys
   225                 230                 235                 240
   Ala Gly Glu Gln Ala Ala Leu Glu Lys Val Asp Glu Ile Lys Ala Leu
                   245                 250                 255
   Leu Ala Glu Gln
               260
<210> 9
   <211> 849
   <212> DNA
   <213> Unknown
<220>
   <223> Obtained from environmental sample
<400> 9
   atgttcaaca aagcacttcc tgcagcggcc gcggtcgccg gtctgttcct ttccacctcg      60
   gccatggcgc tgctgccgga tacgcccgga acccccttcc cgtcggtgtc cagcttcgaa     120
   aggtccggtc cctacagcac caccagccgc agcgaaggcc ccaactgccg gatctatcga     180
   ccgagcaacc tgggccagaa cggcgtccgc cacccggtca tcctctgggg caacggcacc     240
   ggtgccagcc cgaccaccta ccgcggcctg ctcgagcact gggccagcca cggcttcgtg     300
   gtcgccgccg ccgagacctc caacgccggc tccgggcgcg agatgctcaa ctgcctgagc     360
   tacctgcaga ccgaagccgg gcgcagcagc ggcacctacg tcggccggct caatctcggc     420
   cgcgtcggca cgtccggcca ctcgcagggc ggtggcggct cgatcatggc cgggcgcgac     480
   acccggatca aaaccaccgc gcccatgcag ccctacaccc tgggcctggg ccatgtgagt     540
   tcctcgcaga gccagcagaa cggcccgatg ttcctgatgt ccggcagcct ggacaccctg     600
   gccggcccga ccctgaacca ggcgccggtc tatcgccggg ccaacgtgcc ggtgttctgg     660
   ggcaccctgc gcggcgccag ccacttcgtg ccggtcggca gtgccggcgg ttaccgtggc     720
   ccgtccaccg cctggttccg ctaccagctg atggacgata cctcggcgcg cagccagttc     780
   gtcggcacca actgcggcct gtgccgcgac ttctcctgga ccgacatcca gcgcaagggc     840
   gcgctgtaa                                                             849
<210> 10
   <211> 282
   <212> PRT
   <213> Unknown
<220>
   <223> Obtained from environmental sample
<220>
   <221> SIGNAL
   <222> (1)...(23)
<400> 10
   Met Phe Asn Lys Ala Leu Pro Ala Ala Ala Ala Val Ala Gly Leu Phe
   1               5                   10                  15
   Leu Ser Thr Ser Ala Met Ala Leu Leu Pro Asp Thr Pro Gly Thr Pro
               20                  25                  30
   Phe Pro Ser Val Ser Ser Phe Glu Arg Ser Gly Pro Tyr Ser Thr Thr
           35                  40                  45
   Ser Arg Ser Glu Gly Pro Asn Cys Arg Ile Tyr Arg Pro Ser Asn Leu
       50                  55                  60
   Gly Gln Asn Gly Val Arg His Pro Val Ile Leu Trp Gly Asn Gly Thr
   65                  70                  75                  80
   Gly Ala Ser Pro Thr Thr Tyr Arg Gly Leu Leu Glu His Trp Ala Ser
                   85                  90                  95
   His Gly Phe Val Val Ala Ala Ala Glu Thr Ser Asn Ala Gly Ser Gly
               100                 105                 110
   Arg Glu Met Leu Asn Cys Leu Ser Tyr Leu Gln Thr Glu Ala Gly Arg
           115                 120                 125
   Ser Ser Gly Thr Tyr Val Gly Arg Leu Asn Leu Gly Arg Val Gly Thr
       130                 135                 140
   Ser Gly His Ser Gln Gly Gly Gly Gly Ser Ile Met Ala Gly Arg Asp
   145                 150                 155                 160
   Thr Arg Ile Lys Thr Thr Ala Pro Met Gln Pro Tyr Thr Leu Gly Leu
                   165                 170                 175
   Gly His Val Ser Ser Ser Gln Ser Gln Gln Asn Gly Pro Met Phe Leu
               180                 185                 190
   Met Ser Gly Ser Leu Asp Thr Leu Ala Gly Pro Thr Leu Asn Gln Ala
           195                 200                 205
   Pro Val Tyr Arg Arg Ala Asn Val Pro Val Phe Trp Gly Thr Leu Arg
       210                 215                 220
   Gly Ala Ser His Phe Val Pro Val Gly Ser Ala Gly Gly Tyr Arg Gly
   225                 230                 235                 240
   Pro Ser Thr Ala Trp Phe Arg Tyr Gln Leu Met Asp Asp Thr Ser Ala
                   245                 250                 255
   Arg Ser Gln Phe Val Gly Thr Asn Cys Gly Leu Cys Arg Asp Phe Ser
               260                 265                 270
   Trp Thr Asp Ile Gln Arg Lys Gly Ala Leu
           275                 280
<210> 11
   <211> 672
   <212> DNA
   <213> Unknown
<220>
   <223> Obtained from environmental sample
<400> 11
   gtgtccgccc ccgaagccgc gagcgtggag catgcggacc ccggcggggt cgccaccctg     60
   gcggaacgcc ccgcggcgcg tccgcgcatc gtcttcctgg gcgacagcct gaccgccggg    120
   tacggactgg cgcgggagca gtccgtgccc gcgctactcc agcggcggct cgaccgggag    180
   gggtatgagt acgaagtcgt caatgcgggc gtctcgggcg acacgtccgc cggcgggctg    240
   agccggctcg actggtcgct cgacggcgag gtcgccctgc tggtggtgga gctcggcgcc    300
   aacgacgggc tgcgcggcct gcccgtgtcc gcgatgaagc gcaacctgga cgccatcatc    360
   acgcgcgccc gcgcgcgggg catcaccgtg gtgctcgccg gcatggaagc gccgccgaac    420
   tacggcgcgg cctacacgac cgagttccgg caggcgtttc acgatctcgc ccgtacccac    480
   gacgtgccgt tcgtgccgtt ctttctcgaa ggggtagccg gcctgccgca cctcaacatc    540
   gccgacggca tccatcccaa cgccgagggc gcgcgcgtgg tcgaggccaa cgtctggcag    600
   gtgctggaac cgttactcga cgagcctggc cgggcggccg cctcgcgcgc gcagcccgcg    660
   gatggccgct ga                                                        672
<210> 12
   <211> 223
   <212> PRT
   <213> Unknown
<220>
   <223> Obtained from environmental sample
<220>
   <221> DOMAIN
   <222> (30)...(198)
   <223> GDSL-like Lipase/Acylhydrolase
<400> 12
   Met Ser Ala Pro Glu Ala Ala Ser Val Glu His Ala Asp Pro Gly Gly
   1               5                   10                  15
   Val Ala Thr Leu Ala Glu Arg Pro Ala Ala Arg Pro Arg Ile Val Phe
               20                  25                  30
   Leu Gly Asp Ser Leu Thr Ala Gly Tyr Gly Leu Ala Arg Glu Gln Ser
           35                  40                  45
   Val Pro Ala Leu Leu Gln Arg Arg Leu Asp Arg Glu Gly Tyr Glu Tyr
       50                  55                  60
   Glu Val Val Asn Ala Gly Val Ser Gly Asp Thr Ser Ala Gly Gly Leu
   65                  70                  75                  80
   Ser Arg Leu Asp Trp Ser Leu Asp Gly Glu Val Ala Leu Leu Val Val
                   85                  90                  95
   Glu Leu Gly Ala Asn Asp Gly Leu Arg Gly Leu Pro Val Ser Ala Met
               100                 105                 110
   Lys Arg Asn Leu Asp Ala Ile Ile Thr Arg Ala Arg Ala Arg Gly Ile
           115                 120                 125
   Thr Val Val Leu Ala Gly Met Glu Ala Pro Pro Asn Tyr Gly Ala Ala
       130                 135                 140
   Tyr Thr Thr Glu Phe Arg Gln Ala Phe His Asp Leu Ala Arg Thr His
   145                 150                 155                 160
   Asp Val Pro Phe Val Pro Phe Phe Leu Glu Gly Val Ala Gly Leu Pro
                   165                 170                 175
   His Leu Asn Ile Ala Asp Gly Ile His Pro Asn Ala Glu Gly Ala Arg
               180                 185                 190
   Val Val Glu Ala Asn Val Trp Gln Val Leu Glu Pro Leu Leu Asp Glu
           195                 200                 205
   Pro Gly Arg Ala Ala Ala Ser Arg Ala Gln Pro Ala Asp Gly Arg
       210                 215                 220
<210> 13
   <211> 900
   <212> DNA
   <213> Unknown
<220>
   <223> Obtained from environmental sample
<400> 13
   atgaaggcaa gctgccgcga catcgaaatc gaatacgaaa cattcggcca tccggacgac     60
   ccggccatcg tgctgatcat gggactcggt ggccagctca tcctgtggcc ggaagcgttc    120
   tgccgcatgc tggccgacgc tggtcactac gtggtgcgct tcgacaaccg cgacatcggc    180
   ctgtcgacgc atctcgatca ccttccccgc cccaacctgc cgctcgccgc gctccgccag    240
   gcgctgcgcc tgccagttcg cgccagttac atgctcgacg acatggcgga cgacgtcgcg    300
   ggcctgcttg atgcactgaa catcaagcag gcgcacgtcg tcggcgtgtc gatgggcggc    360
   atgatcgccc agctgctggc cgcacggcac gcgacccgcg tgcgcagcct gaccttgctg    420
   atgaccacca gcggcgcgcg caacgttccg ggcccctcac tcggcatgcg catggaaatg    480
   atccgtcgac cgcgcgacac ctcgcgcgag gggctgatcc gccatggtat gcgtacctgg    540
   cggatcatcg gcagcccgac gtacccgaag ccggaagccg aactgcgccg catcgtcgcc    600
   gagggctttg accgcgcgtt tcacccggcc ggtttcatgc gccagctgca cgccgttctc    660
   gcggcaccga gccgcgcacc cctgctgccg cgcatcaaac agccggccga cgtcattcac    720
   ggcgacgccg acctgctggt accagtggca gcggcacgtg atctggtgcg ccgcctgccg    780
   aacgccacgc tcgacatcgt gccgggcatg gggcatgact tcccgaccga gatcatgccg    840
   cgtatcgcgc gccgcattgt cgaaaccgcg gcacgcgacc cgcagcggct cgccgcctag    900
<210> 14
   <211> 299
   <212> PRT
   <213> Unknown
<220>
   <223> Obtained from environmental sample
<220>
   <221> DOMAIN
   <222> (49)...(288)
   <223> alpha/beta hydrolase fold
<400> 14
   Met Lys Ala Ser Cys Arg Asp Ile Glu Ile Glu Tyr Glu Thr Phe Gly
   1               5                   10                  15
   His Pro Asp Asp Pro Ala Ile Val Leu Ile Met Gly Leu Gly Gly Gln
               20                  25                  30
   Leu Ile Leu Trp Pro Glu Ala Phe Cys Arg Met Leu Ala Asp Ala Gly
           35                  40                  45
   His Tyr Val Val Arg Phe Asp Asn Arg Asp Ile Gly Leu Ser Thr His
       50                  55                  60
   Leu Asp His Leu Pro Arg Pro Asn Leu Pro Leu Ala Ala Leu Arg Gln
   65                  70                  75                  80
   Ala Leu Arg Leu Pro Val Arg Ala Ser Tyr Thr Leu Asp Asp Met Ala
                   85                  90                  95
   Asp Asp Val Ala Gly Leu Leu Asp Ala Leu Asn Ile Lys Gln Ala His
               100                 105                 110
   Val Val Gly Val Ser Met Gly Gly Met Ile Ala Gln Leu Leu Ala Ala
           115                 120                 125
   Arg His Ala Thr Arg Val Arg Ser Leu Thr Leu Leu Met Thr Thr Ser
       130                 135                 140
   Gly Ala Arg Asn Val Pro Gly Pro Ser Leu Gly Met Arg Met Glu Met
   145 150 155 160
   Ile Arg Arg Pro Arg Asp Thr Ser Arg Glu Gly Leu Ile Arg His Gly
   165 170 175
   Met Arg Thr Trp Arg Ile Ile Gly Ser Pro Thr Tyr Pro Lys Pro Glu
   180 185 190
   Ala Glu Leu Arg Arg Ile Val Ala Glu Gly Phe Asp Arg Ala Phe His
   195 200 205
   Pro Ala Gly Phe Met Arg Gln Leu His Ala Val Leu Ala Ala Pro Ser
   210 215 220
   Arg Ala Pro Leu Leu Pro Arg Ile Lys Gln Pro Ala Asp Val Ile His
   225 230 235 240
   Gly Asp Ala Asp Leu Leu Val Pro Val Ala Ala Ala Arg Asp Leu Val
   245 250 255
   Arg Arg Leu Pro Asn Ala Thr Leu Asp Ile Val Pro Gly Met Gly His
   260 265 270
   Asp Phe Pro Thr Glu Ile Met Pro Arg Ile Ala Arg Arg Ile Val Glu
   275 280 285
   Thr Ala Ala Arg Asp Pro Gln Arg Leu Ala Ala
   290 295
<210> 15
   <211> 759
   <212> DNA
   <213> Unknown
<220>
   <223> Obtained from environmental sample
<400> 15
   atgggcaaac aaatgcctta tagagtgatt tttccagtaa actaccaaac ttccaaaaaa    60
   cgctatccag tgatttacct tctgcacggc ctttcgggca attacaaaaa ctggaccgag   120
   aaaaccaaat tgacaaaata tgcaaccgaa tacaacttct tgattatcac agttgaaggc   180
   gagaacggtt ggtattctga cagtaaaatc aaacctaaca ggctttacga aagctacata   240
   atccaggaac tgatacctga agttgataaa aagtttcgca caatagcaga tcgcaatcac   300
   agagctatcg caggtctttc gatgggtggt tacggtgcga taaagtttgg cttgaaatat   360
   cctgaaaaat tcgctttgat tgggtccttt agcggcgctc ttgcggcaac ttcaatcaag   420
   gaaggcacag cgcttgagtg gattacaaaa accatcaatg atgctttcgg tcctgaagga   480
   agcgaatcca gaaaagaaaa cgatattttc cagattgcgc gcgacctgaa tgatgaacaa   540
   ataaagaaac ttccctttat ttactttgat tgcggaaccg aagattttct gttcaaagac   600
   aatcaggatt tcatgaaact acttgttgaa aagagaatca aacacgaata tagacaaaag   660
   cccggcaccc atacgtggga atactgggat agccaaataa aagaattctt gggtttagcc   720
   aacaaattta tagaaaaaac tgctttatgg cgatactaa                          759
<210> 16
   <211> 252
   <212> PRT
   <213> Unknown
<220>
   <223> Obtained from environmental sample
<220>
   <221> DOMAIN
   <222> (1)...(290)
   <223> Putative esterase
<400> 16
   Met Gly Lys Gln Met Pro Tyr Arg Val Ile Phe Pro Val Asn Tyr Gln
   1               5                   10                  15
   Thr Ser Lys Lys Arg Tyr Pro Val Ile Tyr Leu Leu His Gly Leu Ser
               20                  25                  30
   Gly Asn Tyr Lys Asn Trp Thr Glu Lys Thr Lys Leu Thr Lys Tyr Ala
           35                  40                  45
   Thr Glu Tyr Asn Phe Leu Ile Ile Thr Val Glu Gly Glu Asn Gly Trp
       50                  55                  60
   Tyr Ser Asp Ser Lys Ile Lys Pro Asn Arg Leu Tyr Glu Ser Tyr Ile
   65                  70                  75                  80
   Ile Gln Glu Leu Ile Pro Glu Val Asp Lys Lys Phe Arg Thr Ile Ala
                   85                  90                  95
   Asp Arg Asn His Arg Ala Ile Ala Gly Leu Ser Met Gly Gly Tyr Gly
               100                 105                 110
   Ala Ile Lys Phe Gly Leu Lys Tyr Pro Glu Lys Phe Ala Leu Ile Gly
           115                 120                 125
   Ser Phe Ser Gly Ala Leu Ala Ala Thr Ser Ile Lys Glu Gly Thr Ala
       130                 135                 140
   Leu Glu Trp Ile Thr Lys Thr Ile Asn Asp Ala Phe Gly Pro Glu Gly
   145                 150                 155                 160
   Ser Glu Ser Arg Lys Glu Asn Asp Ile Phe Gln Ile Ala Arg Asp Leu
                   165                 170                 175
   Asn Asp Glu Gln Ile Lys Lys Leu Pro Phe Ile Tyr Phe Asp Cys Gly
               180                 185                 190
   Thr Glu Asp Phe Leu Phe Lys Asp Asn Gln Asp Phe Met Lys Leu Leu
           195                 200                 205
   Val Glu Lys Arg Ile Lys His Glu Tyr Arg Gln Lys Pro Gly Thr His
       210                 215                 220
   Thr Trp Glu Tyr Trp Asp Ser Gln Ile Lys Glu Phe Leu Gly Leu Ala
   225                 230                 235                 240
   Asn Lys Phe Ile Glu Lys Thr Ala Leu Trp Arg Tyr
                   245                 250
<210> 17
   <211> 753
   <212> DNA
   <213> Unknown
<220>
   <223> Obtained from environmental sample
<400> 17
   atgaaaaagt ataaaacagg cctggtttta agtggtggcg gaacacgtgg ttttgcgcat      60
   ctgggagtga tagcagctct ttacgataag ggaattaaac ccgacataat ttcgggtaca     120
   agtgctggtg ccattgttgg cgcttttatt gctgccggga aaaatccaca cgacgttgtg     180
   gagattttta aaaagggatc gtttttcaat tacacaaaac tacagatacc ccgcgacggt     240
   ttgatgaaac tggatggact gaaggaattg tttcaaaagg aaattcatgt aaaaaacctc     300
   gaagagcttg aaatccccct ttttattgcc atttcgaatt taaataaagg aaccgtggaa     360
   tacagaaata gcggtctttt gggtgaaact gtgcttgcct catcttcgat ccccatactt     420
   tttgctccgg ttttaatcgg cgacgatttg tatgttgacg ggggattaat ggataacatt     480
   ccggttgaac ccatcaaact cgattgtgaa caaatcattg tttcaaatat cagtccgatt     540
   aatcccgttg aaaaaattaa aaatctgatt cacattgcta ctcgtacttt ttatatgagt     600
   gtaaacgcca acatgaaaca ggttaaaaaa tattccaccc attatattga accggacgga     660
   atagatactt acgaaatttt aagccgcacc cacgccgatg agttgtatga acttggatat     720
   aattcgacaa ttaaaatcct gaattcgcac taa                                 753
<210> 18
   <211> 250
   <212> PRT
   <213> Unknown
<220>
   <223> Obtained from environmental sample
<220>
   <221> DOMAIN
   <222> (8)...(166)
   <223> Patatin-like phospholipase
<400> 18
   Met Lys Lys Tyr Lys Thr Gly Leu Val Leu Ser Gly Gly Gly Thr Arg
   1               5                   10                  15
   Gly Phe Ala His Leu Gly Val Ile Ala Ala Leu Tyr Asp Lys Gly Ile
               20                  25                  30
   Lys Pro Asp Ile Ile Ser Gly Thr Ser Ala Gly Ala Ile Val Gly Ala
           35                  40                  45
   Phe Ile Ala Ala Gly Lys Asn Pro His Asp Val Val Glu Ile Phe Lys
       50                  55                  60
   Lys Gly Ser Phe Phe Asn Tyr Thr Lys Leu Gln Ile Pro Arg Asp Gly
   65                  70                  75                  80
   Leu Met Lys Leu Asp Gly Leu Lys Glu Leu Phe Gln Lys Glu Ile His
                   85                  90                  95
   Val Lys Asn Leu Glu Glu Leu Glu Ile Pro Leu Phe Ile Ala Ile Ser
               100                 105                 110
   Asn Leu Asn Lys Gly Thr Val Glu Tyr Arg Asn Ser Gly Leu Leu Gly
           115                 120                 125
   Glu Thr Val Leu Ala Ser Ser Ser Ile Pro Ile Leu Phe Ala Pro Val
       130                 135                 140
   Leu Ile Gly Asp Asp Leu Tyr Val Asp Gly Gly Leu Met Asp Asn Ile
   145                 150                 155                 160
   Pro Val Glu Pro Ile Lys Leu Asp Cys Glu Gln Ile Ile Val Ser Asn
                   165                 170                 175
   Ile Ser Pro Ile Asn Pro Val Glu Lys Ile Lys Asn Leu Ile His Ile
               180                 185                 190
   Ala Thr Arg Thr Phe Tyr Met Ser Val Asn Ala Asn Met Lys Gln Val
           195                 200                 205
   Lys Lys Tyr Ser Thr His Tyr Ile Glu Pro Asp Gly Ile Asp Thr Tyr
       210                 215                 220
   Glu Ile Leu Ser Arg Thr His Ala Asp Glu Leu Tyr Glu Leu Gly Tyr
   225                 230                 235                 240
   Asn Ser Thr Ile Lys Ile Leu Asn Ser His
                   245                 250
<210> 19
   <211> 1071
   <212> DNA
   <213> Unknown
<220>
   <223> Obtained from environmental sample
<400> 19
   atgggtgcga ataatccggt cacaaaattt gtaatggata aggggcaggg cactgcagca      60
   cgtttgctgg attgcctgcc gagctttgca caagaacgtc tggtaaaagc attggattat     120
   ccttatgact atcctgattt ggatccttat ataaaatgct tgatggcgat tcagattaaa     180
   cagggagagc acagttttat tagtgcagat gcggtgcagt cacgtcaact gtttgatgaa     240
   cgcatgaaag ctattcaggc gaaaccgacg ccggtcaagg cagtcgagga tctgcgtttg     300
   ccattgcaaa atggcactat ctttgcccga cattatcatc cggcaccaca gaaaaaattg     360
   ccgatggtca ttttctatca tggtggtgca tttatagtgg gtggtctgga tacgcatgat     420
   gagttctgcc gtttattggc ggtgcatgcc aaggtgcagg tactcagcgt ggcttatccg     480
   ctaacgcccg aatacagtcc tttgcagatg gtacaggtct gtgaagatgc tctggcttgg     540
   gtacatcaaa acatcaagca gttgaaaatc tataaaaacc agattgtagt ggcaggggat     600
   agtgcaggtg gaaatctggc ggcagtggtt gcgcagcgga gtgccgataa aatctatgca     660
   cctcgcgcac agttattgct ttacccggct gtcgatttta aaagccgcca tccttctttt     720
   tatgcataca atcagggcct ggtactttca gctcaggata ttgatctggt gaccaagcta     780
   tatgctgaaa cacatcaggt cgaactggat gatccgctga tttcaccgac ctatggtgaa     840
   ctgaagaatc tgccgcctgc ctatgtgatt accgcccgtc atgatgtgtt gcatgatgag     900
   ggttcaatct atgcgctgaa gttacgtgag aatggggtgc gagtgtatta tcaggagtat     960
   acggatcagg cccacggttt tatcaatctg accccaattc ataaacgttc gaaaaagcag    1020
   gtcattgaac tcagcaagaa tttccgtaaa ttcctggata aaaagatctg a             1071
<210> 20
   <211> 356
   <212> PRT
   <213> Unknown
<220>
   <223> "obtained from environmental sample
<400> 20
   Met Gly Ala Asn Asn Pro Val Thr Lys Phe Val Met Asp Lys Gly Gln
   1               5                   10                  15
   Gly Thr Ala Ala Arg Leu Leu Asp Cys Leu Pro Ser Phe Ala Gln Glu
               20                  25                  30
   Arg Leu Val Lys Ala Leu Asp Tyr Pro Tyr Asp Tyr Pro Asp Leu Asp
           35                  40                  45
   Pro Tyr Ile Lys Cys Leu Met Ala Ile Gln Ile Lys Gln Gly Glu His
       50                  55                  60
   Ser Phe Ile Ser Ala Asp Ala Val Gln Ser Arg Gln Leu Phe Asp Glu
   65                  70                  75                  80
   Arg Met Lys Ala Ile Gln Ala Lys Pro Thr Pro Val Lys Ala Val Glu
                   85                  90                  95
   Asp Leu Arg Leu Pro Leu Gln Asn Gly Thr Ile Phe Ala Arg His Tyr
               100                 105                 110
   His Pro Ala Pro Gln Lys Lys Leu Pro Met Val Ile Phe Tyr His Gly
           115                 120                 125
   Gly Ala Phe Ile Val Gly Gly Leu Asp Thr His Asp Glu Phe Cys Arg
       130                 135                 140
   Leu Leu Ala Val His Ala Lys Val Gln Val Leu Ser Val Ala Tyr Pro
   145                 150                 155                 160
   Leu Thr Pro Glu Tyr Ser Pro Leu Gln Met Val Gln Val Cys Glu Asp
                   165                 170                 175
   Ala Leu Ala Trp Val His Gln Asn Ile Lys Gln Leu Lys Ile Tyr Lys
               180                 185                 190
   Asn Gln Ile Val Val Ala Gly Asp Ser Ala Gly Gly Asn Leu Ala Ala
           195                 200                 205
   Val Val Ala Gln Arg Ser Ala Asp Lys Ile Tyr Ala Pro Arg Ala Gln
       210                 215                 220
   Leu Leu Leu Tyr Pro Ala Val Asp Phe Lys Ser Arg His Pro Ser Phe
   225                 230                 235                 240
   Tyr Ala Tyr Asn Gln Gly Leu Val Leu Ser Ala Gln Asp Ile Asp Leu
   245 250 255
   Val Thr Lys Leu Tyr Ala Glu Thr His Gln Val Glu Leu Asp Asp Pro
   260 265 270
   Leu Ile Ser Pro Thr Tyr Gly Glu Leu Lys Asn Leu Pro Pro Ala Tyr
   275 280 285
   Val Ile Thr Ala Arg His Asp Val Leu His Asp Glu Gly Ser Ile Tyr
   290 295 300
   Ala Leu Lys Leu Arg Glu Asn Gly Val Arg Val Tyr Tyr Gln Glu Tyr
   305 310 315 320
   Thr Asp Gln Ala His Gly Phe Ile Asn Leu Thr Pro Ile His Lys Arg
   325 330 335
   Ser Lys Lys Gln Val Ile Glu Leu Ser Lys Asn Phe Arg Lys Phe Leu
   340 345 350
   Asp Lys Lys Ile
   355
<210> 21
   <211> 1127
   <212> DNA
   <213> Arabidopsis thaliana
<400> 21
   aaaaaaagta aagaaaagaa aaactaataa agaacaaaaa aaatgtcctc ttcttcatca     60
   agaaacgcct ttgaagatgg caaatacaaa tcaaatctct taaccttgga ctcatcatct    120
   cgttgctgca aaataacacc gtcttctaga gcttcaccgt ctccgccaaa gcagctgttg    180
   gtggctacgc cggtggagga aggagattat ccggtggtga tgctcctcca tggttacctt    240
   ctctacaact ccttctattc tcagcttatg ttgcatgtct cttctcatgg cttcatcctc    300
   atcgctcctc agttatatag tatcgccgga ccagacacaa tggatgagat taaatcaacg    360
   gcggagatta tggattggtt atcagtagga cttaatcact ttcttccagc gcaagtaaca    420
   ccaaacctat ccaaatttgc cctctccggc catagccgcg gtggcaaaac cgcgtttgcg    480
   gtcgccttaa agaaatttgg gtactcctcg aatctaaaga tctcgacatt gatcggtata    540
   gatccagtcg atggaacagg gaaagggaaa caaacccctc ctccggtgtt ggcttacctt    600
   ccaaactcat ttgacctaga caaaacgcct atacttgtga tcggttcggg gcttggtgaa    660
   accgctcgga acccattatt cccaccgtgt gcacctcccg gagtgaatca ccgagagttc    720
   tttcgggaat gtcaaggtcc agcatggcat ttcgttgcga aggattatgg gcatttggac    780
   atgcttgatg atgatacaaa agggattaga gggaagagtt cttattgttt gtgtaagaat    840
   ggtgaagaga ggagaccaat gaggagattc gttggtggac ttgttgtatc atttttgaag    900
   gcttatttgg aaggagatga tcgtgaatta gttaagatca aagatgggtg tcacgaggat    960
   gttcccgttg aaattcaaga gtttgaggtt atcatgtaaa cataagtttt tctttagggg   1020
   ctggtttttc tattgtcaat atcatcagct tttgttgctt atggttttac aaacttatat   1080
   tgtacaactc tttaagtcac ctctttgctt atgatattaa cccgatc                 1127
<210> 22
   <211> 318
   <212> PRT
   <213> Arabidopsis thaliana
<400> 22
   Met Ser Ser Ser Ser Ser Arg Asn Ala Phe Glu Asp Gly Lys Tyr Lys
   1               5                   10                  15
   Ser Asn Leu Leu Thr Leu Asp Ser Ser Ser Arg Cys Cys Lys Ile Thr
               20                  25                  30
   Pro Ser Ser Arg Ala Ser Pro Ser Pro Pro Lys Gln Leu Leu Val Ala
           35                  40                  45
   Thr Pro Val Glu Glu Gly Asp Tyr Pro Val Val Met Leu Leu His Gly
       50                  55                  60
   Tyr Leu Leu Tyr Asn Ser Phe Tyr Ser Gln Leu Met Leu His Val Ser
   65                  70                  75                  80
   Ser His Gly Phe Ile Leu Ile Ala Pro Gln Leu Tyr Ser Ile Ala Gly
                   85                  90                  95
   Pro Asp Thr Met Asp Glu Ile Lys Ser Thr Ala Glu Ile Met Asp Trp
               100                 105                 110
   Leu Ser Val Gly Leu Asn His Phe Leu Pro Ala Gln Val Thr Pro Asn
           115                 120                 125
   Leu Ser Lys Phe Ala Leu Ser Gly His Ser Arg Gly Gly Lys Thr Ala
       130                 135                 140
   Phe Ala Val Ala Leu Lys Lys Phe Gly Tyr Ser Ser Asn Leu Lys Ile
   145                 150                 155                 160
   Ser Thr Leu Ile Gly Ile Asp Pro Val Asp Gly Thr Gly Lys Gly Lys
                   165                 170                 175
   Gln Thr Pro Pro Pro Val Leu Ala Tyr Leu Pro Asn Ser Phe Asp Leu
               180                 185                 190
   Asp Lys Thr Pro Ile Leu Val Ile Gly Ser Gly Leu Gly Glu Thr Ala
           195                 200                 205
   Arg Asn Pro Leu Phe Pro Pro Cys Ala Pro Pro Gly Val Asn His Arg
       210                 215                 220
   Glu Phe Phe Arg Glu Cys Gln Gly Pro Ala Trp His Phe Val Ala Lys
   225                 230                 235                 240
   Asp Tyr Gly His Leu Asp Met Leu Asp Asp Asp Thr Lys Gly Ile Arg
                   245                 250                 255
   Gly Lys Ser Ser Tyr Cys Leu Cys Lys Asn Gly Glu Glu Arg Arg Pro
               260                 265                 270
   Met Arg Arg Phe Val Gly Gly Leu Val Val Ser Phe Leu Lys Ala Tyr
           275                 280                 285
   Leu Glu Gly Asp Asp Arg Glu Leu Val Lys Ile Lys Asp Gly Cys His
       290                 295                 300
   Glu Asp Val Pro Val Glu Ile Gln Glu Phe Glu Val Ile Met
   305                 310                 315
<210> 23
   <211> 1157
   <212> DNA
   <213> Ginkgo biloba
<400> 23
   ttgaaaaaca aaaacgaaga agatgaactc agtacttgca cacagccatc ggccatggtt      60
   ttagtgaagg atgtgttcag cgaaggtcct ttacctgttc aaatcctcgc aattccacaa     120
   gccaactcat ctccatgctc aaaattagca gacaaaaacg gaactgcaac cacgccttct     180
   ccttgtcggc ctcctaaacc cctgctgatc gctcttcctt cccaacatgg agattatcct     240
   ctcatcctct ttttccacgg ctatgtactc ctcaattcct tctattctca actcttgcgc     300
   catgttgctt cccatggata catcgccata gctcctcaga tgtacagtgt aattggccca     360
   aatacgactc cagaaatagc cgatgcagcg gccattacag actggttacg agatggactc     420
   tcggataatc ttccgcaagc tttaaacaat catgtgaggc ccaattttga gaaatttgtg     480
   ctagcggggc actcgcgcgg gggtaaagtg gcatttgcac ttgccctagg tcgagtctcg     540
   cagccatctt taaagtactc ggcccttgta ggtcttgatc cagtcgatgg aatgggaaaa     600
   gatcaacaaa ccagtcatcc tattctgtca tacagagagc attcctttga tttgggtatg     660
   ccaacattag tggtaggttc gggcctgggt ccgtgcaaaa gaaaccctct cttccctccc     720
   tgtgctcctc aaggtgttaa ccaccatgat ttcttctacg aatgtgtcgc tcctgcctat     780
   cattttgttg cctctgatta tgggcatctt gatttcttag acgacgacac caaaggaata     840
   agaggaaagg ctacttattg cctctgtaag aatggggaag caagagagcc aatgcggaag     900
   tttagcggtg gaattgtggt tgcatttctt caagcatttc ttggtgataa tcgtggagcc     960
   ctgaatgata ttatggttta tccttcacat gctccagtca agattgagcc tccagagtct    1020
   ttggttacag aagatgtaaa atccccagaa gtcgaattat tacgccgggc agtttgcaga    1080
   tgatgtacca tggtattatg cattaaagga atgtatttgt tattaaaaaa atattaagaa    1140
   gtaaaaaaaa aaaaaaa                                                   1157
<210> 24
   <211> 342
   <212> PRT
   <213> Ginkgo biloba
<400> 24
   Met Val Leu Val Lys Asp Val Phe Ser Glu Gly Pro Leu Pro Val Gln
   1               5                   10                  15
   Ile Leu Ala Ile Pro Gln Ala Asn Ser Ser Pro Cys Ser Lys Leu Ala
               20                  25                  30
   Asp Lys Asn Gly Thr Ala Thr Thr Pro Ser Pro Cys Arg Pro Pro Lys
           35                  40                  45
   Pro Leu Leu Ile Ala Leu Pro Ser Gln His Gly Asp Tyr Pro Leu Ile
       50                  55                  60
   Leu Phe Phe His Gly Tyr Val Leu Leu Asn Ser Phe Tyr Ser Gln Leu
   65                  70                  75                  80
   Leu Arg His Val Ala Ser His Gly Tyr Ile Ala Ile Ala Pro Gln Met
                   85                  90                  95
   Tyr Ser Val Ile Gly Pro Asn Thr Thr Pro Glu Ile Ala Asp Ala Ala
               100                 105                 110
   Ala Ile Thr Asp Trp Leu Arg Asp Gly Leu Ser Asp Asn Leu Pro Gln
           115                 120                 125
   Ala Leu Asn Asn His Val Arg Pro Asn Phe Glu Lys Phe Val Leu Ala
       130                 135                 140
   Gly His Ser Arg Gly Gly Lys Val Ala Phe Ala Leu Ala Leu Gly Arg
   145                 150                 155                 160
   Val Ser Gln Pro Ser Leu Lys Tyr Ser Ala Leu Val Gly Leu Asp Pro
   165 170 175
   Val Asp Gly Met Gly Lys Asp Gln Gln Thr Ser His Pro Ile Leu Ser
   180 185 190
   Tyr Arg Glu His Ser Phe Asp Leu Gly Met Pro Thr Leu Val Val Gly
   195 200 205
   Ser Gly Leu Gly Pro Cys Lys Arg Asn Pro Leu Phe Pro Pro Cys Ala
   210 215 220
   Pro Gln Gly Val Asn His His Asp Phe Phe Tyr Glu Cys Val Ala Pro
   225 230 235 240
   Ala Tyr His Phe Val Ala Ser Asp Tyr Gly His Leu Asp Phe Leu Asp
   245 250 255
   Asp Asp Thr Lys Gly Ile Arg Gly Lys Ala Thr Tyr Cys Leu Cys Lys
   260 265 270
   Asn Gly Glu Ala Arg Glu Pro Met Arg Lys Phe Ser Gly Gly Ile Val
   275 280 285
   Val Ala Phe Leu Gln Ala Phe Leu Gly Asp Asn Arg Gly Ala Leu Asn
   290 295 300
   Asp Ile Met Val Tyr Pro Ser His Ala Pro Val Lys Ile Glu Pro Pro
   305 310 315 320
   Glu Ser Leu Val Thr Glu Asp Val Lys Ser Pro Glu Val Glu Leu Leu
   325 330 335
   Arg Arg Ala Val Cys Arg
   340
<210> 25
   <211> 884
   <212> DNA
   <213> Brassica oleracea
<400> 25
   acacaaaaaa atatataaca caaagaaata gaagaaggaa aaaatgtccc cctcctttct     60
   tttctttact ttgtttttga taaaggaaat gtcctcttca tcatcagcaa actcctttga    120
   ggacggcaaa tacaaaacag atcttttaac agtaggctta tcatcttgct gctggaaaaa    180
   gccctcctct tctccgactc cgcagtctcc gccgaagagg cttttggtgg caacgccggt    240
   ggaggaagga gaatatccgg tggtgatgct cctccatggt taccttctct acaactcatt    300
   ttattcccag cttatgttgc atgtctcttc ccatggcttc attgtcatcg ctccgcagtt    360
   atatagcatt gccggaccag acaccatgga tgagataaaa tcaacggcag agattattga    420
   ttggttatcg gtcggactaa accactttct tccaccacaa gtaacaccaa acctatccaa    480
   gttcgcactc tccggccata gccgtggtgg gaagaccgca tttgccttgg ccttaaagaa    540
   atttggatac tcgtccgacc taaagatctc ggcattgata ggtatagatg ttggaactgt    600
   tttttggaca aatggctatg gccaatattc cggtgaattt ttcgagcaat ttgattgtcg    660
   aaatgaccgg attgtggaat cgtaggattc attgttatga gcactatggt atagtgtaat    720
   catatatcaa aaacgaagtt cgtttgaatg agaaatgaaa gtctaaaata gattatttgt    780
   aaaatatcta tattagaatt atgaggtaag aaacctcttg tgtttaaaat ggagaagtta    840
   taacaaagtt ataaaaaact ttgtaaacaa tttggtgtgt tagc                     884
<210> 26
   <211> 213
   <212> PRT
   <213> Brassica oleracea
<400> 26
   Met Ser Pro Ser Phe Leu Phe Phe Thr Leu Phe Leu Ile Lys Glu Met
   1 5 10 15
   Ser Ser Ser Ser Ser Ala Asn Ser Phe Glu Asp Gly Lys Tyr Lys Thr
   20 25 30
   Asp Leu Leu Thr Val Gly Leu Ser Ser Cys Cys Trp Lys Lys Pro Ser
   35 40 45
   Ser Ser Pro Thr Pro Gln Ser Pro Pro Lys Arg Leu Leu Val Ala Thr
   50 55 60
   Pro Val Glu Glu Gly Glu Tyr Pro Val Val Met Leu Leu His Gly Tyr
   65 70 75 80
   Leu Leu Tyr Asn Ser Phe Tyr Ser Gln Leu Met Leu His Val Ser Ser
   85 90 95
   His Gly Phe Ile Val Ile Ala Pro Gln Leu Tyr Ser Ile Ala Gly Pro
   100 105 110
   Asp Thr Met Asp Glu Ile Lys Ser Thr Ala Glu Ile Ile Asp Trp Leu
   115 120 125
   Ser Val Gly Leu Asn His Phe Leu Pro Pro Gln Val Thr Pro Asn Leu
   130 135 140
   Ser Lys Phe Ala Leu Ser Gly His Ser Arg Gly Gly Lys Thr Ala Phe
   145 150 155 160
   Ala Leu Ala Leu Lys Lys Phe Gly Tyr Ser Ser Asp Leu Lys Ile Ser
   165 170 175
   Ala Leu Ile Gly Ile Asp Val Gly Thr Val Phe Trp Thr Asn Gly Tyr
   180 185 190
   Gly Gln Tyr Ser Gly Glu Phe Phe Glu Gln Phe Asp Cys Arg Asn Asp
   195 200 205
   Arg Ile Val Glu Ser
   210
<210> 27
   <211> 329
   <212> PRT
   <213> Citrus sinensis
<400> 27
   Met Ala Ala Met Val Asp Ala Lys Pro Ala Ala Ser Val Gln Gly Thr
   1               5                   10                  15
   Pro Leu Leu Ala Thr Ala Thr Leu Pro Val Phe Thr Arg Gly Ile Tyr
               20                  25                  30
   Ser Thr Lys Arg Ile Thr Leu Glu Thr Ser Ser Pro Ser Ser Pro Pro
           35                  40                  45
   Pro Pro Lys Pro Leu Ile Ile Val Thr Pro Ala Gly Lys Gly Thr Phe
       50                  55                  60
   Asn Val Ile Leu Phe Leu His Gly Thr Ser Leu Ser Asn Lys Ser Tyr
   65                  70                  75                  80
   Ser Lys Ile Phe Asp His Ile Ala Ser His Gly Phe Ile Val Val Ala
                   85                  90                  95
   Pro Gln Leu Tyr Thr Ser Ile Pro Pro Pro Ser Ala Thr Asn Glu Leu
               100                 105                 110
   Asn Ser Ala Ala Glu Val Ala Glu Trp Leu Pro Gln Gly Leu Gln Gln
           115                 120                 125
   Asn Leu Pro Glu Asn Thr Glu Ala Asn Val Ser Leu Val Ala Val Met
       130                 135                 140
   Gly His Ser Arg Gly Gly Gln Thr Ala Phe Ala Leu Ser Leu Arg Tyr
   145                 150                 155                 160
   Gly Phe Gly Ala Val Ile Gly Leu Asp Pro Val Ala Gly Thr Ser Lys
                   165                 170                 175
   Thr Thr Gly Leu Asp Pro Ser Ile Leu Ser Phe Asp Ser Phe Asp Phe
               180                 185                 190
   Ser Ile Pro Val Thr Val Ile Gly Thr Gly Leu Gly Gly Val Ala Arg
           195                 200                 205
   Cys Ile Thr Ala Cys Ala Pro Glu Gly Ala Asn His Glu Glu Phe Phe
       210                 215                 220
   Asn Arg Cys Lys Asn Ser Ser Arg Ala His Phe Val Ala Thr Asp Tyr
   225                 230                 235                 240
   Gly His Met Asp Ile Leu Asp Asp Asn Pro Ser Asp Val Lys Ser Trp
                   245                 250                 255
   Ala Leu Ser Lys Tyr Phe Cys Lys Asn Gly Asn Glu Ser Arg Asp Pro
               260                 265                 270
   Met Arg Arg Cys Val Ser Gly Ile Val Val Ala Phe Leu Lys Asp Phe
           275                 280                 285
   Phe Tyr Gly Asp Ala Glu Asp Phe Arg Gln Ile Leu Lys Asp Pro Ser
       290                 295                 300
   Phe Ala Pro Ile Lys Leu Asp Ser Val Glu Tyr Ile Asp Ala Ser Ser
   305                 310                 315                 320
   Met Leu Thr Thr Thr His Val Lys Val
                   325